(19) Europäisches Patentamt
European Patent Office
Office européen des brevets

(11) EP 3 407 905 B1

(12) **EUROPEAN PATENT SPECIFICATION**

(45) Date of publication and mention of the grant of the patent:
**17.02.2021 Bulletin 2021/07**

(21) Application number: **17705790.8**

(22) Date of filing: **26.01.2017**

(51) Int Cl.:
***A61K 38/00*** *(2006.01)* ***A61K 38/17*** *(2006.01)*
***C07K 14/47*** *(2006.01)* ***A23L 33/00*** *(2016.01)*

(86) International application number:
**PCT/DK2017/050017**

(87) International publication number:
**WO 2017/129195 (03.08.2017 Gazette 2017/31)**

(54) **METHODS FOR MODULATING INTESTINAL MICROBIOTA**

VERFAHREN ZUR MODULIERUNG DER DARMFLORA

PROCÉDÉS DE MODULATION DU MICROBIOTE INTESTINAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.01.2016 DK 201670041**
**01.07.2016 DK 201670483**

(43) Date of publication of application:
**05.12.2018 Bulletin 2018/49**

(73) Proprietor: **Defensin Therapeutics ApS**
**2200 København N (DK)**

(72) Inventors:
- **NORDKILD, Peter**
**2820 Gentofte (DK)**
- **WEHKAMP, Jan**
**72766 Reutlingen (DE)**
- **KJÆRULFF, Søren**
**2840 Holte (DK)**

(74) Representative: **Høiberg P/S**
**Adelgade 12**
**1304 Copenhagen K (DK)**

(56) References cited:
**WO-A2-2007/133373 WO-A2-2008/115390**
**CN-A- 104 971 343**

- **ZHIRU TANG ET AL: "Oral administration of synthetic porcine beta-defensin-2 improves growth performance and cecal microbial flora and down-regulates the expression of intestinal toll-like receptor-4 and inflammatory cytokines in weaned piglets challenged with enterotoxigeni", ANIMAL SCIENCE JOURNAL - NIHON CHIKUSAN GAKKAIHO, vol. 87, no. 10, 28 December 2015 (2015-12-28), pages 1258-1266, XP055360082, JP ISSN: 1344-3941, DOI: 10.1111/asj.12540**
- **KRENTZ A J ET AL: "New drugs for type 2 diabetes mellitus: What is their place in therapy?", DRUGS, ADIS INTERNATIONAL LTD, NZ, vol. 68, no. 15, 1 January 2008 (2008-01-01), pages 2131-2162, XP009122109, ISSN: 0012-6667, DOI: 10.2165/00003495-200868150-00005**
- **JAN WEHKAMP ET AL: "Mechanisms of Disease: defensins in gastrointestinal diseases", NATURE CLINICAL PRACTICE GASTROENTEROLOGY & HEPATOLOGY, vol. 2, no. 9, 1 September 2005 (2005-09-01), pages 406-415, XP55359967, GB ISSN: 1743-4378, DOI: 10.1038/ncpgasthep0265**
- **KIMINORI NAKAMURA ET AL: "Paneth cell [alpha]-defensins and enteric microbiota in health and disease", BIOSCIENCE OF MICROBIOTA, FOOD AND HEALTH, vol. 35, no. 2, 26 November 2015 (2015-11-26), pages 57-67, XP055359970, DOI: 10.12938/bmfh.2015-019**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Lisa M Neff ET AL: "Diabetes, Metabolic Syndrome and Obesity: Targets and Therapy Dovepress emerging role of GLP-1 receptor agonists in the treatment of obesity", Diabetes, Metabolic Syndrome and Obesity: Targets and Therapy, 1 January 2010 (2010-01-01), pages 3-263, XP055189069, Retrieved from the Internet: URL:http://www.ncbi.nlm.nih.gov/pmc/articles/PMC3047971/pdf/dmso-3-263.pdf [retrieved on 2015-05-13]
- A. EVERARD ET AL: "Cross-talk between Akkermansia muciniphila and intestinal epithelium controls diet-induced obesity", PROCEEDINGS OF THE NATIONAL ACADEMY OF SCIENCES, vol. 110, no. 22, 28 May 2013 (2013-05-28) , pages 9066-9071, XP055074467, ISSN: 0027-8424, DOI: 10.1073/pnas.1219451110
- NITA H. SALZMAN: "Paneth cell defensins and the regulation of the microbiome", GUT MICROBES, vol. 1, no. 6, 1 November 2010 (2010-11-01), pages 401-406, XP055360127, United States ISSN: 1949-0976, DOI: 10.4161/gmic.1.6.14076

SEQUENCE LISTING

<110> Defensin Therapeutics ApS

<120> Methods for Modulating Intestinal Microbiota

<130> P4094PC00

<150> DK PA 2016 70041
<151> 2016-01-26

<150> DK PA 2016 70483
<151> 2016-07-01

<160> 17

<170> PatentIn version 3.5

<210> 1
<211> 40
<212> PRT
<213> Bos taurus

<400> 1

```
Gly Val Gly Asn Pro Val Ser Cys Val Arg Asn Lys Gly Ile Cys Val
1               5                   10                  15

Pro Ile Arg Cys Pro Gly Ser Met Lys Gln Ile Gly Thr Cys Val Gly
            20                  25                  30

Arg Ala Val Lys Cys Cys Arg Lys
        35                  40
```

<210> 2
<211> 34
<212> PRT
<213> Gallus gallus

<400> 2

```
Leu Phe Cys Lys Gly Gly Ser Cys His Phe Gly Gly Cys Pro Ser His
1               5                   10                  15

Leu Ile Lys Val Gly Ser Cys Phe Arg Ser Cys Cys Lys Trp Pro Trp
            20                  25                  30

Asn Ala
```

<210> 3
<211> 44
<212> PRT
<213> Pongo pygmaeus

<400> 3

```
Val Phe Gly Asp Ile Ser Asn Pro Val Thr Cys Leu Arg Ser Gly Ala
1               5                   10                  15

Ile Cys His Pro Gly Phe Cys Pro Arg Arg Tyr Lys His Ile Gly Thr
            20                  25                  30

Cys Gly Leu Ser Val Ile Lys Cys Cys Lys Lys Pro
        35                  40
```

<210> 4
<211> 36
<212> PRT
<213> Homo sapiens

<400> 4

```
Asp His Tyr Asn Cys Val Ser Ser Gly Gly Gln Cys Leu Tyr Ser Ala
1               5                   10                  15

Cys Pro Ile Phe Thr Lys Ile Gln Gly Thr Cys Tyr Arg Gly Lys Ala
            20                  25                  30

Lys Cys Cys Lys
        35
```

<210> 5
<211> 41
<212> PRT
<213> Homo sapiens

<400> 5

```
Gly Ile Gly Asp Pro Val Thr Cys Leu Lys Ser Gly Ala Ile Cys His
1               5                   10                  15

Pro Val Phe Cys Pro Arg Arg Tyr Lys Gln Ile Gly Thr Cys Gly Leu
            20                  25                  30

Pro Gly Thr Lys Cys Cys Lys Lys Pro
        35                  40
```

<210> 6
<211> 45
<212> PRT
<213> Homo sapiens

<400> 6

```
Gly Ile Ile Asn Thr Leu Gln Lys Tyr Tyr Cys Arg Val Arg Gly Gly
1               5                   10                  15

Arg Cys Ala Val Leu Ser Cys Leu Pro Lys Glu Glu Gln Ile Gly Lys
            20                  25                  30

Cys Ser Thr Arg Gly Arg Lys Cys Cys Arg Arg Lys Lys
        35                  40                  45
```

<210> 7
<211> 37
<212> PRT
<213> Homo sapiens

<400> 7

```
Glu Leu Asp Arg Ile Cys Gly Tyr Gly Thr Ala Arg Cys Arg Lys Lys
1               5                   10                  15

Cys Arg Ser Gln Glu Tyr Arg Ile Gly Arg Cys Pro Asn Thr Tyr Ala
            20                  25                  30

Cys Cys Leu Arg Lys
        35
```

<210> 8
<211> 32
<212> PRT
<213> Homo sapiens

<400> 8

```
Ala Thr Cys Tyr Cys Arg Thr Gly Arg Cys Ala Thr Arg Glu Ser Leu
1               5                   10                  15

Ser Gly Val Cys Glu Ile Ser Gly Arg Leu Tyr Arg Leu Cys Cys Arg
            20                  25                  30
```

<210> 9
<211> 32
<212> PRT
<213> Homo sapiens

<400> 9

```
Ala Phe Thr Cys His Cys Arg Arg Ser Cys Tyr Ser Thr Glu Tyr Ser
1               5                   10                  15

Tyr Gly Thr Cys Thr Val Met Gly Ile Asn His Arg Phe Cys Cys Leu
            20                  25                  30
```

<210> 10
<211> 41

<212> PRT
<213> Pan troglodytes

<400> 10

```
Gly Ile Ser Asp Pro Val Thr Cys Leu Lys Ser Gly Ala Ile Cys His
1               5                   10                  15

Pro Val Phe Cys Pro Arg Arg Tyr Lys Gln Ile Gly Thr Cys Gly Leu
            20                  25                  30

Pro Gly Thr Lys Cys Cys Lys Lys Pro
        35                  40
```

<210> 11
<211> 41
<212> PRT
<213> Macaca mulatta

<400> 11

```
Gly Ile Gly Asp Pro Val Thr Cys Leu Lys Asn Gly Ala Ile Cys His
1               5                   10                  15

Pro Val Phe Cys Pro Arg Arg Tyr Lys Gln Ile Gly Thr Cys Gly Leu
            20                  25                  30

Pro Gly Thr Lys Cys Cys Lys Lys Pro
        35                  40
```

<210> 12
<211> 40
<212> PRT
<213> Mus musculus

<400> 12

```
Lys Ile Asn Asn Pro Val Ser Cys Leu Arg Lys Gly Gly Arg Cys Trp
1               5                   10                  15

Asn Arg Cys Ile Gly Asn Thr Arg Gln Ile Gly Ser Cys Gly Val Pro
            20                  25                  30

Phe Leu Lys Cys Cys Lys Arg Lys
        35                  40
```

<210> 13
<211> 40
<212> PRT
<213> Equus caballus

<400> 13

```
Gly Ile Gly Asn Pro Ile Ser Cys Ala Arg Asn Arg Gly Val Cys Ile
1               5                   10                  15

Pro Ile Gly Cys Leu Pro Gly Met Lys Gln Ile Gly Thr Cys Gly Leu
            20                  25                  30

                Pro Gly Thr Lys Cys Cys Arg Lys
                        35                  40
```

<210> 14
<211> 40
<212> PRT
<213> Sus scrofa

<400> 14

```
Asn Ile Gly Asn Ser Val Ser Cys Leu Arg Asn Lys Gly Val Cys Met
1               5                   10                  15

Pro Gly Lys Cys Ala Pro Lys Met Lys Gln Ile Gly Thr Cys Gly Met
            20                  25                  30

Pro Gln Val Lys Cys Cys Lys Arg
        35                  40
```

<210> 15
<211> 40
<212> PRT
<213> Capra aegagrus

<400> 15

```
Gly Ile Ile Asn His Arg Ser Cys Tyr Arg Asn Lys Gly Val Cys Ala
1               5                   10                  15

Pro Ala Arg Cys Pro Arg Asn Met Arg Gln Ile Gly Thr Cys His Gly
            20                  25                  30

Pro Pro Val Lys Cys Cys Arg Lys
        35                  40
```

<210> 16
<211> 37
<212> PRT
<213> Homo sapiens

<400> 16

```
Leu Leu Gly Asp Phe Phe Arg Lys Ser Lys Glu Lys Ile Gly Lys Glu
1               5                   10                  15

Phe Lys Arg Ile Val Gln Arg Ile Lys Asp Phe Leu Arg Asn Leu Val
            20                  25                  30

Pro Arg Thr Glu Ser
        35
```

<210> 17
<211> 37
<212> PRT
<213> Homo sapiens

<400> 17

```
Pro Val Thr Cys Leu Lys Ser Gly Ala Ile Cys His Pro Val Phe Cys
1               5                   10                  15

Pro Arg Arg Tyr Lys Gln Ile Gly Thr Cys Gly Leu Pro Gly Thr Lys
            20                  25                  30

Cys Cys Lys Lys Pro
        35
```

## Description

### Field

The present disclosure relates to methods for modulating or stabilizing the intestinal microbiota by orally administering one or more defensins. The methods can be used to treat or prevent gut inflammation, colorectal cancer, metabolic syndrome, obesity, prediabetes, diabetes and cardiovascular disease as well as for promotion of lean growth in meat production.

### Background

Intestinal microbiota

The increasing prevalence of common disorders like obesity and obesity related diseases is tightly associated with our westernized lifestyle and diet. The most prominent obesity-related ailments are insulin resistance, overt type 2 diabetes (T2D) and certain cancers (Faulds & Dahlman-Wright, 2012). While the aetiology of these diseases is complex, many of them are characterized by a general state of low-grade inflammation, which may originate from a dysregulated intestinal microbiota (Everard & Cani, 2013; Belkaid & Hand, 2014). Even though the challenges associated with modern human lifestyles and animal meat production may seem far apart, it is envisaged that impaired intestinal health is a common denominator. Dysregulated intestinal health is indeed associated with an array of diverse diseases like obesity (Ridaura et al, 2013), type 2 diabetes (Qin et al, 2012), rheumatoid arthritis (Zhang et al, 2015) and colorectal cancer (Feng et al, 2015). Recently, a connection between intestinal microbiota, and in particular the presence of certain lipopolysaccharides from *Bacteroides,* and the higher rate of occurrence of type 1 diabetes in Finland in comparison to neighbouring areas has been reported (Leviten 2016).

Obesity and its concomitant low-grade inflammation form a potent driver of dysregulated metabolic homeostasis. Turnbaugh et al. (2006) found that obesity-associated microbiota had an increased capacity for energy harvest, and 2 weeks after transplantation of microbiota from obese mice, germ-free mice showed significantly greater increase in fat mass than similar transplantation from lean mice. Turnbaugh et al. (2008) further and significantly discovered that changes in intestinal microbial composition were completely reversed after a shift back to the original diet in mice temporarily fed a high fat/sugar "Western" diet. These findings were confirmed in man by Vrieze et al. (2012), who demonstrated that transfer of intestinal microbiota from lean human donors increased insulin sensitivity in individuals with metabolic syndrome.

Manipulation of intestinal microbiota to increase weight and weight-gain rates has been employed for many years in agricultural live-stock through the use of low-dose antibiotics and probiotics such as *Lactobacillus ingluviei.* Intestinal microbiota manipulation for weight gain has been demonstrated in chickens (Khan et al, 2007), in ducks (Angelakis & Raoult, 2010), and in mice (Angelakis et al, 2012). In humans, infants receiving antibiotics have also been found to be larger than their controls (Trasande et al, 2012), while early exposure to oral antibiotics is associated with overweight in children (Ajslev et al, 2014). And in pregnant women, the physiological increase in adiposity and potential development of gestational diabetes in the third trimester appears to be associated with a profound change in intestinal microbiota (Koren etal, 2012).

The intestinal mucosa is by far the largest body surface (approximately 200 $m^2$) exposed to the external environment. As such, the intestinal surface is in intimate contact with foreign material, metabolites derived from our diet, and the estimated $10^{14}$ bacteria - the intestinal microbiota - that inhabit our intestine. Thus the intestinal barrier is under constant and intense immune surveillance, requiring a dynamic crosstalk among the immune system, dietary components, and the intestinal microbiota. Diet interventions have tremendous impact on immune regulation (Mowat & Agace, 2014) and intestinal microbiota composition (Walter, 2015), both of which independently and synergistically influence metabolic homeostasis. In this regard, two very recent papers emphasize the (adverse) potential of food additives in microbiota-modulated changes to metabolic homeostasis. A recent paper (Chassaing et al, 2015) illustrated how dietary emulsifiers impair glucose tolerance, thus increasing weight gain as well as colitis susceptibility by induction of a dysregulated intestinal microbiota. The observations could not be replicated in germ-free (GF) mice, suggesting a pivotal role for the intestinal microbiota. Similarly, Suez et al. (2014) recently showed how non-caloric artificial sweeteners induced metabolic dysfunction through alterations of the intestinal microbiota. The authors validated their findings by faecal transfer to GF mice, after which the GF mice rapidly developed glucose intolerance. These observations mirror a pioneering study in GF mice (Bäckhed et al, 2007), elucidating the role of intestinal microbes in the maintenance of metabolic health. This study showed that in the absence of commensal microbes, thereby causing an imbalanced mucosal immune homeostasis, the adipose tissues decreased in size and function in response to a high fat diet. Despite lack of weight gain, which normally would appear as a healthy phenotype, ectopic lipid accumulation (hepatic steatosis & increased levels of serum triglycerides) resulted in severe metabolic disorders. In man, it has been shown that gene richness of the microbiota is associated with a healthy phenotype, whereas gene poverty (low gene counts) correlates with increased risk of metabolic disorders (Le Chatelier et al, 2013).

## Defensins

Defensins represent one of the dominant innate host defences that serve to maintain a healthy microbiome and ward off potential pathogens (Wehkamp et al, 2002 and Salzman et al, 2007). Defensins are peptides possessing antimicrobial activity against Gram positive and negative bacteria, fungi and archaea as well as anti-inflammatory activity increasing anti-inflammatory cytokines and decreasing inflammatory cytokines.

The human defensins are small cationic peptides that can be divided into α- and β-defensins based on the topology of their three intramolecular cysteine disulphide bonds. The human α-defensins can be further subdivided into those that were first isolated from neutrophil granules (HNP1-4) and the intestinal α-defensins that are expressed by Paneth cells in the crypts of the small intestine (HD5 and HD6 or DEFA5 and DEFA6). The β-defensins (DEFBn) are mainly produced by epithelial cells in various tissues and organs including the skin, eye, middle ear, mouth, trachea, lungs, gastrointestinal tract, liver, urogenital system, kidneys, vagina, pancreas and mammary glands. The best characterized members of the human β-defensin family are hBD1-4. Some of the human defensins are produced constitutively, whereas others are induced by pro-inflammatory cytokines or exogenous microbial products. Some of the human defensins are already expressed in the amniotic fluid at increasing levels with gestational age, protecting the fetus in the womb. Breast milk and in particular the first milk, colostrum, contains both α- and β-defensins, but only a few of them are found in significant concentrations in maternal milk (Armogida et al, 2004).

Liu et al. (2008) and WO 2008/115390, found that HNP-1 and HNP-2, both produced by leucocytes and belonging to a subgroup of α-defensins in the blood, were able to inhibit glycogenolysis and gluconeogenesis in isolated hepatocytes through an intracellular mechanism distinctly different from the classical insulin signalling pathway. WO 2007/133373 relates to fusion proteins comprising TNFα and a chemokine or a defensin and their use as an antigen for inducing an immune response against TNFα for use in the treatment of diseases mediated by pathological TNFα, such as chronic neuropathic pain, chronic inflammation, insulin resistance, diabetes, hyperlipidemia, hypertriglyceridemia, hypercholesterolemia, and artherosclerosis.

CN104971343 relates to a high fat diet and vitamin D deficient mouse model of metabolic disorders and treatment of the mouse model with a mutant HD5 (HD5(T7H)). Tang et al. 2015 (Animal Science Journal, 2015, 87(10), 1258-1266) relates to the use of porcine beta defensin 2 (pBD2) for the treatment of weaning piglets challenged with *E. coli.*

## Summary

The invention is defined by the appended claims. Any disclosure not falling under the scope of the appended claims does not form part of the invention.

The present disclosure demonstrates that mammalian, intestinal α- and β-defensins, orally administered, have the ability of maintaining a normal microbiota composition in the intestine of a mouse fed a high fat diet. The data in the examples demonstrate that oral administration of mammalian α- and/or β-defensins results in stabilization or normalization of a dysbiotic microbiota. Defensins are therefore useful in treatment or prevention of colorectal cancer, an endocrine, nutritional, metabolic or cardiovascular disease or as lean growth promoters in meat production.

As demonstrated in examples 1 and 3, an oral dosage of human alfa defensin 5 (HD5) or human beta-defensin 2 (hBD2) prevents or reduces weight gain in mice kept on a high fat diet. The animal model is a model of metabolic syndrome and early type 2 diabetes. Unless treated, the mice when fed the high fat diet develop obesity by accumulating fat, in particular abdominal fat and liver fat. The mice further develop signs of diabetes, such as insulin resistance, and impaired glucose tolerance.

Without HD5 or hBD2 in the diet the animals gain considerably more weight on the high fat diet and eventually develop signs of diabetes and obesity. The reduced weight gain occurred as a reduction in accumulation of fat mass. The animals receiving a dosage of HD5 or hBD2 show increased glucose tolerance and decreased insulin resistance compared to non-treated animals on the high-fat diet.

Similarly, the examples demonstrate that oral administration of HD5 to animals with a dysbiotic microflora can at least partially normalize the microflora. Therefore, HD5 and other defensins can be used for normalization of dysbiotic microflora or for normalization of microflora. High fat diet and diet with high sugar content are known to induce dysbiotic microflora. Thus defensins can be used to treat such dysbiotic microflora. Defensins can also be used prophylactically for subjects that are to undergo treatments that are expected to affect the microflora negatively, e.g. antibiotic treatment, immunosuppressive treatment, chemotherapy, immunotherapy, or radiation therapy.

One aspect of the present disclosure relates to a method for treatment of gut inflammation in non-human animals, the method comprising administration of an effective amount of a defensin, a mammalian or poultry α- and/or β- defensin to a subject in need thereof.

One aspect of the present disclosure relates to a method for treatment of gut inflammation in humans, the method comprising administration of an effective amount of a defensin, a mammalian or poultry α- and β- defensin to a subject in need thereof.

One aspect of the present disclosure relates to a method for treatment of gut inflammation, wherein the inflammation is located in the mouth, oesophagus, stomach, duodenum, jejunum, ileum, cecum, rectum, and/or anal canal of an animal, the method comprising administration of an effective amount of a defensin, a mammalian or poultry α- or β-defensin to a subject in need thereof.

One aspect of the present disclosure relates to a method for maintaining a normal microbiota composition in the intestine, the method comprising administration of an effective amount of a defensin, a mammalian or poultry α-defensin and/or β-defensin and/or a glucagon-like-peptide 1 (GLP-1)/GLP-1 analog to a subject in need thereof.

One aspect of the present disclosure relates to a method for treatment of a dysbiotic microbiota in the intestine, the method comprising administration of an effective amount of a defensin, a mammalian or poultry α-defensin and/or β-defensin and/or a glucagon-like-peptide 1 (GLP-1)/GLP-1 analog to a subject in need thereof.

One aspect of the present disclosure relates to a method for increasing gene richness of the intestinal microbiota, the method comprising administration of an effective amount of a defensin, a mammalian or poultry α-defensin and/or β-defensin and/or a glucagon-like-peptide 1 (GLP-1)/GLP-1 analog.

One aspect of the present disclosure relates to a method for increasing the number of phylae of the intestinal microbiota, said method comprising administration of an effective amount of a defensin, a mammalian or poultry α-defensin and/or β-defensin and/or a glucagon-like-peptide 1 (GLP-1)/GLP-1 analog.

One aspect of the present disclosure relates to a method for increasing the production of short fatty acids in the intestinal microbiota, said method comprising administration of an effective amount of a defensin, a mammalian or poultry α-defensin and/or β-defensin and/or a glucagon-like-peptide 1 (GLP-1)/GLP-1 analog.

One aspect of the present disclosure relates to a method for increasing the butyrate production or decreasing the acetate production of the intestinal microbiota, said method comprising administration of an effective amount of a defensin, a mammalian or poultry α-defensin and/or β-defensin and/or a glucagon-like-peptide 1 (GLP-1)/GLP-1 analog.

One aspect of the present disclosure relates to a method for increasing the number of bacteria belonging to a genus selected from a group composed of *Bacterioidetes, Faecalibacterium, Roseburia, Blautia, Ruminococcus, Bifidobacterium, Methanobrevibacter, Lactobacillus, Coprococcus, Clostridium, Allobaculum, Alloprevotella, Akkermansia, Eubacterium* in the intestine, said method comprising administration of an effective amount of a mammalian or poultry α-defensin and/or β-defensin and/or a glucagon-like-peptide 1 (GLP-1)/GLP-1 analog. Preferably, the genus of bacterium includes one or more of *Allobaculum, Alloprevotella, Akkermansia,* and *Lactobacillus.*

One aspect of the present disclosure relates to a method for decreasing the number of bacteria selected from a group composed of *Bacteroidetes fragilis, Sutturella wadsworthia, Veillonella parvula, Escherichi coli, Haemophilus parainfluenzae, Fusobacterium nucleatum, Eikenella corodens, Gemella moribillum* in the intestine, said method comprising administration of an effective amount of a mammalian or poultry α-defensin and/or β-defensin and/or a glucagon-like-peptide 1 (GLP-1)/GLP-1 analog.

One aspect of the present disclosure relates to a method for treatment of colorectal cancer, an endocrine, nutritional, metabolic or cardiovascular disease, said method comprising administration of an effective amount of a defensin, a mammalian or poultry α-defensin and/or β-defensin to a subject in need thereof. As defensins can be used to treat obesity and various symptoms of type 2 diabetes, and be used to normalize microflora, they can also reduce the risk of contracting one or more of the mentioned disorders.

One aspect of the present disclosure relates to a method for promotion of lean growth in animal meat production, said method comprising administration of an effective amount of a defensin, a mammalian or poultry α-defensin and/or β-defensin to a subject in need thereof. This aspect of the present disclosure is supported by the demonstration that administration of HD5 or hBD2 to obese mice leads to a reduction in fat percentage, i.e. the defensins favour lean growth over fat growth.

One aspect of the present disclosure relates to a composition comprising at least one mammalian or poultry α-defensin and at least one mammalian or poultry β-defensin.

One aspect of the present disclosure relates to a composition comprising at least one mammalian α- or β-defensin in combination with either insulin/insulin analogs and/or glucagon like peptide-1 (GLP-1)/GLP-1 analogs and/or glucagon like peptide-2 (GLP-2)/GLP-2 analogs and/or a dipeptidyl peptidase IV (DPP-IV) inhibitor and/or metformin and/or a sodium glucose transporter-2 (SGLT-2) inhibitor and/or a Glucagon receptor antagonist and/or a transient receptor potential cation channel subfamily V member 1 (TRPV1) antagonist or a combination of these. In one aspect of the present disclosure the defensin is HD5 or hBD-2.

One aspect of the present disclosure relates to a composition comprising at least one mammalian α- or β-defensin for use in combination with chemotherapy, immunotherapy, radiotherapy or a combination of these. In one aspect of the present disclosure the defensin is HD5 or hBD-2, preferably when the defensin is orally administered.

it is known in the art that defensins, including human beta-defensin 2 are strong anti-inflammatory agents (WO 2010/007165). The present inventors have demonstrated the anti-obesity and anti-diabetic effects of orally administered human beta-defensin 2. Another intestinal hormone, GLP-1, and GLP-1 analogs such as Liraglutid can likewise be used to treat obesity and diabetes. The present inventors demonstrate in Example 4 that parenterally administered Liraglutid has no effect on various inflammatory and anti-inflammatory cytokines. Therefore, GLP-1 and GLP-1 analogs have a different mode of action compared to defensins. Therefore, the present inventors contemplate administering a combination of at least one defensin with at least one GLP-1 or GLP-1 analog to treat the indications as herein described.

**Description of Drawings**

The invention is defined by the appended claims. Any drawings not falling under the scope of the appended claims does not form part of the invention.

Figure 1A. Schematic outline of the experimental set up for investigating the effects of mammalian alfa and/or beta defensins on mice metabolism. At week -1, the C57/BL/6J mice were divided in groups and cages, so that there were 3 mice per cage and cages per group. Between week -1 and 0, the mice were clinically examined by magnetic resonance scan to estimate fat distribution. At week 0, 1 and 4, the microbiome of the faeces was analysed. At week 4, in addition to analysis of the microbiota, the mice were scanned and blood glucose and insulin levels were measured. At week 6, the energy consumption was assessed by analysing nitrogen and lipid content of the faeces. At week 7, insulin tolerance test (ITT) was conducted. At week 8, oral glucose tolerance test (OGTT) and glucose-stimulated insulin secretion (GSIS) were conducted. At week 9 (termination), several analyses were conducted, in particular the mice were weighed and scanned, and plasma composition and microbiota composition of colon, cecum and small intestine were assessed. In addition, histological analysis and protein/RNA analysis were performed on muscular tissue (quadriceps), iWAT, eWAT, iBAT, liver, colon, jejunum, ileum and duodenum.

Figure 1B. Schematic outline of the experimental set up for investigating the effects of mammalian alfa and/or beta defensins on mice metabolism. At week -1, the C57/BL/6J mice arrived. At week 0 feces was collected. During run-in between week 0 and week 12 the mice were fed a high fat diet. At week 12 the mice were clinically examined by magnetic resonance scan to estimate fat distribution, feces was collected and oral glucose tolerance test (OGTT) and glucose-stimulated insulin secretion (GSIS) performed. At week 13-0 the mice were divided in groups and cages with 4 mice percage and 3 cages per group. At week 0, 12 and 13-10, the microbiome of the faeces was analysed. At week 13-2, 13-4, 13-6, 13-8 and 13-10 the mice were scanned and blood glucose and insulin levels were measured. At week 13-9, insulin tolerance test (ITT) was conducted. At week 13-10 (termination), several analyses were conducted, in particular the mice were weighed and scanned, and plasma composition and microbiota composition of colon, cecum and small intestine were assessed. In addition, iWAT, eWAT and liver weight were measured.

Figure 2. Clustal W (2.1) multiple sequence alignment of human beta defensin 1-4.

Figure 3. Clustal W (2.1) multiple sequence alignment of human alpha defensin 5 and 6.

Figure 4: Clustal W (2.1) multiple sequence alignment of human neutrophil peptide 1-3.

Figure 5: Clustal W (2.1) multiple sequence alignment of human, Rhesus macaque, chimpanzee and orangutan beta defensin 2.
In the Clustal W alignments:

* indicates positions which have a single, fully conserved residue.

indicates that one of the following ’strong’ groups is fully conserved:
-S,T,A; N,E,Q,K; N,H,Q,K; N,D,E,Q; Q,H,R,K; M,I,L,V; M,I,L,F; H,Y; F,Y,W.
indicates that one of the following ’weaker’ groups is fully conserved:
-C,S,A; A,T,V; S,A,G; S,T,N,K; S,T,P,A; S,G,N,D; S,N,D,E,Q,K; N,D,E,Q,H,K; N,E,Q,H,R,K; V,L,I,M; H,F,Y.

Figure 6: Weight change (A) and weight development (B) and cumulative feed intake (C) over 7 weeks treatment of mice with low fat diet (LFD), high fat diet (HFD) or HFD and defensin hBD2 (HFD + P2).

A: Weight change. N = 12. Both HFDs are significantly different from the LFD reference group. Asterisks depicts differences between HFD and HFD + P2. Both HFD are significantly different from LFD from week w (p<0.001). 2-way ANOVA, Tukey Post test.

B: Weight development. N = 12. Both HFDs are significantly different from the LFD reference group. Asterisks depicts differences between HFD and HFD + P2. Both HFD are significantly different from LFD from week w ($p<0.001$). 2-way ANOVA, Tukey Post test.

C: Cumulative feed intake. The LFD reference group has the same amount of both sucrose and protein per gram of feed compared to the HFD groups.

Figure 7: Lean/fat mass development over 7 weeks treatment of mice with low fat diet (LFD), high fat diet (HFD) or HFD and defensin hBD2 (HFD + P2). (A) Lean mass development at week 1 and at week 7. (B) Fat mass development at week 1 and at week 7. A: Lean mass week -1 and week 7. N = 12. One-way ANOVA, Tukey post test. B: Fat mass week -1 and week 7. N = 12. One-way ANOVA, Tukey post test.

Figure 8: Glucose homeostasis in mice treated for 7 weeks with low fat diet (LFD), high fat diet (HFD) or HFD and defensin hBD2 (HFD + P2). (A) Insulin tolerance test (ITT). (B) Oral glucose tolerance test. (C) Glucose stimulated insulin secretion (GSIS). (D) 5-hour fasting insulin test.

A: Insulin Tolerance test 7 weeks post diet initiation. N = 6 per group. The HFDs are compared to the LFD reference group. Only statistically significant changes are depicted. Asterisks above the upper curve indicate difference between HFD and LFD.

B: Glucose Tolerance test 7 weeks post diet initiation. N = 11-12 per group. The HFDs are compared to the LFD reference group. Only statistically significant changes are depicted. Asterisks above the upper curve indicate difference between HFD and LFD and asterisks below the middle curve indicate differences between HFD +P2 and LFD.

C: Glucose Stimulated Insulin Secretion (during the GTT) 7 weeks post diet initiation. N = 11-12 per group. The HFDs are compared to the LFD reference group. Only statistically significant changes are depicted. Asterisks above the upper curve indicate differences between HFD and LFD. HFD + P2 and LFD are not statically significant at any time point.

D: 5h Fasting insulin 7 weeks after diet initiation. N = 11-12 per group. A-C: 2-way ANOVA, Dunnett post test. D: 1-way ANOVA, Tukey post test.

Figure 9A, 9B and 9C: Weight development (a) feed efficiency (b) and energy intake (c) over 10 weeks treatment of mice with low fat diet (low fat), high fat diet (high fat) or high fat diet and preventive treatment with defensin hBD2 (high fat + hBD-2). Significance: Low fat vs. High fat = A; Low fat vs. High fat + hBD-2 = B;
High fat vs. High fat + hBD-2 = C.

9A. Weight development. Two-way ANOVA with Tukey correction (matched values stacked)

9B. Feed efficiency (gram of gained weight adjusted for average food intake in the cage). One-way ANOVA with Tukey correction correction NB! n=4 due to co-caging. 9C. Energy intake. Two-way ANOVA with Tukey correction (matched values stacked)

Figure 10A, 10B and 10C: Fat as a percent of total body weight (a), liver weight in gram (b) and weight of epididymal fat (eWAT) in gram (c) over 10 weeks treatment of mice with low fat diet (low fat), high fat diet (high fat) or high fat diet and preventive treatment with defensin hBD2 (hBD-2).
Significance: Low fat vs. High fat = A; Low fat vs. High fat + hBD-2 = B; High fat vs. High fat + hBD-2 = C.

10A. Fat percentage of total body weight in different weeks. Two-way ANOVA with Tukey correction (matched values stacked).

10B. Weight of epididymal adipose tissue (visceral AT) at termination. One-way ANOVA with Tukey correction.

10C. Weight at termination. One-way ANOVA with Tukey correction

Figure 11A and 11B: Glucose homeostasis in mice treated for 10 weeks with low fat diet (low fat), high fat diet (high fat) or high fat diet and preventive treatment with defensin hBD2 (high fat + hBD-2). (a) Oral glucose tolerance test. (b) Glucose stimulated insulin secretion (GSIS).
Significance: Low fat vs. High fat = A; Low fat vs. High fat + hBD-2 = B; High fat vs. High fat + hBD-2 = C.

11A. Oral glucose tolerance test of week 7. Two-way ANOVA with Tukey correction (matched values stacked).

11B. Glucose-stimulated insulin secretion of week 7 taken during oGTT. Two-way ANOVA with Tukey correction (matched values stacked).

Figure 12A and 12B: Glucose homeostasis in mice treated for 10 weeks with low fat diet (low fat), high fat diet (high fat) or high fat diet and preventive treatment with defensin hBD2 (high fat + hBD-2). (a) Insulin tolerance test (ITT). (b) HOMA-IR. Significance: Low fat vs. High fat = A; Low fat vs. High fat + hBD-2 = B;
High fat vs. High fat + hBD-2 = C

12a. Insulin tolerance test of week 8. Two-way ANOVA with Tukey correction (matched values stacked).
12b. Homeostasis Model Assesment (HOMA) of week 9. One-way ANOVA with Tukey correction.

Figure 13A and 13B: Weight development (a) and weight change (b) over 10 week's treatment of mice with low fat diet (low fat), high fat diet (high fat) or high fat diet and intervention treatment with defensin hBD2 (high fat + hBD-2). Significance: Low fat vs. High fat = A; Low fat vs. High fat + hBD-2 = B; High fat vs. High fat + hBD-2 = C.

13A. Weight development. Two-way ANOVA with Tukey correction (matched values stacked).
13B. Weight change from week 13 at the end of the run-in period and the following 10 weeks on experimental diets. Two-way ANOVA with Tukey correction (matched values stacked).

Figure 14A and 14B: Fat as a percent of total body weight (a) and change in fat % from week 0-4 in gram (b) over 10 weeks treatment of mice with low fat diet (low fat), high fat diet (high fat) or high fat diet and intervention treatment with defensin hBD2 (hBD-2).
Significance: Low fat vs. High fat = A; Low fat vs. High fat + hBD-2 = B; High fat vs. High fat + hBD-2 = C-

14A. Fat percentage of total body weight in different weeks. Two-way ANOVA with Tukey correction (matched values stacked).
14B. Change of fat percentage from end of the run-in and 4 weeks on experimental diets. One-way ANOVA with Tukey correction.

Figure 15A and 15B: Liver weight in gram (a) and weight of epididymal fat (eWAT) in gram (b) over 10 week's treatment of mice with low fat diet (low fat), high fat diet (high fat) or high fat diet and intervention treatment with defensin hBD2 (hBD-2).
Significance: Low fat vs. High fat = A; Low fat vs. High fat + hBD-2 = B; High fat vs. High fat + hBD-2 = C.

15A. Weight of liver at termination. One-way ANOVA with Tukey correction.
15B. Weight of epididymal adipose tissue (visceral fat) at termination. One-way ANOVA with Tukey correction.

Figure 16A, 16B and 16C: Glucose homeostasis in mice treated for 10 weeks with low fat diet (low fat), high fat diet (high fat) or high fat diet and intervention treatment with defensin hBD2 (high fat + hBD-2). (a) Oral glucose tolerance test from cage 1 (b) Oral glucose tolerance test from mouse D1. (c) Insulin tolerance test (ITT).
Significance: Low fat vs. High fat = A; Low fat vs. High fat + hBD-2 = B; High fat vs. High fat + hBD-2 = C.

16A Oral glucose tolerance tests repeated biweekly from end of run-in period (Week 13-0) showing the first cage of High fat + hBD-2 group.
16B. Oral glucose tolerance tests repeated biweekly from end of run-in period (Week 13-0) showing ONLY mouse D1 of the High fat + hBD-2 group.
16C. Insulin tolerance test of week 9. Two-way ANOVA with Tukey correction (matched values stacked).

Figure 17A, 17B and 17C: Weight development (a) feed efficiency (b) and energy intake (c) over 10 week's treatment of mice with low fat diet (low fat), high fat diet (high fat) or high fat diet and preventive treatment with defensin HD5 (high fat + HD5). Significance: Low fat vs. High fat = A; Low fat vs. High fat + HD-5 = B; High fat vs. High fat + HD-5 = C.

17A. Weight development. Two-way ANOVA with Tukey correction (matched values stacked).
17B. Feed efficiency (gram of gained weight adjusted for average food intake in the cage). One-way ANOVA Tukey correction NB! n=4 due to co-caging.
17C. Energy intake. Two-way ANOVA with Tukey correction (matched values stacked).

Figure 18A, 18B and 18C: Fat as a percent of total body weight (a), liver weight in gram (b) and weight of epididymal fat (eWAT) in gram (c) over 10 week's treatment of mice with low fat diet (low fat), high fat diet (high fat) or high fat diet and preventive treatment with defensin HD5 (HD5).
Significance: Low fat vs. High fat = A; Low fat vs. High fat + HD-5 = B; High fat vs. High fat + HD-5 = C.

18A. Fat percentage of total body weight in different weeks. Two-way ANOVA with Tukey correction (matched values stacked).
18B. Weight of the liver at termination. One-way ANOVA Tukey correction
18C. Weight of epididymal adipose tissue (visceral AT) at termination. One-way ANOVA with Tukey correction.

Figure 19A and 19B: Glucose homeostasis in mice treated for 10 weeks with low fat diet (low fat), high fat diet (high fat) or high fat diet and preventive treatment with defensin HD5 (high fat + HD5). (a) Oral glucose tolerance test. (b) Glucose stimulated insulin secretion (GSIS).
Significance: Low fat vs. High fat = A; Low fat vs. High fat + HD-5 = B; High fat vs. High fat + HD-5 = C.

19A. Oral glucose tolerance test of week 7. Two-way ANOVA with Tukey correction (matched values stacked).
19B. Glucose-stimulated insulin secretion of week 7 taken during oGTT. Two-way ANOVA with Tukey correction (matched values stacked).

Figure 20A and 20B: Glucose homeostasis in mice treated for 10 weeks with low fat diet (low fat), high fat diet (high fat) or high fat diet and preventive treatment with defensin HD5 (high fat + HD5). (a) Insulin tolerance test (ITT). (b) HOMA-IR. Significance: Low fat vs. High fat = A; Low fat vs. High fat + HD-5 = B; High fat vs. High fat + HD-5 = C.

20A. Insulin tolerance test of week 8. Two-way ANOVA with Tukey correction (matched values stacked).
20B. Homeostasis Model Assesment (HOMA) of week 9. One-way ANOVA with Tukey correction.

Figure 21A and 21B. The figures illustrate changes from week 1 (Wk1) to week 10 (Wk10) for the four treatments in Example 1: High fat diet with HD5 - HF HD5; high fat diet with hBD2 - HD hBD2; High fat diet - no treatment: HF; Low fat diet: LF. Weighted unifrac analysis of microbiota at week 1 (21A) and week 10 (21B) i.e. relative abundance of bacterial species. The microbiota from mice fed a HFD plus HD5 gradually approached the bacterial flora of mice fed a LFD i.e. normalization of microbiota. Abbreviations: Wk 1 - week 1; W10-week 10.

Figure 21C. Illustration of the species of microbiota contributing to the change. The changes of microbiota were primarily driven by an increased abundance of Allobaculum and Lactobacillus and a decrease in abundance of clostridium. Allobaculum is a short chain fatty acid producing species. Lactobacillus is a bacteria with anti-inflammatory properties.

Figure 22A and 22B: Weight development (a) and weight change (b) over 10 week's treatment of mice with low fat diet (low fat), high fat diet (high fat) or high fat diet and intervention treatment with defensin HD5 (high fat + HD5). Significance: Low fat vs. High fat = A; Low fat vs. High fat + HD-5 = B; High fat vs. High fat + HD-5 = C.

22A. Weight development. Two-way ANOVA with Tukey correction (matched values stacked).
22B. Weight change from week 13 at the end of the run-in period and the following 10 weeks on experimental diets. Two-way ANOVA with Tukey correction (matched values stacked).

Figure 23A and 23B: Fat as a percent of total body weight (a) and change in fat % from week 0-4 in gram (b) over 10 week's treatment of mice with low fat diet (low fat), high fat diet (high fat) or high fat diet and intervention treatment with defensin HD5 (HD-5). Significance: Low fat vs. High fat = A; Low fat vs. High fat + HD-5 = B; High fat vs. High fat + HD-5 = C.

23A. Fat percentage of total body weight in different weeks. Two-way ANOVA with Tukey correction (matched values stacked).
23B. Change of fat percentage from end of the run-in and to week 4 on experimental diets. One-way ANOVA with Tukey correction.

Figure 24A and 24B: Liver weight in gram (a) and weight of epididymal fat (eWAT) in gram (b) over 10 week's treatment of mice with low fat diet (low fat), high fat diet (high fat) or high fat diet and intervention treatment with defensin HD5 (HD-5).
Significance: Low fat vs. High fat = A; Low fat vs. High fat + HD-5 = B; High fat vs. High fat + HD-5 = C.

24A. Weight of liver at termination. One-way ANOVA with Tukey correction.
24B. Weight of epididymal adipose tissue (visceral AT) at termination. One-way ANOVA with Tukey correction.

Figure 25A and 25B: Glucose homeostasis in mice treated for 10 weeks with low fat diet (low fat), high fat diet (high fat) or high fat diet and intervention treatment with defensin HD5 (high fat + HD5). (a) Oral glucose tolerance test from cage 2 (b) Insulin tolerance test (ITT).
Significance: Low fat vs. High fat = A; Low fat vs. High fat + HD-5 = B; High fat vs. High fat + HD-5 = C.

25A. Oral glucose tolerance tests repeated biweekly from end of run-in period (Week 13-0) showing the second cage of High fat + HD-5 group.
25B. Insulin tolerance test of week 9. Two-way ANOVA with Tukey correction (matched values stacked).

Figure 26. Schematic outline of the experimental set up for investigating the effects of a GLP-1 analog (Liraglutid) on mouse gut inflammation and microbiota. At week -40, the C57/Bl/6J DIO mice arrived. The mice were fed a high fat diet 60% fat, SSNIFF (Diet #D12492) or purina chow for 38 weeks to achieve an average body weight of 55 gram. From week -2 the mice were single housed. Faecal samples were collected on day -1 and 27 for 16S RNA analysis. Samples from ilium were collected 2 cm from caecum at day 28.

Figure 27A. Results of microbiome analysis from example 4. Mice were treated for four weeks with Liraglutid or vehicle (DIO Vehicle).
Unweighted unifrac analysis of microbiota at day -1 and day 28 illustrates changes in the microbiome of the two treatment groups from the start till the end of the experiment.
The microbiota from mice fed a HFD plus Liraglutid gradually approached the bacterial flora of mice fed a LFD, while the microbiota of the vehicle treated mice did not change during the study.

Figure 27B. The changes of microbiota with species of microbiota added. The changes were primarily driven by an increased abundance of *Akkermansia* and *Alloprevotella. Akkermansia* is a short chain fatty acid producing species.

Figure 28. Pharmacokinetic data following oral administration of 4 mg/kg hBD-2 to female NMRI mice. The Y-axis shows hBD2 in μg/g tissue. The results are given as group mean +/-SEM.

Figure 29. Pharmacokinetic data for hBD-2 following subcutaneous (SC) and intravenous (IV) administration of 1 mg/kg respectively. The Y-axis shows hBD2 in μg/mL. The different curves represent different experiments and detection methods (HPLC and ELISA).

Figure 30. Pharmacokinetic data for "hBD-2-albumin fusion N-terminal" following subcutaneous and intravenous administration of 16.5 mg/kg respectively. The Y-axis shows the concentration of the fusion protein in μg/mL. The results are the mean of 4 mice/sampling time +/- SD.

Figure 31. Pharmacokinetic data for "hBD-2-albumin fusion C-terminal" following subcutaneous and intravenous administration of 16.5 mg/kg respectively. The Y-axis shows the concentration of the fusion protein in μg/mL. The results are the mean of 4 mice/sampling time +/- SD.

## Detailed description

The invention is defined by the appended claims. Any embodiment not falling under the scope of the appended claims does not form part of the invention.

## Definitions:

Defensin: The term "defensin" as used herein refers to polypeptides recognized by a person skilled in the art as belonging to the defensin class of antimicrobial peptides. The defensins belong to the alfa defensin class or to the beta defensin class. Examples of defensins include human intestinal alpha defensin 5 (HD5; SEQ ID NO. 8); human alpha defensin 6 (HD6; SEQ ID NO. 9); human neutrophil peptide 1 (HNP-1); human neutrophil peptide 2 (HNP-2); human neutrophil peptide 3 (HNP-3), all belonging to the alfa defensin class; and also human beta defensin 1 (hBD1; SEQ ID NO. 4); human beta defensin 2 (hBD2; SEQ ID NO. 5); human beta defensin 3 (hBD3; SEQ ID NO. 6); human beta defensin 4 (hBD4; SEQ ID NO. 7), chimpanzee beta defensin 2 (SEQ ID NO: 10), macaque beta defensin 2 (SEQ ID NO: 11), orangutan beta defensin 2 (SEQ ID NO: 3), mouse beta defensin 3 (SEQ ID NO: 12), horse beta defensin 2 (SEQ ID NO: 13), porcine beta defensin 1 (SEQ ID NO: 14), goat beta defensin 2 (SEQ ID NO: 15), bovine beta defensin 2 (SEQ ID NO: 1), chicken beta defensin 2 (SEQ ID NO: 2) human LL37 (SEQ ID NO: 16), derived from human cathelicidin, and truncated hBD2 (SEQ ID NO: 17) belonging to the beta defensin class. Defensins may be glycosylated and defensins may be proteolytically cleaved into smaller bioactive fragments. Glycosylated defensins and defensin fragments are also included within the scope of the present disclosure.

Defensins are expressed as precursors and are processed by cleavage of the signal peptide and in some cases pro-peptides as well before secretion into the extracellular space. The above-identified sequences represent the predicted mature bioactive defensins. It will be understood by one of skill in the art that processing may differ from cell to cell and that the resulting secreted mature peptide may differ by one or two C- or N-terminal amino acids from the predicted sequences and still retain their bioactivity.

Gut: The gut is a tube used by animals to transfer food to the digestion organs and it includes the digestion organs themselves. Human gut as used herein refers to a digestive system composed of mouth, oesophagus, stomach, duodenum, jejunum, ileum, cecum, colon, rectum, and anal canal. Some embodiments of the present disclosure refer to parts of the human gut and in particular to mouth, oesophagus, stomach, duodenum, jejunum, ileum, cecum, colon, rectum, and anal canal. Other embodiments of the present disclosure refer to all these parts except the colon. Ruminant gut as referred herein is a gut composed of mouth, oesophagus, stomach, duodenum, jejunum, ileum, cecum, colon, rectum, and anal canal, but characterized by the fact that the stomach is divided in four compartments, rumen, reticulum, omasum, and abomasum. Poultry gut as referred herein is a gut composed of oesophagus, stomach, duodenum, jejunum, ileum, cecum, colon, rectum, and anal canal, but characterized by the fact that the stomach is divided in proventriculus or true stomach and gizzard. In some cases, a muscular pouch along the oesophagus called a crop is present.

"Glucagon-like peptide-1. GLP-1 is a neuropeptide and an incretin derived from the transcription product of the proglucagon gene. The major source of GLP-1 in the periphery is the intestinal L cell, that secretes GLP-1 as a gut hormone. The biologically active forms of GLP-1 are: GLP-1-(7-37) and GLP-1-(7-36)NH2. These peptides result from selective cleavage of the proglucagon molecule.

GLP-1 secretion by ileal L cells is dependent on the presence of nutrients in the lumen of the small intestine. The secretagogues (agents that cause or stimulate secretion) of this hormone include major nutrients like carbohydrates, proteins and lipids. Once in the circulation, GLP-1 has a half-life of less than 2 minutes, due to rapid degradation by the enzyme dipeptidyl peptidase-4.

GLP-1 is a potent antihyperglycemic hormone, inducing the beta β-cells of the pancreas to release the hormone insulin in response to rising glucose, while suppressing glucagon secretion. Such glucose-dependent action is particularly attractive because an unregulated release of insulin, when the plasma glucose concentration is in the normal fasting range, or poorly-timed insulin injections, can cause a dangerous fall in blood glucose - hypoglycemia. This does not happen as a result of GLP-1 because GLP-1 no longer stimulates the β-cells to release more insulin when blood glucose levels are in the fasting range. In addition, GLP-1 inhibits gastric secretion and motility. This delays and protracts carbohydrate absorption and contributes to a satiating effect.

Identity: The relatedness between two amino acid sequences or between two nucleotide sequences is described by the parameter "identity".

The degree of identity between two amino acid sequences is determined using the Needleman-Wunsch algorithm (Needleman and Wunsch, 1970, J. Mol. Biol. 48: 443-453) as implemented in the Needle program of the EMBOSS package (Rice *et al.,* 2000, http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

Lean growth promotion: The term "lean growth promotion" or "lean growth enhancement" as used herein refer to feeding of livestock or domestic animals e.g. cows, pigs, sheep, goats, horses, ducks, geese, pigeons, turkeys, quails and chickens in the meat production industry where fast but lean increase of body mass is the objective.

Livestock: cattle, horses, poultry, and similar animals kept for domestic use.

Normal microbiota: The term "normal microbiota" is used herein to indicate a microbiota that is not dysbiotic. Normal microbiota is characterized by having a large gene and phylae richness. Normal microbiota is characterized by comprising bacteria belonging to the genera *Bacterioidetes, Faecalibacterium, Roseburia, Blautia, Ruminococcus, Coprococcus, Bifidobacterium, Methanobrevibacter, Lactobacillus, Coprococcus, Clostridium, Akkermansia, Eubacterium*

Treatment: The terms "treatment" and "treating" as used herein refer to the management and care of a patient for the purpose of combating a condition, disease or disorder. The term is intended to include the full spectrum of treatments for a given condition from which the patient is suffering, such as administration of the active compound for the purpose of: alleviating or relieving symptoms or complications; delaying the progression of the condition, disease or disorder; curing or eliminating the condition, disease or disorder; and/or preventing the condition, disease or disorder, wherein "preventing" or "prevention" is to be understood to refer to the management and care of a patient for the purpose of hindering, reducing or delaying the development of the condition, disease or disorder, and includes the administration of the active compounds to prevent or reduce the risk of the onset of symptoms or complications. The patient to be treated is preferably a mammalian, in particular a human being. The patients to be treated can be of various ages.

Subject, patient: A subject is an individual of one of the species of mammals or poultry disclosed herein. A patient is a subject, which has been diagnosed with a particular disorder.

**Mammalian and poultry alfa defensins and mammalian and poultry beta defensins**

This disclosure relates to uses of defensins, mammalian and poultry alfa and/or beta defensins, such as bovine, porcine, sheep, mouse, monkey, horse and poultry such as chicken, turkey, duck, goose, quail, pigeon mouse, monkey or human beta defensins, more preferably Hominidae, more preferably human alfa and/or beta defensins in the treatment of indications as herein disclosed, including but not limited to gut inflammation, or colorectal cancer, or an endocrine, nutritional, metabolic or cardiovascular disease.

The LL37 fragment of cathelicidin is also contemplated for uses according to the disclosure. LL37 has the sequence of SEQ ID NO 16.

According to particularly preferred embodiments of the present disclosure, the defensins are alpha or beta defensins.

In an embodiment of the present disclosure, LL37, the mammalian alfa and/or beta defensins have a degree of identity of at least 80%, preferably at least 85%, more preferably at least 90%, and most preferably at least 95% to any of the amino acid sequences of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 and/or SEQ ID NO 17. In another embodiment of the present disclosure, a defensin differs from one of the SEQ ID NO:1-17 by less than 10, such as less than 8, for example less than 5, such as less than 4, for example less than 3, such as less than 2 amino acids.

In a preferred embodiment of the present disclosure, the human alfa defensins consist of (alfa defensin 5 (SEQ ID NO: 8) and/or alfa defensin 6 (SEQ ID NO:9). In a preferred embodiment of the present disclosure, the mammalian beta defensins consist of human beta defensin 1 (SEQ ID NO:4), human beta defensin 2 (SEQ ID NO:5), human beta defensin 3 (SEQ ID NO:6), human beta defensin 4 (SEQ ID NO:7), and/or truncated human beta defensin 2 (SEQ ID NO 17).

In a preferred embodiment of the present disclosure, a human alfa defensin has a degree of identity of at least 80%, preferably at least 85%, more preferably at least 90%, and most preferably at least 95% to the amino acid sequence of SEQ ID NO:8. In a preferred embodiment of the present disclosure, the human mammalian alfa defensins consist of alfa defensin 5 (SEQ ID NO:8). In a preferred embodiment of the present disclosure, the human beta defensin has a degree of identity of at least 80%, preferably at least 85%, more preferably at least 90%, and most preferably at least 95% to the amino acid sequence of SEQ ID NO:5. In a preferred embodiment of the present disclosure, the human beta defensin consists of human beta defensin 2 (SEQ ID NO:5). Another preferred human beta defensin is truncated human beta defensin 2 (SEQ ID NO:17). Truncated hBD2 (SE ID NO:17) has anti-inflammatory effects comparable to those of hBD2 (SEQ ID NO:5) (WO 2013/026794).

For species other than human beings, the subjects are preferably treated with a defensin originating from the same or a related species or a defensin sharing at least least 80%, preferably at least 85%, more preferably at least 90%, and most preferably at least 95% to the amino acid sequence of a defensin from that same species (for example the defensin having an amino acid sequence selected from SEQ ID NO:1-3 and 10-15). For example, it is conceivable that poultry can be treated with an orthologous defensin from the same or another bird species.

In yet another embodiment of the present disclosure, the mammalian alfa defensins comprise of human alfa defensins and/or mouse alfa defensins, and functionally equivalent variants thereof. Preferably, the mammalian alfa defensin is a human alpha defensin, which may consist of human alfa defensin 5, human alfa defensin 6 and functionally equivalent variants thereof. More preferably, the mammalian alfa defensins consist of human alfa defensin 5, and functionally equivalent variants or orthologues thereof.

In yet a further embodiment of the present disclosure, the mammalian beta defensins consist of human beta defensins and/or mouse beta defensins, and functionally equivalent variants thereof. Preferably, the mammalian or poultry beta defensins consist of human beta defensin 1, human beta defensin 2, human beta defensin 3, human beta defensin 4, Chimpanzee beta defensin 2, Macaque beta defensin 2, and mouse beta defensin 3, orangutan beta defensin 2, horse beta defensin 2, porcine beta defensin 1, goat beta defensin 2, bovine beta defensin 2, chicken beta defensin 2 and functionally equivalent variants thereof. More preferably, the mammalian beta defensins comprise of human beta defensin 1, human beta defensin 2, human beta defensin 3, human beta defensin 4 and functionally equivalent variants thereof. Even more preferably, the mammalian beta defensins consist of human beta defensin 2, and functionally equivalent variants or orthologues thereof.

In one embodiment of the present disclosure, the methods comprise administration of an effective amount of at least one mammalian or poultry α-defensin to a subject in need of such treatment. In other embodiments of the present disclosure, the provided methods comprise administration of an effective amount of at least one mammalian or poultry β-defensin to a subject in need of such treatment. In a further embodiment of the present disclosure, the provided methods comprise administration of an effective amount of at least one mammalian or poultry α-defensin and at least one mammalian or poultry β-defensin to a subject in need of such treatment. A preferred embodiment of the present disclosure provides administration of the mammalian alfa defensin HD5 and/or the mammalian beta defensin hBD-2

A "functionally equivalent variant" of a mammalian (e.g. human) or poultry alfa or beta defensin is a modified mammalian (e.g. human) or poultry alfa or beta defensin exhibiting approximatively the same effect on microbiota in the intestine as the parent mammalian (e.g. human) or poultry alfa and/or beta defensins. A functionally equivalent variant of a mammalian (e.g. human) or poultry defensin may comprise 1-5 amino acid modifications, preferably 1-4 amino acid modifications, more preferably 1-3 amino acid modifications, most preferably 1-2 amino acid modification(s), and in particular one amino acid modification, as compared to the mammalian (e.g. human) or poultry defensin amino acid sequence. Preferably, for beta mammalian defensins, compared to human beta defensin 2, having SEQ ID NO 5.

The term "modification" means herein any chemical modification of a mammalian (e.g. human) or poultry defensin. The modification(s) can be substitution(s), deletion(s) and/or insertions(s) of the amino acid(s) as well as replacement(s) of amino acid side chain(s); or use of unnatural amino acids with similar characteristics in the amino acid sequence. In particular the modification(s) can be amidations, such as amidation of the C-terminus.

Preferably, amino acid modifications are of a minor nature, that is conservative amino acid substitutions or insertions that do not significantly affect the folding and/or activity of the polypeptide; single deletions; small amino- or carboxyl-terminal extensions; or a small extension that facilitates purification by changing net charge or another function, such as a poly-histidine tag, an antigenic epitope or a binding domain. In one embodiment of the present disclosure the small extension, such as a poly-histidine tag, an antigenic epitope or a binding domain is attached to the mammalian (e.g. human) or poultry alfa or beta defensin through a small linker peptide of up to about 20-25 residues and said linker may contain a restriction enzyme cleavage site. The Clustal W alignments in Figures 2 to 5 can be used to predict which amino acid residues can be substituted without substantially affecting the biological activity of the protein. The sequences were aligned using Clustal W 2.1 (http://www.geno,me.jp/tools/clustalw/) and the following settings: Gap Open Penalty: 10, Gap Extension Penalty: 0,05, Weight Transition: NO, Hydrophilic Residues for Proteins: GPSNDQE, Hydrophilic Gaps: YES, Weight Matrix: BLOSUM (for PROTEIN). Substitutions within the following group (Clustal W, 'strong' conservation group) are to be regarded as conservative substitutions:

-S,T,A; N,E,Q,K; N,H,Q,K; N,D,E,Q; Q,H,R,K; M,I,L,V; M,I,L,F; H,Y; F,Y,W.

Substitutions within the following group (Clustal W, 'weak' conservation group) are to be regarded as semi-conservative substitutions:

-C,S,A; A,T,V; S,A,G; S,T,N,K; S,T,P,A; S,G,N,D; S,N,D,E,Q,K; N,D,E,Q,H,K; N,E,Q,H,R,K; V,L,I,M; H,F,Y.

Examples of conservative substitutions are substitutions made within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter specific activity are known in the art and are described, for example, by Neurath and Hill (1979). The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In addition to the 20 standard amino acids, non-standard amino acids (such as 4-hydroxyproline, 6-*N*-methyl lysine, 2-aminoisobutyric acid, isovaline, and alpha-methyl serine) may be substituted for amino acid residues of a wild-type polypeptide. A limited number of non-conservative amino acids, amino acids that are not encoded by the genetic code, and unnatural amino acids may be substituted for amino acid residues. "Unnatural amino acids" have been modified after protein synthesis, and/or have a chemical structure in their side chain(s) different from that of the standard amino acids. Unnatural amino acids can be chemically synthesized, and preferably, are commercially available, and include pipecolic acid, thiazolidine carboxylic acid, dehydroproline, 3- and 4-methylproline, and 3,3-dimethylproline.

Essential amino acids in a mammalian or poultry alfa and/or beta defensin can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for biological activity (*i.e.,* activity against an inflammatory bowel disease and/or suppression of TNF-alpha activity) to identify amino acid residues that are critical to the activity of the molecule. See also, Hilton et al., 1996, J. Biol. Chem. 271: 4699-4708. The identities of essential amino acids can also be inferred from analysis of identities with polypeptides which are related to mammalian or poultry alfa and/or beta defensins (see Clustal W alignment in figures 2 to 5).

Single or multiple amino acid substitutions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413;

or WO 95/22625. Other methods that can be used include errorprone PCR, phage display (*e.g.,* Lowman et al., 1991, Biochem. 30:10832-10837; U.S. Patent No. 5,223,409; WO 92/06204), and region-directed mutagenesis (Derbyshire et al., 1986, Gene 46:145; Ner et al., 1988, DNA 7:127).

When the result of a given substitution cannot be predicted with certainty, the derivatives may be readily assayed according to the methods described herein above to determine the presence or absence of biological activity.

Defensins as disclosed herein may be subject to glycosylation. Furthermore, it is known in the art that naturally occurring defensins may be subject to proteolytical processing and be cleaved into smaller bioactive fragments. Glycosylated defensins and bioactive fragments of defensins are included within the present disclosure. Furthermore included within the scope of the present disclosure are inducers of defensins. It is known in the art that e.g. Vitamin D and *E. coli Nissle* can induce secretion of defensins and can thus be used to treat the indications as described herein.

**Combinations of alpha and beta-defensins**

In one aspect of the present disclosure there is provided a composition comprising at least one mammalian or poultry α-defensin and at least one mammalian or poultry β-defensin. As demonstrated in Examples 1 and 3 alpha and beta defensins can be administered orally. An exemplary alpha-defensin, HD5, in particular decreases ectopic lipid accumulation and an exemplary beta-defensin, hBD2, in particular improves the glucoregulatory pathway. Therefore the combination of an alpha and beta defensin may lead to particularly effective treatment of obesity and the endocrine indications as herein described.

The mammalian α-defensin may be selected from the group consisting of HD5, and HD6, and the at least one mammalian β-defensin may be selected from hBD-1, hBD-2, hBD-3 and hBD-4.

Preferably, the composition comprises HD5 and hBD-2.

The composition may further comprise a pharmaceutically acceptable excipient and being sterile and be formulated as a sterile and isotonic solution.

The ration between alpha and beta-defensin may be any ration. In some embodiments of the present disclosure, the composition comprises essentially equal amounts of at least one mammalian or poultry α-defensin and at least one mammalian or poultry β-defensin on a molarity basis or weight basis or on a mg/mL basis.

**Long-acting defensins**

The half-life of an α- or β-defensin may be extended by fusing or conjugating the α- or β-defensin with another molecule i.e. constructing a long acting biologically active α- or β-defensin linked to a pharmaceutically acceptable molecule providing an *in vivo* plasma half-life of the α- or β-defensin, which is increased substantially compared to the *in vivo* plasma half-life of the α- or β-defensin administered in the same manner as the α- or β-defensin.

A long acting biologically active α- or β-defensin comprising a mammal α-defensin or analog thereof or a mammal β-defensin or analog thereof linked to a pharmaceutically acceptable molecule selected from a molecule having binding to a mammal neonatal Fc receptor, transferrin or a CH3(CH2)nCO-, wherein n is 8 to 22 or a polymer.

The α- or β-defensin agonist may also be of non-mammalian origin, and may be selected from small organic molecules, peptides, polypeptides and proteins.

The α- or β-defensin agonist may be linked to the pharmaceutically acceptable molecule in various ways as described in the prior art literature, such as without limitation chemical coupling through a bifunctional linker, gene technologically by coupling the N-terminal or C-terminal of the defensin, such as α-defensin or β-defensin, to the pharmaceutically acceptable molecule, such as albumin or albumin analog. In particular, the N-terminal of albumin or an albumin analogue, e.g. human albumin, can be coupled to the C-terminal of an α-defensin or β-defensin, or the N-terminal of an α- or β-defensin; or the C-terminal of albumin, e.g. human albumin, can be coupled to the C-terminal of an α-defensin or β-defensin, or the N-terminal of an α- or β-defensin. A linker sequence can be inserted between the albumin and the α- or β-defensin chain. The α- or β-defensin agonist may be linked to the pharmaceutically acceptable molecule through a stable linker or a more labile linker. Several linkers are known in the art, including bifunctional PEG molecules (e.g. see Paige et.al Pharmaceutical Research, vol. 12, no. 12, 1995), hydrolysable linkers (Shechter et al. Bioconjugate Chem. 2005,16: 913- 920 and International Journal of Peptide Research and Therapeutics, Vol. 13, Nos. 1-2, June 2007 and WO2009095479), PDPH and EMCH see e.g. in WO2010092135. In the special case where chemical conjugation (linking of two or more molecules) of the α- or β-defensin agonist, to the pharmaceutically acceptable molecule, strongly reduce the functional α- or β-defensin activity, it may be preferable to use a more labile linker that can release the functional α- or β-defensin agonist.

Half-life extension may also be accomplished through acylation of the peptide backbone with a spacer e.g. γ-L-glutamyl spacer and a C-18 fatty di-acid chain to Lysine. The fatty di-acid site chain and the spacer mediate a strong but reversible binding to albumin, slowing release from the injection site and reducing renal clearance.

**Methods and Uses**

As demonstrated in example 4, administration of Liraglutid, a GLP-1 analog leads to changes in the microflora in obese mice fed a high fat dies. The changes are towards a more healthy or normal microflora inter alia with an increase in bacterial species that favour production of short chain fatty acids. Therefore, the inventors contemplate treatment of a dysbiotic microflora and other uses as described herein by administration of GLP-1 or a GLP-1 analog.

Preferably, GLP-1 or GLP1 analogs are administered parenterally through either subcutaneous or intramuscular administration. The GLP-1 analog may be selected from exenatide, liraglutide, lixisenatide, albiglutide, and dulaglutide.

Human alfa defensin 5 and human beta defensin 2 are found to be able to maintain or stabilize a normal microbiota in the intestine and even treat or normalize a dysbiotic microbiota in the intestine; thus showing potent activity as a medicament for treatment of colorectal cancer, gut inflammation, endocrine, nutritional, metabolic or cardiovascular diseases or as lean growth promoters. Therefore, one aspect of the present disclosure provides methods for treatment of gut inflammation in general or for treatment of colorectal cancer, an endocrine, nutritional, metabolic or cardiovascular disease by administering an effective amount of a mammalian $\alpha$-defensin and/or $\beta$-defensin to a subject in need of such treatment. Examples of such diseases are type 1 diabetes, type 2 diabetes, metabolic syndrome, systemic low grade inflammation, obesity, insulin resistance, glucose intolerance and cardiovascular disease.

In particular, it has been demonstrated that HD5 and hBD2 can be used to treat insulin resistance improving insulin sensitivity and glucose tolerance as well as treating or preventing obesity. HD5 in particular may decrease ectopic lipid accumulation whereas hBD2 in particular may improve the glucoregulatory efficacy.

Prevention of obesity or induction of weight loss or prevention of weight gain preferably involves a reduction in or prevention of accumulation of visceral fat, a reduction or prevention of an increase of the fat percentage, or a reduction of or prevention of increase in waist circumference.

The provided methods can treat or prevent gut inflammation by changing bacterial phenotypes through a change at the transcriptional level as well as structure and composition of the intestinal bacterial flora of a subject affected by one of the said conditions as described herein.

The provided methods can treat colorectal cancer, endocrine, nutritional, metabolic or cardiovascular diseases by changing structure and composition of the intestinal microbiota and thus the metabolome of a subject affected by one of the said conditions as described herein.

One aspect of the present disclosure provides methods for treatment of gut inflammation in human, wherein the inflammation is located in the mouth, oesophagus, stomach, duodenum, jejunum, ileum, cecum, colon, rectum, and/or anal canal of an animal, by administering an effective amount of a mammalian $\alpha$-defensin and/or $\beta$-defensin to a subject in need of such treatment. Preferably the defensin is human alpha defensin. In other preferred embodiments of the present disclosure the defensin is a human beta-defensin, preferably hBD2, and inflammation is reduced in the mouth, oesophagus, stomach, duodenum, jejunum, ileum, cecum, rectum, and/or anal canal.

One aspect of the present disclosure provides methods for treatment of gut inflammation by administering an effective amount of a mammalian or poultry $\alpha$-defensin and $\beta$-defensin to a subject in need of such treatment.

One aspect of the present disclosure provides methods for stabilizing or maintaining a normal microbiota in the intestine. Another aspect of the present disclosure provides methods for treatment or normalization of a dysbiotic microbiota in the intestine by administering an effective amount of a mammalian or poultry $\alpha$-defensin and/or $\beta$-defensin and/or GLP-1/GLP-1 analog to a subject in need of such treatment.

A further aspect of the present disclosure provides methods for increasing gene richness of the intestinal microbiota by administering an effective amount of a mammalian or poultry $\alpha$-defensin and/or $\beta$-defensin and/or GLP-1/GLP-1 analog to a subject in need of such treatment.

One aspect of the present disclosure provides methods for increasing the number of phylae of the intestinal microbiota by administering an effective amount of a mammalian or poultry $\alpha$-defensin and/or $\beta$-defensin and/or GLP-1/GLP-1 analog to a subject in need of such treatment.

One aspect of the present disclosure provides methods for increasing the butyrate production and/or decreasing the acetate production from the intestinal microbiota by administering an effective amount of a mammalian or poultry $\alpha$-defensin and/or $\beta$-defensin and/or GLP-1/GLP-1 analog to a subject in need of such treatment.

One aspect of the present disclosure provides methods for increasing production of short chain fatty acids from the intestinal microbiota by administering an effective amount of a mammalian or poultry $\alpha$-defensin and/or $\beta$-defensin and/or GLP-1/GLP-1 analog to a subject in need of such treatment.

Some aspects of the present disclosure provide methods for increasing the number of bacteria belonging to a genus selected from a group composed of *Bacterioidetes, Faecalibacterium, Roseburia, Blautia, Ruminococcus, Coprococcus, Bifidobacterium, Methanobrevibacter, Lactobacillus, Clostridium, Allobaculum, Alloprevotella, Akkermansia, Eubacterium* in the intestinal microbiota by administering an effective amount of a mammalian or poultry $\alpha$-defensin and/or $\beta$-defensin and/or GLP-1/GLP-1 analog to a subject in need of such treatment. Preferably the bacteria are *Allobaculum, Alloprevotella, Akkermansia,* or *Lactobacillus.*

In a preferred embodiment of the present disclosure, methods for increasing the number of bacteria selected from a group composed of *Bacteroides vulgatus, Bacteroides caccae, Faecalibacterium prausnitzii, Roseburia intestinalis, Blautia hansenii, Ruminococcus gnavus, Coprococcus comes, Clostridium nexile, Clostridium bolteae, Bifidobacterium longum, Bifidobacterium bifidum, Bifidobacterium adolescentis, Bifidobacterium dentum, Lactobacillus gasseri, Lactobacillus plantarum, Akkermansia muciniphila, Eubacterium rectale* are provided. The provided methods increase the number of bacteria that are typical of a healthy gut microbiota.

One aspect of the present disclosure provides methods for decreasing the number of bacteria belonging to a genus selected from a group composed of *Bacteroidetes fragilis, Sutturella wadsworthia, Veillonella parvula, Escherichi coli, Haemophilus parainfluenzae, Fusobacterium nucleatum, Eikenella corodens, Gemella moribillum* in the intestinal microbiota by administering an effective amount of a mammalian or poultry α-defensin and/or β-defensin and/or GLP-1/GLP-1 analog to a subject in need of such treatment. The provided methods decrease the number of bacteria that are typical of a dysbiotic microbiota in the intestine of a subject in need of treatment.

Therefore, the described gut inflammation, colorectal cancer, endocrine, nutritional, metabolic or cardiovascular diseases that can be treated using the methods disclosed in preferred embodiments of the present disclosure are characterized by a dysbiotic microbiota in the intestine of the subjects in need of the treatment. In some embodiments of the present disclosure, a dysbiotic microbiota in the intestine of a subject in need of treatment provided by the disclosed methods has low gene richness. In other embodiments of the present disclosure, a dysbiotic microbiota in the intestine of a subject in need of treatment provided by the disclosed methods has a low number of phylae. In other embodiments of the present disclosure, a dysbiotic microbiota in the intestine of a subject in need of treatment provided by the disclosed methods has an increased production of acetate from the microbiota. By the disclosed methods the increased acetate production may be reduced in favour of butyrate production.

In preferred embodiments of the present disclosure, a dysbiotic microbiota in the intestine of a subject in need of treatment provided by the disclosed methods has a low number of bacteria belonging to a genus selected from a group composed of *Bacterioidetes, Faecalibacterium, Roseburia, Blautia, Ruminococcus, Coprococcus, Bifidobacterium, Methanobrevibacter, Lactobacillus, Clostridium, Allobaculum, Alloprevotella Akkermansia,* and *Eubacterium.* In more preferred embodiments of the present disclosure, a dysbiotic microbiota in the intestine of a subject in need of treatment provided by the disclosed methods has a low number of bacteria selected from a group composed of *Bacteroidetes vulgatus, Bacteroides caccae, Faecalibacterium prausnitzii, Roseburia intestinalis, Blautia hansenii, Ruminococcus gnavus, Coprococcus comes, Clostridium nexile, Clostridium bolteae, Bifidobacterium longum, Bifidobacterium bifidum, Bifidobacterium adolescentis, Bifidobacterium dentum, Lactobacillus gasseri, Lactobacillus plantarum, Akkermansia muciniphila, Eubacterium rectale.* In further embodiments of the present disclosure, a dysbiotic microbiota in the intestine of a subject in need of treatment has a high number of bacteria selected from a group composed of *Bacteroides fragilis, Sutturella wadsworthia, Veillonella parvula, Escherichi coli, Haemophilus parainfluenzae, Fusobacterium nucleatum, Eikenella corodens, Gemella moribillum.*

The methods disclosed in preferred embodiments of the present disclosure can, via administration of at least a mammalian or poultry alfa defensin and/or at least a mammalian or poultry beta defensin and/or at least a GLP-1/GLP-1 analog treat a dysbiotic microbiota and metabolome

The disclosed methods can be used for treatment, prevention or normalization of a dysbiotic microbiota and/or metabolome in the intestine of a subject that has undertaken and/or is undertaking an antibiotic treatment or chemotherapy or immunotherapy or immunosuppressive therapy or another treatment that has negative effects on the intestinal microbiota.

The disclosed methods can also be used for treatment, prevention or normalization of a dysbiotic microbiota in the mouth of a subject such as a subject affected by periodontitis including gingivitis. Periodontitis can be caused by smoking and stress and can also be drug-induced, e.g. by chemotherapy, immunotherapy and immunosuppressive therapy.

The subject in need of the treatment provided by the disclosed methods is affected by gut inflammation or colorectal cancer or an endocrine, nutritional, metabolic or cardiovascular disease. In one embodiment of the present disclosure, the subject in need of the treatment has BMI of 25 or more, such as 30 or more, for example 35 or more, such as 40 or more. In another embodiment of the present disclosure, the subject in need of the treatment has a waist/hip ratio of at least 0.80, for example 0.80-0.84, such as at least 0.85 (female) or at least 0.90, for example 0.9-0.99, such as above 1.00 (male). In a further embodiment of the present disclosure, the subject in need of the treatment has fasting blood glucose of at least 6.1 mmol/l, for example at least 7.0 mmol/l. In an even further embodiment of the present disclosure, the subject in need of the treatment has a glycated haemoglobin ($HbA_{1C}$) level of at least 42 mmol/mol Hb, such as between 42 and 46 mmol/mol Hb, such as at least 48 mmol/mol Hb.

The subject in need of the treatment provided by the disclosed methods may present one or more of the following symptoms:

- Elevated blood pressure: ≥ 140/90 mmHg;

- Dyslipidemia: triglycerides (TG): ≥ 1.695 mmol/L and high-density lipoprotein cholesterol (HDL-C) ≤ 0.9 mmol/L (male), ≤ 1.0 mmol/L (female);
- Central obesity: waist:hip ratio > 0.90 (male); > 0.85 (female), or body mass index > 30 kg/m$^2$; and
- Microalbuminuria: urinary albumin excretion ratio ≥ 20 μg/min or albumin:creatinine ratio ≥ 30 mg/g.

In one embodiment of the present disclosure, the administration of at least one mammalian or poultry α-defensin and/or at least one mammalian or poultry β-defensin, according to the disclosed methods, is generally oral.

Mammalian or poultry alfa and beta defensins can be employed therapeutically in compositions formulated for administration by any conventional route. In one embodiment of the present disclosure, mammalian and poultry alfa and/or beta defensins are administered orally. It is known that human beta-defensin 2 can be administered orally to treat inflammatory bowel disease in the colon. The current inventors have surprisingly demonstrated that also a human alpha defensin, HD5 can be administered orally and that it maintains its bioactivity in the gut, despite passing through the acidic stomach.

Oral administration is normally for enteral drug delivery, wherein the agent is delivered through the enteral mucosa. However, as demonstrated by the current inventors, hBD2 is not absorbed from the gut to any detectable extent. It is expected that other defensins are also not absorbed from the gut.

In one embodiment of the present disclosure, mammalian and poultry alfa and/or beta defensins are administered subcutaneously. In particular it is contemplated that hBD2 and HD5 may be administered subcutaneously.

Within some embodiments of the present disclosure, compositions, of preferred embodiments of the present disclosure may be formulized as a lyophilizate, utilizing appropriate excipients that provide stability as a lyophilizate, and subsequent to rehydration.

Pharmaceutical or animal feed compositions containing a mammalian alfa defensin and/or a mammalian beta defensin, such as a human alfa defensin and/or a human beta defensin, can be manufactured according to conventional methods, e.g., by mixing, granulating, coating, dissolving or lyophilizing processes. In a preferred embodiment of the present disclosure, pharmaceutical compositions containing a mammalian alfa defensin and/or a mammalian beta defensin are formulated as a sterile and isotonic solution.

The provided pharmaceutical compositions comprise, in one embodiment of the present disclosure, at least one mammalian alfa defensin. Examples of mammalian alfa defensins are HD5 and HD6. In a preferred embodiment of the present disclosure, the compositions comprise the mammalian alfa defensin HD5. The pharmaceutical compositions comprise, in another embodiment of the present disclosure, at least one mammalian beta defensin. Examples of mammalian beta defensins are hBD1, hBD2, hBD3 and hBD4. In a preferred embodiment of the present disclosure, the compositions comprise the mammalian beta defensin hBD2. The pharmaceutical compositions comprise, in a further embodiment of the present disclosure, at least one mammalian alfa defensin and at least one mammalian beta defensin. Examples of mammalian alfa defensins are HD5 and HD6. Examples of mammalian beta defensins are hBD1, hBD2, hBD3 and hBD4. In a preferred embodiment of the present disclosure, the compositions comprise the mammalian alfa defensin HD5 and the mammalian beta defensin hBD2. In other embodiments of the present disclosure the compositions or feed compositions comprise one or more non-human defensins selected from defensins having an amino acid sequence selected from SEQ ID NO: 1-3 and 10-17 as well as sequence variants and fragments as herein defined.

Pharmaceutical compositions of preferred embodiments of the present disclosure comprise a mammalian alfa defensin and/or a mammalian beta defensin, such as a human alfa defensin and a human beta defensin, and a pharmaceutically acceptable carrier and/or diluent.

Pharmaceutically and animal feed acceptable carriers and/or diluents are familiar to those skilled in the art. For compositions formulated as liquid solutions, acceptable carriers and/or diluents include saline and sterile water, and may optionally include antioxidants, buffers, bacteriostats, and other common additives.

The disclosed compound may be formulated in a wide variety of formulations for oral administration. Solid form preparations may include powders, tablets, drops, capsules, cachets, lozenges, and dispersible granules. Other forms suitable for oral administration may include liquid form preparations including emulsions, syrups, elixirs, aqueous solutions, aqueous suspensions, toothpaste, gel dentrifrice, chewing gum, or solid form preparations which are intended to be converted shortly before use to liquid form preparations, such as solutions, suspensions, and emulsions.

The disclosed compound may be formulated in a wide variety of formulations for subcutaneous administration.

The formulation can contain (in addition to a mammalian alfa defensin and/or a mammalian beta defensin, and other optional active ingredients) carriers, fillers, disintegrators, flow conditioners, sugars and sweeteners, fragrances, preservatives, stabilizers, wetting agents, emulsifiers, solubilizers, salts for regulating osmotic pressure, buffers, diluents, dispersing and surface-active agents, binders, lubricants, and/or other pharmaceutical excipients as are known in the art.

One skilled in this art may further formulate mammalian or poultry alfa defensin and mammalian or poultry beta defensins in an appropriate manner, and in accordance with accepted practices, such as those described in Remington's Pharmaceutical Sciences, Gennaro (1990).

A mammalian alfa defensin and a mammalian beta defensin, such as a human alfa defensin and a human beta defensin, can be used alone, or in combination therapies with one, two, or more other pharmaceutical compounds or drug substances, for example with insulin/insulin analogs and/or glucagon like peptide-1 (GLP-1)/GLP-1 analogs and/or glucagon like peptide-2 (GLP-2)/GLP-2 analogs and/or a dipeptidyl peptidase IV (DPP-IV) inhibitor and/or metformin and/or a sodium glucose transporter-2 (SGLT-2) inhibitor and/or a Glucagon receptor antagonist and/or a transient receptor potential cation channel subfamily V member 1 (TRPV1) antagonist and/or with one or more pharmaceutically acceptable excipient(s). Preferably, the insulin or insulin analogs or GLP-1 or GLP1 analogs are administered parenterally through either subcutaneous or intramuscular administration. The GLP-1 analog may be selected from exenatide, liraglutide, lixisenatide, albiglutide, and dulaglutide, and the insulin analog may be selected from Lispro, Aspart, Glulisine, Detemir insulin, Degludec insulin, and Glargine insulin.

A mammalian alfa defensin and a mammalian beta defensin, such as a human alfa defensin and a human beta defensin, may also be used in combination therapies with either chemotherapy, immunotherapy, radiotherapy or a combination of these.

**Methods and Uses in Livestock**

The methods disclosed herein can be used with livestock to improve their growth rates and feed efficiency. The methods could for example be used as an alternative to antibiotics.

One aspect of the present disclosure provides methods for treatment of gut inflammation in non-human animals by administering an effective amount of a mammalian or poultry $\alpha$-defensin and/or $\beta$-defensin to a subject in need of such treatment.

Another aspect of the present disclosure relates to a method for promotion of lean growth in animal meat production, said method comprising administration of an effective amount of a mammalian or poultry $\alpha$-defensin and/or $\beta$-defensin to a subject in need thereof. The defensins can be used as alternatives to hormones, steroids and antibiotics. As demonstrated in Examples 1 and 3, administration of HD5 and hBD2 to mice fed on a high fat diet promotes lean growth as it prevents accumulation of fat mass.

***In vitro* synthesis**

Mammalian and poultry alfa defensins and mammalian and poultry beta defensins may be prepared by *in vitro* synthesis, using conventional methods as known in the art. Various commercial synthetic apparatuses are available, for example automated synthesizers by Applied Biosystems Inc., Beckman, etc. By using synthesizers, naturally occurring amino acids may be substituted with unnatural amino acids, particularly D-isomers (or D-forms) e.g. D-alanine and D-isoleucine, diastereoisomers, side chains having different lengths or functionalities, and the like. The particular sequence and the manner of preparation will be determined by convenience, economics, purity required, and the like.

Chemical linking may be provided to various peptides or proteins comprising convenient functionalities for bonding, such as amino groups for amide or substituted amine formation, e.g. reductive amination, thiol groups for thioether or disulphide formation, carboxyl groups for amide formation, and the like.

If desired, various groups may be introduced into the peptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

Mammalian and poultry alfa defensins and mammalian and poultry beta defensins, or functional equivalents thereof, may also be isolated and purified in accordance with conventional methods of recombinant synthesis. Recombinant synthesis may be performed using appropriate expression vectors and a eukaryotic expression system. A solution may be prepared of the expression host and the media and the defensins present purified using HPLC, exclusion chromatography, gel electrophoresis, affinity chromatography, or other purification technique. Methods for recombinant expression of human beta defensin 2 in E. coli are disclosed in WO 2010/007166 (Novozymes).

**Dosages**

A mammalian alfa defensin and a mammalian beta defensin, such as a human alfa defensin and a human beta defensin, are preferably employed in pharmaceutical compositions in an amount which is effective to treat gut inflammation, colorectal cancer, an endocrine, nutritional, metabolic or cardiovascular disease, preferably with acceptable toxicity to the patient. A mammalian or poultry alfa defensin and a mammalian or poultry beta defensin are preferably applied in animal feed to promote lean growth. A mammalian or poultry alfa defensin and a mammalian or poultry beta defensin, such as a human alfa defensin and a human beta defensin, are also preferably employed in pharmaceutical compositions or in animal feed in an amount which is effective to maintain a normal microbiota composition in the intestine or to treat or normalize a dysbiotic microbiota in the intestine, preferably with acceptable toxicity to the patient or the animal in need of the treatment.

For such treatments, the appropriate dosage will, of course, vary depending upon, for example, the chemical nature and the pharmacokinetic data of a compound used, the individual host, the mode of administration and the nature and severity of the conditions being treated. The term "mg HD5 equivalents" as used herein refers to equimolar concentration of human alfa and beta defensins in comparison to the concentration of HD5. The term "mg hBD-2 equivalents" as used herein refers to equimolar concentration of human alfa and beta defensins in comparison to the concentration of hBD-2. As the molecular weights of the disclosed defensins are comparable, the term "mg HD5 equivalent" and "mg hBD2 equivalent" may simply mean mg of the defensin used.

However, in general, for satisfactory results in mammals or poultry, for example humans, an indicated daily dosage of a human alfa defensin is preferably from about 0.1 mg HD5 equivalents/kg body weight to about 10 mg HD5 equivalents/kg body weight, more preferably from about 0.5 mg HD5 equivalents/kg body weight to about 10 mg HD5 equivalents/kg body weight; such as 1 mg HD5 equivalents/kg body weight to 10 mg HD5 equivalents/kg body weight, more preferably from about 1.2 mg HD5 equivalents/kg body weight to about 10 mg HD5 equivalents/kg body weight, preferably from about 1.2 mg HD5 equivalents/kg body weight to about 5 mg HD5 equivalents/kg body weight, even more preferably 1.2 mg HD5 equivalents/kg body weight, for example, administered in divided doses up to one, two or three times a day.

In one embodiment of the present disclosure an indicated daily dosage of a human beta defensin is preferably from about 0.1 mg hBD-2 equivalents/kg body weight to about 10 mg hBD-2 equivalents/kg body weight, more preferably from about 0.5 mg hBD-2 equivalents/kg body weight to about 10 mg hBD-2 equivalents/kg body weight; such as 1 mg hBD-2 equivalents/kg body weight to 10 mg hBD-2 equivalents/kg body weight, more preferably from about 1.2 mg hBD-2 equivalents/kg body weight to about 10 mg hBD-2 equivalents/kg body weight, preferably from about 1.2 mg hBD-2 equivalents/kg body weight to about 5 mg hBD-2 equivalents/kg body weight, even more preferably 1.2 mg hBD-2 equivalents/kg body weight, for example, administered in divided doses up to one, two or three times a day.

In one embodiment of the present disclosure, an indicated daily dosage of a human alfa defensin together with a human beta defensin is preferably from about 0.1 mg hBD-2 equivalents/kg body weight to about 10 mg hBD-2 equivalents/kg body weight, more preferably from about 0.5 mg hBD-2 equivalents/kg body weight to about 10 mg hBD-2 equivalents/kg body weight; such as 1 mg hBD-2 equivalents/kg body weight to 10 mg hBD-2 equivalents/kg body weight, more preferably from about 1.2 mg hBD-2 equivalents/kg body weight to about 10 mg hBD-2 equivalents/kg body weight, preferably from about 1.2 mg hBD-2 equivalents/kg body weight to about 5 mg hBD-2 equivalents/kg body weight, even more preferably 1.2 mg hBD-2 equivalents/kg body weight, for example, administered in divided doses up to one, two or three times a day.

In general, in mammals, for example humans, an indicated daily dosage of a human alfa defensin is preferably from about 0.1 mg HD5/kg body weight to about 10 mg HD5/kg body weight, more preferably from about 0.5 mg HD5/kg body weight to about 10 mg HD5/kg body weight; such as 1 mg HD5/kg body weight to 10 mg HD5/kg body weight, more preferably from about 1.2 mg HD5/kg body weight to about 10 mg HD5/kg body weight, preferably from about 1.2 mg HD5/kg body weight to about 5 mg HD5/kg body weight, even more preferably 1.2 mg HD5/kg body weight, for example, administered in divided doses up to one, two or three times a day. Similar dosages can be used for other alpha-defensins.

In one embodiment of the present disclosure an indicated daily dosage of a human beta defensin is preferably from about 0.1 mg hBD-2/kg body weight to about 10 mg hBD-2/kg body weight, more preferably from about 0.5 mg hBD-2/kg body weight to about 10 mg hBD-2/kg body weight; such as 1 mg hBD-2/kg body weight to 10 mg hBD-2/kg body weight, more preferably from about 1.2 mg hBD-2/kg body weight to about 10 mg hBD-2/kg body weight, preferably from about 1.2 mg hBD-2/kg body weight to about 5 mg hBD-2/kg body weight, even more preferably 1.2 mg hBD-2/kg body weight, for example, administered in divided doses up to one, two or three times a day. Similar dosages can be used for other beta-defensins.

An indicated daily dosage of a human alfa defensin together with a human beta defensin is preferably from about 0.1 mg defensin/kg body weight to about 10 mg defensin /kg body weight, more preferably from about 0.5 mg defensin/kg body weight to about 10 mg defensin/kg body weight; such as 1 mg defensin/kg body weight to 10 mg defensin/kg body weight, more preferably from about 1.2 mg defensin/kg body weight to about 10 mg defensin/kg body weight, preferably from about 1.2 mg defensin/kg body weight to about 5 mg defensin/kg body weight, even more preferably 1.2 mg defensin/kg body weight, for example, administered in divided doses up to one, two or three times a day.

When two different defensins are administered in one dosage, the dosage may comprise equal or approximately equal amounts of the two defensins determined on a weight basis or on a molar basis. The ratio may also differ so that the ratio of alpha defensin to beta-defensin varies from 10:1 to 1:10, such as 5:1 to 1:5, for example 2:1 to 1:2 determined on a weight or molar basis.

The daily dosage could correspond to 0.6 mg HD5/kg body weight plus 0.6 mg hBD-2/kg body weight.

The compounds of preferred embodiments of the present disclosure can be administered to mammals or poultry, for example humans, piglets or calves by similar modes of administration at similar dosages than conventionally used.

In certain embodiments of the present disclosure, the pharmaceutical compositions or animal feed compositions of preferred embodiments of the present disclosure can include a mammalian or poultry alfa defensin and/or a mammalian or poultry beta defensin, such as a human alfa defensin and/or a human beta defensin, in an amount of about 0.5 mg or less to about 1500 mg or more per unit dosage form, preferably from about 0.1, 0 3, 0.5, 0.6, 0.7, 0.8, or 0.9 mg to about 150, 200, 250, 300, 350, 400, 450, 500, 600, 700, 800, 900, or 1000 mg, and more preferably from about 1, 2, 3, 4, 5, 6, 7, 8, 9, 10, 15, 20, or 25 mg to about 30, 35, 40, 45, 50, 55, 60, 65, 70, 75, 80, 85, 90, 95, or 100 mg. In certain embodiments of the present disclosure, however, lower or higher dosages than those mentioned above may be preferred. Appropriate concentrations and dosages can be readily determined by one skilled in the art. In certain embodiments of the present disclosure, the pharmaceutical compositions of preferred embodiments of the present disclosure include a mammalian alfa defensin, such as a human alfa defensin. In other embodiments of the present disclosure, the pharmaceutical compositions of preferred embodiments of the present disclosure include a mammalian beta defensin, such as a human beta defensin. In further embodiments of the present disclosure, the pharmaceutical compositions of preferred embodiments of the present disclosure include a mammalian alfa defensin and a mammalian beta defensin, such as a human alfa defensin and a human beta defensin, wherein the alfa and the beta defensins are present in equal amounts on a molarity basis or on a mg/mL basis.

In one embodiment of the present disclosure, the mammalian or poultry alfa and/or beta defensin is administered at least once daily, such as at least twice daily, for example at least 3 times daily.

**Examples**

Examples not falling under the scope of the appended claims do not form part of the invention.

**Example 1.**

Modulation of gut microbiota and prevention ofgut inflammation and metabolic syndrome by prophylactic treatment with defensins.

Materials and Methods

Mice: Mice were housed in trios, 4 cages per group. Feed intake was registered daily just before lights were turned off (6 pm). Individual mice were subjected to experimental procedures in altered order both group and cage wise. Mice were kept at room temperature under a 12-hour light/dark cycle at SPF standard conditions.

Diets: For dosing, the average weight was estimated to be 25 grams per mouse. They eat approximately 3 grams of feed per mouse per day.

Treatment regime (Figure 1A): Mice were fed either a high fat diet (HFD) or a low fat (LF) control diet. The HFD contains 4 subgroups; 1 hBD2, 1 HD5, 1 hBD2/HD5 and 1 standard HFD without supplementation of defensins. Defensin concentration is 1,2mg hBD2 per kg mouse per day. HD5 is given in equimolar concentration to hBD2. The combinatory group is given 50% hBD2 + 50% HD5, hence a total amount of defensins equivalent to the remaining test groups.

Tests: Insulin tolerance test (ITT), glucose-stimulated insulin secretion (GSIS) test, oral glucose tolerance test (OGTT) and five hours fasting insulin test were performed over two days, with 50% of the mice per group per day, hence avoiding day to day variation as a confounding factor.

Microbial analyses were carried out to study the microbiota of the intestine. Longitudinal 16S characterization was conducted on 4 paired samples from 60 mice, 240 samples in total. Each mouse was sampled prior to diet change, 1 week post diet change, 4 weeks post diet change and at termination, thus ensuring a thorough characterization of the faecal microbiota as a result of defensin treatment. Additionally, the content of the small intestine was analysed at termination, hence providing valuable insight to possible alterations at the key site of nutrient uptake.

A full metabolomic profile of the cecal content may be conducted to allow translation of microbial alterations into whole-body metabolism. A detailed histological and immunohistochemical analysis of duodenum, jejunum, ileum and colon may also be performed.

Results from the hBD2+HD5 treatment were excluded from the analysis.

**Results**

Weight change. While the food intake was similar in all three experimental diet groups (Figure 6C), both High Fat Diet (HFD) groups gained significantly more weight than the Low Fat Diet (LFD) reference group over the 10 week study period (***p<0.0001 2-way ANOVA, Tukey Post Test). The HFD plus hBD-2 group, however, gained significantly less weight than the HFD reference group (*p= 0.0028) (Figure 6A and B).

Lean/fat mass development. The lean/fat mass was equally distributed between the three experimental groups at study outset (figure 7A and B). At the end of the study both HFD groups had gained the same amount of lean mass (Figure 7B), which was significantly higher than the LFD group (*p<0.0001, One-way ANOVA, Tukey Post Test), probably due to increased body mass. At the end of the study, the HFD plus hBD-2 group trended towards increased fat mass compared to the LFD group (Figure 7B). However this was not statistically significant (*p=0.25). The HFD group had gained almost four times the amount of fat mass compared with the LFD group and 2 times the amount of fat mass compared to the HFD plus hBD-2 group (*p<0.0001 and *p=0.005, respectively) (Figure 7B).

Insulin Tolerance test. Both the LFD group and the HFD plus hBD-2 group were significantly more sensitive to insulin than the HFD group (p< 0.05) (Figure 8a).

Glucose Tolerance test. The HFD group was glucose intolerant with a prolonged clearance of glucose from peak at 15 min to semi-clearance at 120 min. The LFD group had a rapid clearance of glucose from peak at 15 min. The HFD plus hBD-2 group had a slightly prolonged glucose clearance but reached significantly lower glucose levels than the HFD group (p<0.05) (Figure 8B).

Glucose Stimulated Insulin Secretion. The HFD group had impaired glucose homeostasis with a significantly higher and sustained insulin concentration following glucose administration (p<0.05). The LFD group had almost no increase in insulin concentration following glucose stimulation. The HFD plus hBD-2 group had a higher but not significantly different insulin concentration than the LFD group (Figure 8C).

Five Hour Fasting insulin. The HFD group were profoundly diabetic with a significantly higher fasting insulin level than the LFD (*p=0.0004) and a borderline significantly higher fasting insulin than the HFD plus hBD-2 group (*p = 0.057). There was no significant difference between the LFD and HFD plus hBD-2 groups (*p = 0.17) (Figure 8D).
*Tukey post test, otherwise Dunnett post hoc test.

**After completion of the study in Example 1, the results have been analysed again and are presented below and in the accompanying drawings:**

Weight change. While the food intake was similar in all three experimental diet groups, both High Fat Diet (HFD) groups gained significantly more weight than the Low Fat Diet (LFD) reference group over the 10 week study period (*p<0.0001 2-way ANOVA, Tukey Post Test). The HFD plus hBD-2 group, however, gained significantly less weight than the HFD reference group (*p= 0.0028) (Figure 9a). Figure 9B illustrates the feed efficiency, and Figure 9C the energy intake.

Lean/fat mass development. The lean/fat mass was equally distributed between the three experimental groups at study outset. At the end of the study both HFD groups had gained the same amount of lean mass, which was significantly higher than the LFD group (*p<0.0001, One-way ANOVA, Tukey Post Test), probably due to increased body mass. At the end of the study, the HFD plus hBD-2 group trended towards increased fat mass compared to the LFD group. However this was not statistically significant (*p=0.25). The HFD group had gained almost three times the amount of fat mass in percent of total body weight compared with the LFD group and two times the amount of fat mass in percent of total body weight compared to the HFD plus hBD-2 group (*p<0.0001 and *p=0.005, respectively) (Figure 10a). There was no statistical difference between the three groups in terms of liver weight (figure 10b), whereas HFD plus hBD-2 had significantly less visceral fat (eWAT) than the HFD group (figure 10c).

Glucose Tolerance test. The HFD group was glucose intolerant with a prolonged clearance of glucose from peak at 15 min to semi-clearance at 120 min. The LFD group had a rapid clearance of glucose from peak at 15 min. The HFD plus hBD-2 group had a slightly prolonged glucose clearance but reached significantly lower glucose levels than the HFD group (p<0.05) (Figure 11a).

Glucose Stimulated Insulin Secretion. The HFD group had impaired glucose homeostasis with a significantly higher and sustained insulin concentration following glucose administration (p<0.05). The LFD group had almost no increase in insulin concentration following glucose stimulation. The HFD plus hBD-2 group had a higher but not significantly different insulin concentration from the LFD group (Figure 11b).

Insulin Tolerance test. Both the LFD group and the HFD plus hBD-2 group were significantly more sensitive to insulin than the HFD group (p< 0.05) (Figure 12A). There was no statistical significant difference between the LFD reference group and the HFD plus hBD-2 group.

Homeostasis Model Assessment (HOMA-IR). The relationship between insulin resistance and beta cell function as assessed by the HOMA-IR index was statistically significantly lower for the HFD plus hBD-2 group compared with the HFD group (figure 12b).
*Tukey post test, otherwise Dunnett post hoc test.

Conclusions of hBD-2 as prevention against development of diabetes and obesity in high fat diet fed mice:

- Uniform weight gain (with low intragroup SD)
- 50% of the HFD-hBD2-fed mice had a Body Fat Percent resembling LFD reference mice, despite being fed 60% HFD. A few mice had even lower fat% than the lowest LFD reference mice.

- The best protected hBD2-fed mice had the same or smaller visceral fat mass than LFD reference mic, which is highly unusual on a 60% HFD!

- Improved insulin sensitivity! hBD2 fed mice were not significantly different from the LFD reference mice. Both insulin tolerance test and HOMA-IR indicate improved insulin signaling.

- Glucose tolerance was markedly improved compared to HFD control mice. Importantly, both glucose tolerance and the glucose stimulated insulin response during the glucose challenge was improved. This means that hBD2-fed mice required less insulin to handle the glucose bolus better than the HFD control mice did.

Conclusions of HD5 as prevention against development of diabetes and obesity in high fat diet fed mice:

- HD5-fed mice gained less weight than HFD-fed control mice (Figure 17A), although the effect was not statistically significant. There was also a tendency towards lower feed efficiency (Figure 17B) and energy intake (Figure 17C).

- HD5-fed mice had borderline less fat % (Figure 18A) and borderline less visceral fat than did HFD-fed control mice (Figure 18C). No statistically significant difference with respect to liver weight was found (figure 18B)

- There was no significant improvement in glucose tolerance (Figure 19A), glucose-stimulated insulin secretion (figure 19B), insulin tolerance (Figure 20A), and HOMA-IR (Figure 20B), although the HD5 fed mice did perform better than the HFD-fed control mice. The difference in results obtained with hBD2 and HD5 suggest differences in mode of action between hBD2 and HD5.

With respect to modulation of microbiota, the results in Figure 21A from week 1 of the study demonstrates that a change in microflora has already happened within the first week of the study. The three HFD groups have comparable microflora different from the microflora of the LFD group.

At the completion of the study after 10 weeks, the microflora of the HFD-HD5 group had changed and was now intermediate between the LFD group and the non-treated HFD group (Figure 21B). The conclusion is that oral administration of HD5 leads to a partial normalization of the microflora so that it is more similar to the microflora of the LFD group than the non-treated group.

In the HFD groups, at termination, mice treated with alfa defensin (HD5) showed a normalization of the microbiota with an increased abundance of *Allobaculum* and *Lactobacillus* and a decrease in abundance of *Clostridium* in the intestinal microbiota than mice that were not treated (Figure 21C). *Allobaculum* is a short chain fatty acid producing species. Lactobacillus is a bacteria with anti-inflammatory properties.

The results may be interpreted to mean that in the HFD groups, at termination, mice treated with alfa, beta and alfa and beta defensins show higher gene richness and higher number of bacteria in the intestinal microbiota than mice that were not treated.

In the LFD control group, at termination, mice show healthy unaltered microbiota in the intestine.

**Example 2.** Modulation of gut microbiota by defensins.

Invertebrates: For *in vivo* proof of concept one may employ the invertebrate wax moth model *Galleria mellonella (G. mellonella).* Faeces can be analysed after forced-feed administration of $\alpha$- and/or $\beta$- defensins (Giannouli et al. 2014)(Favre-Godal et al. 2014).

Example 3. Modulation of gut microbiota, gut inflammation and metabolic syndrome parameters by interventional treatment with defensins in obese mice.

Mice and diets. The experiment elucidates the effect of hBD-2 and HD5 in the treatment of metabolic syndrome (MetS) in diet-induced obese mice. A run-in period of 13 weeks where mice were fed a very HFD (60% energy from fat) preceded the intervention. Only mice meeting the criteria of a minimum of 12 gram weight gain (approximately 50% of initial bodyweight) during the run-in period were included in the final analyses. Mice that did not meet these criteria stayed in their respective cages as hierarchy 'keepers'. They were exposed to all experimental tests, but excluded from the analyses.

Treatment regimen (figure 1B). Before the intervention all mice were MR scanned and an OGTT was performed. Cages of mice were allocated to experimental groups based on their fat mass. All subsequent measures were paired with data from the same mouse before the intervention.

A LFD (low fat diet) reference group was running in parallel. As controls for the intervention 2 additional groups were included: 1 very HFD and 1 LFD. Experimental mice stayed on the very HFD during the intervention. The mice were on the experimental diet for 10 weeks. They were co-housed throughout the experiment, 4 mice per cage, 3 cages per group. All tests ran over 3 days, 1 cage per group per day.

Tests. Insulin tolerance test (ITT), glucose-stimulated insulin secretion (GSIS) test, and oral glucose tolerance test (OGTT) and five hours fasting insulin test were performed over three days, with 1/3 of the mice per group per day, hence avoiding day to day variation as a confounding factor.

Microbial analyses may be carried out to study the microbiota of the intestine. Longitudinal 16S characterization may be conducted on 7 paired samples from 60 mice, 240 samples in total. Each mouse may be sampled prior to diet change, 2 weeks, 4 weeks, 6 weeks, 8 weeks post diet change and at termination, thus ensuring a thorough characterization of the faecal microbiota as a result of defensin treatment.

Additionally, the content of the small intestine may be analysed at termination (via 16S or deep sequencing), hence providing valuable insight to possible alterations at the key site of nutrient uptake.

Lastly, a full metabolomic profile of the cecal content may be conducted to allow translation of microbial alterations into whole-body metabolism. A detailed histological and immunohistochemical analysis of duodenum, jejunum, ileum and colon may also be performed.

hBD-2 as treatment of metabolic syndrome in obese high fat diet fed mice:

Results.

Weight change. The standard high fat diet (HFD) fed groups had an equal food intake throughout the entire study period and had the same weight development with equal fat and lean mass the first 13 weeks, thus having the same starting point prior to the dietary intervention. The weight gain was significantly larger than in the low fat diet fed (LFD) group (*p<0.05 2-way ANOVA) (Figure 13A). After the dietary intervention the HFD groups continued to increase in weight, however the HFD plus hBD-2 group tended to gain less weight the first 4 weeks post dietary intervention, although not significantly (*p=0.07 2-way ANOVA). From week 4 to the end of the study period the HFD plus hBD-2 group gained similar weight as the standard HFD group (*p=0.82 2-way ANOVA) (Figure 13B).

Fat percentage. The fat percentage of total body weight was similar between the three experimental groups at onset of the study period. At the point of dietary intervention the fat percentage of the two HFD fed groups was the same and both were significantly larger than the LFD fed group, which was consistent throughout the 10 weeks post dietary intervention (*p<0.05 2-way ANOVA) (Figure 14A). At weeks 4 post dietary intervention -75% of the HFD plus hBD-2 group had a smaller fat percentage than before the intervention, dramatically contrasting the standard HFD group, where all mice had increased fat percentages. (Figure 14B) The change in fat percentage was significantly smaller in the HFD plus hBD-2 group than the standard HFD group at this time point. (*p=0.003 2-way ANOVA). The weight of the liver at termination was significantly larger in the HFD fed groups compared to the LFD group (*p<0.05 One-way ANOVA) (Figure 15A). The amount of visceral fat (eWAT) at termination was also higher in the HFD groups compared to the LFD (*p<0.05 One-way ANOVA). There was no significant difference in visceral fat (eWAT) between the HFD fed groups (Figure 15B).

Glucose Tolerance Test. The glucose tolerance improved rapidly from the point of dietary intervention in the HFD plus hBD-2 group, which showed a smaller peak in blood glucose as well as a faster clearance of glucose already after 2 weeks (Figure 16A). The most glucose intolerant mouse in the study was observed to improve drastically the first two weeks after being switched from a standard HFD to HFD plus hBD-2 (Figure 16B).

Insulin Tolerance Test. The LFD group was significantly more sensitive to insulin than both HFD groups (*p<0.05 2-way ANOVA). The HFD plus hBD-2 group was simultaneously more insulin sensitive compared to the HFD control group, implying an improvement in insulin tolerance since the dietary intervention (*p<0.05 2-way ANOVA) (Figure 16C).

Conclusions of hBD-2 as intervention against development of diabetes and obesity in high fat diet fed mice:

- Overall, hBD-2-fed mice gained less weight the first 4 weeks of intervention than did HFD control mice (Figure 143).

- 7/8 obese and glucose intolerant mice significantly improved their glucose tolerance following only 2 weeks of intervention (Figure 14a). A single mouse was the most glucose intolerant mouse at baseline with a fat mass of approximately 20 gram out of 50 grams body weight. Despite this severely unhealthy phenotype, the mouse was completely rescued in terms of glucose intolerance by 2 weeks of intervention (Figure 16B).

- On a whole-body level, hBD-2-fed mice were less insulin resistant than HFD control mice (Figure 16C). This is a key point as severe systemic insulin resistance is extraordinarily difficult to reverse and a main limitation in treatment of human diseases (e.g. Diabetes, CVDs, certain cancers among others)

HD-5 as treatment of metabolic syndrome in obese high fat diet fed mice:

Weight change. All HFD fed groups had the same food intake during the study period and equal weight gain during the run-in period of 13 weeks (Figure 22A). After dietary intervention the group fed HFD plus HD-5 gained significantly less weight than the HFD control (*$p<0.05$ 2-way ANOVA) (Figure 22B). In addition, a tendency of decreasing fat percentage in the HFD plus HD-5 group was observed (Figure 23A), and a significantly lower fat percentage in the HFD plus HD-5 was measured 4 weeks after dietary change in comparison to the HFD control (*$p=0.009$ 2-way ANOVA) (Figure 23B). The weight of the liver at termination tended to be decreased in the HFD plus HD-5 fed group compared to the HFD control. Specifically, -50% of the standard HFD fed mice scored higher than the highest HFD plus HD-5 fed mouse (Figure 24A). The weight of visceral fat was larger in the HFD fed groups than the LFD fed group. (*$p<0.05$ One-way ANOVA) (Figure 24B).

Glucose tolerance test. The glucose tolerance for the HFD+HD5 treated animals in a representative cage, Cage 2, improved over time from the start of the intervention (week 13-0) until week 13.8 (Figure 25A)

Insulin tolerance test. The LFD group was significantly more insulin sensitive than the HFD fed groups (*$p<0.05$ 2-way ANOVA). The HFD plus HD-5 group was more insulin sensitive than the HFD control, implying an improvement in insulin tolerance since the dietary intervention.(*$p<0.05$ 2-way ANOVA) (Figure 25B).

Conclusions of HD5 as intervention against development of diabetes and obesity in high fat diet fed mice:

- HD5-fed mice had significantly decreased weight change compared with HFD-fed control mice (Figure 22B).

- There was a general tendency to decreased fat mass of obese HFD-HD5-fed mice (Figure 23A and B).

- Liver mass tended to be decreased in HD5-fed mice as compared to HFD-fed control mice (Figure 24a). Since the visceral and subcutaneous depots were not significantly different (Figure 24b), this observation suggests that the modestly decreased fat% in HD5 mice is restricted to hepatic lipolysis/lipid oxidation.

- Glucose tolerance improved over time in the HD5 fed mice (figure 25A),

- HD5 fed mice were less insulin resistant than HFD-fed control mice (Figure 25b).

The changes observed are consistent with a higher gene richness and higher number of phylae in the intestinal microbiota in the HFD groups treated with defensins than in mice that are not treated.

**Example 4.** Modulation of gut microbiota and treatment of gut inflammation by interventional treatment with a glucagon like peptide-1 analog.

**Materials and Methods.**

Mice: 4 week old C57Bl/6J DIO male mice were fed a high fat diet (HFD 60% fat, SSNIFF (Diet #D12492)) or purina chow for 36 weeks. The HFD fed group had reached an average weight of approximately 55 gram by start of intervention. The mice were group-housed 10 per cage until week -2. From week -2 the mice were single housed throughout the study. Feed intake was registered daily just before lights were turned off at 3 pm. Individual mice were subjected to experimental procedures in altered order both group and cage wise. Mice were kept at room temperature under a 12-hour light/dark cycle at SPF standard conditions.
Treatment regime (Figure 26): Mice were fed either a high fat diet (HFD) or a low fat (LF) control diet. The HFD contained 2 subgroups; 1 GLP-1 and 1 standard HFD without supplementation of GLP-1. GLP-1 was dissolved in PBS and 0.1 % BSA was added. GLP-1 was administered at 0.2 mg/kg BID subcutaneously.
Analysis. 16S RNA microbiome analysis was performed on day -1 and 27 of the study. A sample from ilium was taken approximately 2 cm from caecum and snap frozen in liquid nitrogen for analysis of concentration of cytokines IFN$\gamma$, TNF-$\alpha$, IL-1$\beta$, IL12p70, IL-2, IL-4, IL-5, IL-6, IL-8 and IL-10.

**Results**

An unweighted unifrac analysis i.e. relative abundance of bacterial species. At the start of the study (day 1), the four groups have comparable microflora as expected. However, following 4 week's treatment the microbiota from the mice fed a high fat diet and interventional treatment with GLP-1 (Liraglutid) differed significantly from the microflora of the control (vehicle treated) group (Figure 27A).

The observed changes of gut microbiota are primarily driven by an increased abundance of *Akkermansia* and *Alloprevotella. Akkermansia* is a short chain fatty acid producing species (Figure 27B). Increase in the amount of these bacteria is considered indicative of a more healthy or normalized microflora. Short chain fatty acids are known to have a positive effect on induction of endogenous GLP-1.

Surprisingly no anti-inflammatory effect of the GLP-1 (Liraglutid) was observed as there was no statistical difference in the concentration of cytokines in ilium for any of the cytokines between the three study groups.

**Example 5.**

Pharmacokinetic study to determine oral bioavailability and establish pharmacokinetic profile of hBD-2 following single oral gavage of 4 mg/kg administration to NMRI mice.

**Materials and methods**

Treatment regimen: 21 female NMRI mice were dosed by oral gavage 5 ml/kg using a gavage tube and a 1 ml syringe according to the individual body weight obtained on the day of dosing. Urine was strived sampled at random time points by gently massaging the inguinal area of the abdomen. The first blood sample was taken using a submandibular sampling method. The second blood sample was collected from Isoflurane anaesthetised mice. Intestinal samples were taken after euthanasia. The abdomen of each mouse was opened and three sections (jejunum, duodenum, and colon) of the intestines were sampled.

**Results**

hBD-2 does not seem to be absorbed from the healthy intestine as it could not be detected by HPLC in any of the serum or urine samples as all values were below the detection level of < 10 pg/ml. This indicates that hBD-2 is not systemically available after oral dosing of 4 mg/kg in mice.
Orally administered hBD2 remains detectable in the colon content up to 360 minutes after administration (Figure 28).

**Example 6.**

To investigate and compare the pharmacokinetic profiles of hBD-2 fused to the C-terminal (molecular weight 71.336 Da) or N-terminal (molecular weight 71.666 Da) of human serum albumin following subcutaneous or intravenous administration of a molar equivalent to 1 mg/kg hBD-2 (molecular weight 66437 Da) to NMRI female mice.

**Material and methods**

Treatment regimen: The animals were dosed 10 ml/kg of stock concentration of 1.65 mg/ml according to the individual body weight (300 μL for a 30 gram mouse). First blood sample was taken using a submandibular sampling method and the second following Isoflurane anaesthesia and euthanasia.

**Results**

hBD-2 showed a half-life of 1 hour and the two fused proteins a half-life of 12 hours. AUC was changed dramatically. Renal clearances were also changed from 10 ml/min for hBD-2 (Figure 29) to 0.5-2.2 ml/min for the two fused molecules (Figures 30, 31). The example demonstrates that the half life of hBD2 can be extended markedly by Cor N-terminal conjugation to albumin.

**Example 7.**

To determine and assess the anti-inflammatory effect of "hBD-2-albumin fusion N-terminal" in an acute 10-day Dextran Sodium Sulphate (DSS) induced colitis model in mice.

**Material and methods**

Treatment regimen: "hBD-2-albumin N-terminal" was administered intravenously via the tail vein or subcutaneously with the use of a sterile 25G needle in a dosing volume of 10 ml/kg body weight. The animals received 1 dose daily for 10 executive days. The active control Dexamethasone (DEX) was given subcutaneously at a dose of 1 mg/kg in a dosing volume of 10 ml/kg body weight OD.

**Results**

Treatment with "hBD-2-albumin N-terminal" resulted in a significant inhibition of the disease activity index (DAI) when administered daily at a dose of 1.65 mg/kg via the intravenous route (p<0.05). Additionally, on day 10 a significant inhibition of the DAI score was also observed when the "hBD-2-albumin N-terminal" was administered daily at a dose of 1.65 mg/kg and at a dose of 125 mg/kg subcutaneously respectively (p<0.05).
Administration of dextran sodium sulphate resulted in a significant inflammation and injury of the colonic tissue as evidenced after histological examination. Treatment with "hBD-2-albumin N-terminal" did not result in any statistically significant reduction of this histological damage, but similarly the active control DEX failed to significantly reduce histological injury.
The results further showed a significant increase in body weight on day 7 in the animals treated with "hBD-2-albumin N-terminal" despite a transient fall in body weight on days 2 and 3. In contrast the DEX treated animals displayed a very significant decrease in body weight from day 5 onwards (p<0.01). This indicates that "hBD-2-albumin N-terminal" maintains a normal microbiota and thus has a weight preserving effect.
The example demonstrates the hBD2-albumin fusion N-terminal is biologically active in an animal model of an inflammatory condition.

**Example 8.**

To determine and assess the anti-inflammatory effect of "hBD-2-albumin fusion C-terminal" in an acute 10-day Dextran Sodium Sulphate (DSS) induced colitis model in mice.

**Material and methods**

Treatment regimen: "hBD-2-albumin C-terminal" was administered intravenously via the tail vein or subcutaneously with the use of a sterile 25G needle in a dosing volume of 10 ml/kg body weight. The animals received 1 dose daily for 10 executive days. The active control Prednisolone (Pred) was given orally by gavage at a dose of 1 mg/kg in a dosing volume of 10 ml/kg body weight OD.

**Results**

Treatment with "hBD-2-albumin C-terminal" resulted in a significant inhibition of the DAI when administered daily at a dose of 1.6 mg/kg via the intravenous route (p<0.05). Aditionally "hBD-2-albumin C-terminal" resulted in a significant inhibition of the DAI when administered on alternative days 0, 2, 4, 6, 8 and 10 at a dose of 1.6 mg/kg via the intravenous route (p<0.05). Daily treatment with Prednisolon resulted in a significant inhibition of the DAI on day 9 (p<0.05).
Administration of dextran sodium sulphate resulted in a significant inflammation and injury of the colonic tissue as evidenced after histological examination. Treatment with "hBD-2-albumin C-terminal" at a dose of 1.6 mg/kg resulted in a statistically significant reduction of this histological damage (p<0.05). Similarly, daily treatment with "hBD-2-albumin C-terminal" at a dose of 1.6 mg/kg and of 16.5 mg/kg on days 0, 2, 4, 6, 8, and 10 resulted in a significant reduction of the histological damage to the colon (p<0.01). Treatment with the active control Prednisolon failed to significantly reduce histological injury in the proximal part of the colon but did reduce the injury in the distal colon (p<0.01).
The results further showed a significant increase in body weight in the animals treated with "hBD-2-albumin C-terminal" (p<0.05). This indicates that "hBD-2-albumin C-terminal" maintains a normal microbiota and thus has a weight preserving effect.
The example demonstrates the hBD2-albumin fusion C-terminal is biologically active in an animal model of an inflammatory condition.

**Example 9. Sequences**

| SEQ ID NO. | Name | Sequence |
|---|---|---|
| 1 | Bovine beta defensin 2 | GVGNPVSCVRNKGICVPIRCPGSMKQIGTCVGRAVKCCRK |
| 2 | Chicken beta defensin 2 | LFCKGGSCHFGGCPSHLIKVGSCFRSCCKWPWNA |
| 3 | Orangutan beta defensin 2 | VFGDISNPVTCLRSGAICHPGFCPRRYKHIGTCGLSVIKCCKKP |
| 4 | hBD1 | DHYNCVSSGGQCLYSACPIFTKIQGTCYRGKAKCCK |
| 5 | hBD2 | GIGDPVTCLKSGAICHPVFCPRRYKQIGTCGLPGTKCCKKP |

(continued)

| SEQ ID NO. | Name | Sequence |
|---|---|---|
| 6 | hBD3 | GII NTLQKYYCRVRGGRCAVLSCLPKEEQIGKCSTRGRKCCRRKK |
| 7 | hBD4 | ELDRICGYGTARCRKKCRSQEYRIGRCPNTYACCLRK |
| 8 | HD5 | ATCYCRTGRCATRESLSGVCEISGRLYRLCCR |
| 9 | HD6 | AFTCHCRRSCYSTEYSYGTCTVMGINHRFCCL |
| 10 | Chimpanzee beta defensin 2 | GISDPVTCLKSGAICHPVFCPRRYKQIGTCGLPGTKCCKKP |
| 11 | Macaque beta defensin 2 | GIGDPVTCLKNGAICHPVFCPRRYKQIGTCGLPGTKCCKKP |
| 12 | Mouse beta defensin 3 | KINNPVSCLRKGGRCWNRCIGNTRQIGSCGVPFLKCCKRK |
| 13 | Horse beta defensin 2 | GIGNPISCARNRGVCIPIGCLPGMKQIGTCGLPGTKCCRK |
| 14 | Porcine beta defensin 1 | NIGNSVSCLRNKGVCMPGKCAPKMKQIGTCGMPQVKCCKR |
| 15 | Goat beta defensin 2 | GIINHRSCYRNKGVCAPARCPRNMRQIGTCHGPPVKCCRK |
| 16 | human LL37 | LLGDFFRKSKEKIGKEFKRIVQRIKDFLRNLVPRTES |
| 17 | truncated hBD2 | PVTCLKSGAICHPVFCPRRYKQIGTCGLPGTKCCKKP |

**References**

Ajslev TA, et al, 2014. Trends in parent-child correlations of childhood body mass index during the development of the obesity epidemic. PLoS One 9(10).

Angelakis E and Raoult D, 2010. The increase of Lactobacillus species in the gut flora of newborn broiler chicks and ducks is associated with weight gain. PLoS One 5(5).

Angelakis E., et al 2012. An evaluation of the effects of Lactobacillus ingluviei on body weight, the intestinal microbiome and metabolism in mice. Microb Pathog 52(1):61-8.

Armogida SA, et al, 2004. Identification and quantification of innate immune system mediators in human breast milk. Allergy Asthma Proc. 25(5):297-304.

Bäckhed F, et al, 2007. Mechanisms underlying the resistance to diet-induced obesity in germ-free mice. Proc Natl Acad Sci USA 104(3):979-84.

Belkaid Wand Hand TW, 2014. Role of the microbiota in immunity and inflammation. Cell 157(1):121-41.

Bowie JU and Sauer RT, 1989. Identifying determinants of folding and activity for a protein of unknown structure. Proc. Natl. Acad. Sci. USA 86: 2152-2156;

Chassaing B, et al, 2015. Dietary emulsifiers impact the mouse gut microbiota promoting colitis and metabolic syndrome. Nature 519(7541):92-6.

Cunningham BC and Wells JA, 1989. High-resolution epitope mapping of hGH-receptor interactions by alanine-scanning mutagenesis. Science 244: 1081-1085.

Derbyshire KM, Salvo JJ and Grindley ND, 1986. A simple and efficient procedure for saturation mutagenesis using mixed oligodeoxynucleotides. Gene 46:145-152.

Everard A and Cani PD, 2013. Diabetes, obesity and gut microbiota. Best Pract Res Clin Gastroenterol 27(1):73-83.

Faulds MH and Dahlman-Wright K, 2012. Metabolic diseases and cancer risk. Curr Opin Oncol. 24(1):58-61.

Favre-Godal Q, et al, 2014. Comprehensive approach for the detection of antifungal compounds using a susceptible strain of Candida albicans and confirmation of in vivo activity with the Galleria mellonella model. Phytochemistry. 105: 68-78.

Feng Q, et al, 2015. Gut microbiome development along the colorectal adenoma-carcinoma sequence. Nat Commun 6:6528.

Giannouli M, et al. Use of larvae of the wax moth Galleria mellonella as an in vivo model to study the virulence of Helicobacter pylori. 2014. BMC Microbiol14: 228.

Hilton DJ, et al, 1996. Saturation mutagenesis of the WSXWS motif of the erythropoietin receptor. J. Biol. Chem. 271: 4699-4708.

Khan M, et al, 2007. Growth-promoting effects of single-dose intragastrically administered probiotics in chickens.

Br Poult Sci 48(6):732-5.
Koren O, et al, 2012. Host remodeling of the gut microbiome and metabolic changes during pregnancy. Cell 150(3):470-80.
Le Chatelier E et al. 2013. Richness of human gut microbiome correlates with metabolic markers. Nature 500(7464):541-6.
Leviten M, 2016. The Finnish connection. Biocentury Innovations, June 16.
Liu hY et al, 2008. Suppression of hepatic glucose production by human neutrophil $\alpha$-defensin through a signaling pathway distinct from insulin. The Journal of Biological Chemistry 283(18):12056-12063.
Lowman HB, Bass SH, Simpson N and Wells JA, 1991. Selecting high-affinity binding proteins by monovalent phage display. Biochem 30:10832-10837.
Mowat AM and Agace WW, 2014. Regional specialization within the intestinal immune system. Nat Rev Immunol. 14(10):667-85.
Needleman SB and Wunsch CD, 1970. A general method applicable to the search for similarities in the amino acid sequence of two proteins. J. Mol. Biol. 48: 443-453.
Ner SS, Goodin DB and Smith M, 1988. A simple and efficient procedure for generating random point mutations and for codon replacements using mixed oligodeoxynucleotides. DNA 7:127-134.
Neurath H and Hill RL, 1979. The Proteins. Academic Press, New York.
Qin J, et al 2012. A metagenome-wide association study of gut microbiota in type 2 diabetes. Nature 490(7418):55-60.
Reidhaar-Olson JF and Sauer RT, 1988. Combinatorial cassette mutagenesis as a probe of the informational content of protein sequences. Science 241:53-57;
Gennaro AR, 1990. Remington 's Pharmaceutical Sciences. Ed. Mack Publishing Co., Easton, PA.
Rice P, Longden I and Bleasby A, 2000. EMBOSS: the European Molecular Biology Open Software Suite. Trends in Genetics 16: 276-277.
Ridaura VK, et al, 2013. Gut microbiota from twins discordant for obesity modulate metabolism in mice. Science 341(6150):1241214.
Salzman NH, Underwood MA and Bevins CL, 2007. Paneth cells, defensins, and the commensal microbiota: a hypothesis on intimate interplay at the intestinal mucosa. Semin Immunol 19(2):70-83.
Suez J, et al, 2014. Artificial sweeteners induce glucose intolerance by altering the gut microbiota. Nature 514(7521):181-6.
Trasande L, Blustein J, Liu M, Corwin E, Cox LM and Blaser MJ, 2012. Infant antibiotic exposures and early-life body mass. Int J Obes (Lond) 37(1):16-23.
Turnbaugh PJ, Ley RE, Mahowald MA, Magrini V, Mardis ER and Gordon JI, 2006. An obesity-associated gut microbiome with increased capacity for energy harvest. Nature. 2006 Dec 21;444(7122):1027-31.
Turnbaugh PJ, Bäckhed F, Fulton L and Gordon JI, 2008. Diet-induced obesity is linked to marked but reversible alterations in the mouse distal gut microbiome. Cell Host Microbe 3(4):213-23.
Vrieze A, et al 2012. Transfer of intestinal microbiota from lean donors increases insulin sensitivity in individuals with metabolic syndrome. Gastroent 143(4):913-6.
Walter, 2015. Murine gut microbiota-diet trumps genes. Cell Host Microbe 17(1):3-5.
Wehkamp J, et al, 2002. Innate immunity and colonic inflammation: enhanced expression of epithelial alpha-defensins. Dig Dis Sci. 47(6):1349-55.
Zhang X, et al, 2015. The oral and gut microbiomes are perturbed in rheumatoid arthritis and partly normalized after treatment. Nat Med 21(8):895-905.

And following patents and patent applications:

WO 2010/007166
WO 92/06204
WO 95/17413
WO 95/22625
US 5,223,409

## Claims

1. A β-defensin selected from the group consisting of hBD1 (SEQ ID NO: 4), hBD2 (SEQ ID NO: 5), hBD3 (SEQ ID NO: 6), hBD4 (SEQ ID NO: 7), truncated hBD2 (SEQ ID NO: 17) or a β-defensin that differs from one of hBD1, hBD2, hBD3, hBD4, or truncated hBD2 (SEQ ID NO: 17) by less than 5 conservative amino acid substitutions for use in the treatment of type 2 diabetes, metabolic syndrome, systemic low grade inflammation, obesity, insulin resistance and/or glucose intolerance; wherein said β-defensin is administered by oral or subcutaneous administration to a subject in need thereof.

2. An α-defensin selected from the group consisting of HD5 (SEQ ID NO: 8) and HD6 (SEQ ID NO: 9) for use in the treatment of type 2 diabetes, metabolic syndrome, obesity, insulin resistance and/or glucose intolerance; wherein said α-defensin is administered by oral administration to a subject in need thereof.

3. A β-defensin selected from the group consisting of hBD1 (SEQ ID NO: 4), hBD2 (SEQ ID NO: 5), hBD3 (SEQ ID NO: 6), hBD4 (SEQ ID NO: 7), truncated hBD2 (SEQ ID NO: 17) or a defensin that differs from one of hBD1, hBD2, hBD3, hBD4, or truncated hBD2 (SEQ ID NO: 17) by less than 5 conservative amino acid substitutions for use in the treatment of increasing gene richness of the intestinal microbiota and/or increasing the number of phylae of the intestinal microbiota, wherein the β-defensin is administered by oral or subcutaneous administration to a subject in need thereof.

4. An α-defensin selected from the group consisting of HD5 (SEQ ID NO: 8) and HD6 (SEQ ID NO: 9) for use in the treatment or normalization of a dysbiotic microbiota in the intestine, increasing gene richness of the intestinal microbiota, increasing the number of phylae of the intestinal microbiota, or increasing the production of short chain fatty acids from the intestinal microbiota/metabolome, wherein the α-defensin is administered by oral administration to a subject in need thereof.

5. The α-defensin for use according to claim 4, wherein the number of bacteria belonging to the genus *Allobaculum* is increased.

6. The α-defensin or β-defensin for use according to any one of the preceding claims, wherein the treatment comprises reduction in weight or prevention of weight gain, and/or lowering of fasting blood glucose.

7. The α-defensin or β-defensin for the use according to any one of the preceding claims, wherein the defensin is HD5 or hBD2.

8. The α-defensin or β-defensin for use according to any one of the preceding claims, wherein said defensin further comprises at least one additional moiety selected from a group consisting of a cell penetrating peptide (CPP), an Albumin Binding Moiety (ABM), a detectable moiety (Z), and a half-life extending peptide, preferably a half-life extending peptide, optionally wherein the half-life extending peptide is selected from a group consisting of a molecule capable of binding to a neonatal Fc receptor (FcRn), transferrin, albumin (HAS), XTEN® or PEG, a homo-amino acid polymer (HAP), a proline-alanine-serine polymer (PAS), or an elastin-like peptide (ELP), hyaluronic acid, a negatively charged highly siacylated peptide such as the carboxy-terminal peptide (CTP) of chorionic gonadotropin (CG) β-chain, human IgG, and CH3(CH2)nCO- wherein n is 8 to 22.

9. The α-defensin or β-defensin for use according to any one of the preceding claims, wherein the subject has:

   I) a BMI of 25 or more, such as 30 or more, for example 35 or more, such as 40 or more;
   II) a waist/hip ratio of at least 0.80, for example 0.80-0.84, such as at least 0.85 (female) or at least 0.90, for example 0.9-0.99, such as above 1.00 (male);
   III) a fasting blood glucose of at least 6.1 mmol/l, for example at least 7.0 mmol/l;
   IV) a glycated haemoglobin level of at least 42 mmol/mol Hb, such as between 42 and 46 mmol/mol Hb, such as at least 48 mmol/mol Hb;
   V) Elevated blood pressure: ≥ 140/90 mmHg;
   VI) Dyslipidemia: triglycerides (TG): ≥ 1.695 mmol/L and high-density lipoprotein cholesterol (HDL-C) ≤ 0.9 mmol/L (male), ≤ 1.0 mmol/L (female); and/or
   VII) Microalbuminuria: urinary albumin excretion ratio ≥ 20 μg/min or albumin:creatinine ratio ≥ 30 mg/g.

10. The α-defensin or β-defensin for use according to any one of the preceding claims, wherein
the β-defensin is administered to a subject in need thereof at a daily dosage between 0.1 mg hBD2 /kg and 10 mg hBD2 /kg; or
wherein the α-defensin is administered to a subject in need thereof at a daily dosage between 0.1 mg HD5/kg and 10 mg HD5/kg.

11. The α-defensin or β-defensin for use according to any one of the preceding claims, wherein said defensin is administered in combination with insulin/insulin analogs and/or glucagon like peptide-1 (GLP-1)/GLP-1 analogs and/or glucagon like peptide-2 (GLP-2)/GLP-2 analogs and/or a dipeptidyl peptidase IV (DPP-IV) inhibitor and/or metformin and/or a sodium glucose transporter-2 (SGLT-2) inhibitor and/or a Glucagon receptor antagonist and/or a transient receptor potential cation channel subfamily V member 1 (TRPV1) antagonist or a combination of these, optionally wherein the GLP-1 analog is selected from exenatide, liraglutide, lixisenatide, albiglutide, and dulaglutide; or Lispro, Aspart, Glulisine, Detemir insulin, Degludec insulin, and Glargine insulin.

12. The α-defensin or β-defensin for use according to any one of the preceding claims, wherein the α-defensin or β-defensin is administered to a subject in need thereof at least once daily, such as once a day, two times per day, or three times per day.

13. The α-defensin or β-defensin for use according to any one of claims 3 to 5, wherein the subject has undertaken and/or is undertaking an antibiotic treatment or chemotherapy or immunotherapy or immunosuppressive therapy or another treatment that has negative effects on the intestinal microbiota.

14. A method for promotion of lean growth in livestock, the method comprising administration of an effective amount of a mammalian α-defensin and/or β-defensin selected from the group consisting of hBD1 (SEQ ID NO: 4), hBD2 (SEQ ID NO: 5), hBD3 (SEQ ID NO: 6), hBD4 (SEQ ID NO: 7), truncated hBD2 (SEQ ID NO: 17), a β-defensin that differs from one of hBD1, hBD2, hBD3, hBD4, or truncated hBD2 (SEQ ID NO: 17) by less than 5 conservative amino acid substitutions, HD5 (SEQ ID NO: 8), and HD6 (SEQ ID NO: 9) to a subject in need thereof, wherein the livestock is selected from cows, pigs, sheep, goats, horses, ducks, geese, pigeons, turkeys, quails and chickens.

15. The method according to claim 14, wherein the mammalian α-defensin and/or β-defensin is administered by mixing into the animal feed.

## Patentansprüche

1. β-Defensin, ausgewählt aus der Gruppe bestehend aus hBD1 (SEQ ID NO: 4), hBD2 (SEQ ID NO: 5), hBD3 (SEQ ID NO: 6), hBD4 (SEQ ID NO: 7), gekürztem hBD2 (SEQ ID NO: 17) oder einem β-Defensin, das sich von einem von hBD1, hBD2, hBD3, hBD4 oder gekürztem hBD2 (SEQ ID NO: 17) um weniger als 5 konservative Aminosäuresubstitutionen unterscheidet, zur Verwendung bei der Behandlung von Typ-2-Diabetes, metabolischem Syndrom, systemischer niedriggradiger Entzündung, Adipositas, Insulinresistenz und/oder Glucoseintoleranz; wobei das β-Defensin durch orale oder subkutane Verabreichung an ein Subjekt verabreicht wird, das dessen bedarf.

2. α-Defensin, ausgewählt aus der Gruppe bestehend aus HD5 (SEQ ID NO: 8) und HD6 (SEQ ID NO: 9), zur Verwendung bei der Behandlung von Typ-2-Diabetes, metabolischem Syndrom, Adipositas, Insulinresistenz und/oder Glucoseintoleranz; wobei das α-Defensin durch orale Verabreichung an ein Subjekt verabreicht wird, das dessen bedarf.

3. β-Defensin, ausgewählt aus der Gruppe bestehend aus hBD1 (SEQ ID NO: 4), hBD2 (SEQ ID NO: 5), hBD3 (SEQ ID NO: 6), hBD4 (SEQ ID NO: 7), gekürztem hBD2 (SEQ ID NO: 17) oder einem Defensin, das sich von einem von hBD1, hBD2, hBD3, hBD4 oder gekürztem hBD2 (SEQ ID NO: 17) um weniger als 5 konservative Aminosäuresubstitutionen unterscheidet, zur Verwendung bei der Behandlung von zunehmendem Genreichtum der Darmflora und/oder der Zunahme der Anzahl von Phyla der Darmflora, wobei das β-Defensin durch orale oder subkutane Verabreichung an ein Subjekt verabreicht wird, das dessen bedarf.

4. α-Defensin, ausgewählt aus der Gruppe bestehend aus HD5 (SEQ ID NO: 8) und HD6 (SEQ ID NO: 9), zur Verwendung bei der Behandlung oder Normalisierung einer Dysbiose der Darmflora, des zunehmenden Genreichtums der Darmflora, der Zunahme der Anzahl von Phyla der Darmflora oder der Zunahme der Produktion von kurzkettigen Fettsäuren aus der Darmflora/dem Metabolom, wobei das α-Defensin durch orale Verabreichung an ein Subjekt verabreicht wird, das dessen bedarf.

5. α-Defensin zur Verwendung nach Anspruch 4, wobei die Anzahl der Bakterien, die zu der Gattung *Allobaculum* gehören, erhöht ist.

6. α-Defensin oder β-Defensin zur Verwendung nach einem der vorstehenden Ansprüche, wobei die Behandlung eine Gewichtsreduzierung oder eine Prävention einer Gewichtszunahme und/oder eine Absenkung des Blutzuckers in nüchternem Zustand umfasst.

7. α-Defensin oder β-Defensin zur Verwendung nach einem der vorstehenden Ansprüche, wobei es sich bei dem Defensin um HD5 oder hBD2 handelt.

8. α-Defensin oder β-Defensin zur Verwendung nach einem der vorstehenden Ansprüche, wobei das Defensin ferner mindestens eine zusätzliche Gruppierung umfasst, die ausgewählt ist aus einer Gruppe bestehend aus einem Zellpenetrationspeptid (CPP) einer Albuminbindungsgruppierung (ABM), einer nachweisbaren Gruppierung (Z) und einem die Halbwertszeit verlängerndem Peptid, vorzugsweise einem die Halbwertszeit verlängerndem Peptid, optional wobei das die Halbwertszeit verlängernde Peptid ausgewählt ist aus einer Gruppe bestehend aus einem Molekül, das in der Lage ist, an einen neonatalen Fc-Rezeptor (FcRn) zu binden, Transferrin, Albumin (HAS), XTEN® oder PEG, einem Homoaminosäurepolymer (HAP), einem Prolin-Alanin-Serin-Polymer (PAS) oder einem elastinähnlichem Peptid (ELP), Hyaluronsäure, einem negativ geladenen hochgradig siacylierten Peptid, wie zum Beispiel dem carboxyterminalen Peptid (CTP) der Choriongonadotropin(CG) - β-Kette, menschlichem IgG und CH3(CH2)nCO-, wobei n 8 bis 22 ist.

9. α-Defensin oder β-Defensin zur Verwendung nach einem der vorstehenden Ansprüche, wobei das Subjekt Folgendes aufweist:

   I) einen BMI von 25 oder mehr, wie zum Beispiel 30 oder mehr, zum Beispiel 35 oder mehr, wie zum Beispiel 40 oder mehr;
   II) eine Taille-Hüfte-Verhältnis von mindestens 0,80, zum Beispiel 0,80-0,84, wie zum Beispiel mindestens 0,85 (Frauen) oder mindestens 0,90, zum Beispiel 0,9-0,99, wie zum Beispiel über 1,00 (Männer);

III) einen Blutzucker im nüchternen Zustand von mindestens 6,1 mmol/l, zum Beispiel mindestens 7,0 mmol/l;
IV) einen Spiegel von glykiertem Hämoglobin von mindestens 42 mmol/mol Hb, wie zum Beispiel zwischen 42 und 46 mmol/mol Hb, wie zum Beispiel mindestens 48 mmol/mol Hb;
V) einen erhöhten Blutdruck: ≥ 140/90 mmHg;
VI) Dyslipidämie: Triglyceride (TG): ≥ 1,695 mmol/l und Lipoprotein hoher Dichte/Cholesterin (HDL-C) ≤ 0,9 mmol/l (Männer), ≤ 1,0 mmol/l (Frauen); und/oder
VII) Mikroalbuminurie: Urin-Albumin-Ausscheidungsverhältnis ≥ 20 µg/min oder Albumin-Kreatinin-Verhältnis ≥ 30 mg/g.

**10.** α-Defensin oder β-Defensin zur Verwendung nach einem der vorstehenden Ansprüche, wobei das β-Defensin in einer täglichen Dosis zwischen 0,1 mg hBD2/kg und 10 mg hBD2/kg an ein Subjekt verabreicht wird, das dessen bedarf; oder
wobei das α-Defensin in einer täglichen Dosis zwischen 0,1 mg HD5/kg und 10 mg HD5/kg an ein Subjekt verabreicht wird, das dessen bedarf.

**11.** α-Defensin oder β-Defensin zur Verwendung nach einem der vorstehenden Ansprüche, wobei das Defensin in Kombination mit Insulin/Insulinanaloga und/oder glucagonähnlichem Peptid-1 (GLP1)/GLP-1-Analoga und/oder glucagonähnlichem Peptid-2 (GLP2)/GLP-2-Analoga und/oder Dipeptidylpeptidase-IV(DPP-IV)-Hemmer und/oder Metformin und/oder einem Natrium/Glucose-Transporter-2(SGLT-2)-Hemmer und/oder einem Glucagonrezeptor-Antagonisten und/oder einem Transient-Receptor-Potential-Kation-Kanal-Unterfamilie-V-Mitglied-1(TRPV1)-Antagonisten oder einer Kombination davon verabreicht wird, optional wobei das GLP-1 analog ausgewählt ist aus Exenatid, Liraglutid, Lixisenatid, Albiglutid und Dulaglutid; oder
lispro, aspart, glulisin, Insulin detemir, Insulin degludec, und Insulin glargin.

**12.** α-Defensin oder β-Defensin zur Verwendung nach einem der vorstehenden Ansprüche, wobei das α-Defensin oder β-Defensin mindestens einmal täglich, wie zum Beispiel einmal am Tag, zweimal am Tag oder dreimal am Tag, an ein Subjekt verabreicht wird, das dessen bedarf.

**13.** α-Defensin oder β-Defensin zur Verwendung nach einem der Ansprüche 3 bis 5, wobei das Subjekt einer Antibiotikabehandlung oder Chemotherapie oder Immuntherapie oder immunsuppressiven Therapie oder einer anderen Behandlung, die negative Effekte auf die Darmflora aufweist, unterzogen wurde und/oder unterzogen wird.

**14.** Verfahren zur Förderung von magerem Wachstum bei Nutztieren, wobei das Verfahren eine Verabreichung einer wirksamen Menge eines Säuger-α-Defensins und/oder -β-Defensins, ausgewählt aus der Gruppe bestehend aus hBD1 (SEQ ID NO: 4), hBD2 (SEQ ID NO: 5), hBD3 (SEQ ID NO: 6), hBD4 (SEQ ID NO: 7), gekürztem hBD2 (SEQ ID NO: 17) oder einem β-Defensin, das sich von einem von hBD1, hBD2, hBD3, hBD4 oder gekürztem hBD2 (SEQ ID NO: 17) um weniger als 5 konservative Aminosäuresubstitutionen unterscheidet, HD5 (SEQ ID NO: 8) und HD6 (SEQ ID NO: 9), an ein Subjekt, das dessen bedarf, umfasst, wobei die Nutztiere ausgewählt sind aus Kühen, Schweinen, Schafen, Ziegen, Pferden, Enten, Gänsen, Tauben, Truthähnen, Wachteln und Hühnern.

**15.** Verfahren nach Anspruch 14, wobei das Säuger-α-Defensin und/oder -β-Defens in durch Mischen in das Tierfutter verabreicht wird.

## Revendications

**1.** β-défensine choisie dans le groupe constitué de hBD1 (SEQ ID NO: 4), hBD2 (SEQ ID NO: 5), hBD3 (SEQ ID NO: 6), hBD4 (SEQ ID NO: 7), hBD2 tronquée (SEQ ID NO: 17) ou β-défensine qui diffère de l'une des hBD1, hBD2, hBD3, hBD4 ou hBD2 tronquée (SEQ ID NO: 17) par moins de 5 substitutions conservatrices d'acides aminés à utiliser dans le traitement du diabète de type 2, du syndrome métabolique, de l'inflammation systémique de bas grade, de l'obésité, de la résistance à l'insuline et/ou de l'intolérance au glucose ; ladite β-défensine étant administrée par voie orale ou sous-cutanée à un sujet qui en a besoin.

**2.** α-défensine choisie dans le groupe constitué de HD5 (SEQ ID NO: 8) et HD6 (SEQ ID NO: 9) à utiliser dans le traitement du diabète de type 2, du syndrome métabolique, de l'obésité, de la résistance à l'insuline et/ou de l'intolérance au glucose ; ladite α-défensine étant administrée par voie orale à un sujet qui en a besoin.

**3.** β-défensine choisie dans le groupe constitué de hBD1 (SEQ ID NO: 4), hBD2 (SEQ ID NO: 5), hBD3 (SEQ ID NO:

6), hBD4 (SEQ ID NO: 7), hBD2 tronquée (SEQ ID NO: 17) ou défensine qui diffère de l'une des hBD1, hBD2, hBD3, hBD4 ou hBD2 tronquée (SEQ ID NO: 17) par moins de 5 substitutions conservatrices d'acides aminés à utiliser dans le traitement de l'augmentation de la richesse génétique du microbiote intestinal et/ou de l'augmentation du nombre de phylums du microbiote intestinal, la β-défensine étant administrée par voie orale ou sous-cutanée à un sujet qui en a besoin.

**4.** α-défensine choisie dans le groupe constitué de HD5 (SEQ ID NO: 8) et HD6 (SEQ ID NO: 9) à utiliser dans le traitement ou la normalisation d'un microbiote dysbiotique dans l'intestin, l'augmentation de la richesse génétique du microbiote intestinal, l'augmentation du nombre de phylums du microbiote intestinal, ou l'augmentation de la production d'acides gras à chaîne courte à partir du microbiote/métabolome intestinal, l'α-défensine étant administrée par voie orale à un sujet qui en a besoin.

**5.** α-défensine à utiliser selon la revendication 4, dans laquelle le nombre de bactéries appartenant au genre *Allobaculum* est augmenté.

**6.** α-défensine ou β-défensine à utiliser selon l'une quelconque des revendications précédentes, dans laquelle le traitement comprend une réduction de poids ou la prévention d'une prise de poids, et/ou une diminution de la glycémie à jeun.

**7.** α-défensine ou β-défensine à utiliser selon l'une quelconque des revendications précédentes, la défensine étant HD5 ou hBD2.

**8.** α-défensine ou β-défensine à utiliser selon l'une quelconque des revendications précédentes, ladite défensine comprenant en outre au moins un fragment supplémentaire choisi dans un groupe constitué d'un peptide pénétrant dans les cellules (CPP), d'un fragment de liaison à l'albumine (ABM), d'un fragment détectable (Z), et d'un peptide d'extension de demi-vie, de préférence un peptide d'extension de demi-vie, éventuellement dans laquelle le peptide d'extension de demi-vie est choisi dans un groupe constitué d'une molécule pouvant se lier à un récepteur Fc néonatal (FcRn), d'une transferrine, d'une albumine (HAS), de XTEN® ou de PEG, d'un homopolymère d'acides aminés (HAP), d'un polymère de proline-alanine-sérine (PAS) ou d'un peptide de type élastine (PEL), d'un acide hyaluronique, d'un peptide hautement sialylé chargé négativement tel que le peptide carboxy-terminal (CTP) de la chaîne β de la gonadotrophine chorionique (CG), d'IgG humaine et de CH3(CH2)nCO- où n est compris entre 8 et 22.

**9.** α-défensine ou β-défensine à utiliser selon l'une quelconque des revendications précédentes, dans laquelle le sujet présente :

I) un IMC de 25 ou plus, tel que 30 ou plus, par exemple 35 ou plus, tel que 40 ou plus ;
II) un rapport taille/hanches d'au moins 0,80, par exemple de 0,80 à 0,84, tel qu'au moins 0,85 (femme) ou au moins 0,90, par exemple de 0,9 à 0,99, tel que supérieur à 1,00 (homme) ;
III) une glycémie à jeun d'au moins 6,1 mmol/l, par exemple d'au moins 7,0 mmol/l ;
IV) un taux d'hémoglobine glyquée d'au moins 42 mmol/mol de Hb, tel que de 42 à 46 mmol/mol de Hb, tel qu'au moins 48 mmol/mol de Hb ;
V) tension artérielle élevée : ≥ 140/90 mmHg ;
VI) dyslipidémie : triglycérides (TG) : ≥ 1,695 mmol/L et cholestérol à lipoprotéines de haute densité (HDL-C) ≤ 0,9 mmol/L (homme), ≤ 1,0 mmol/L (femme) ; et/ou
VII) microalbuminurie : taux d'excrétion urinaire d'albumine ≥ 20 μg/min ou rapport albumine/créatinine ≥ 30 mg/g.

**10.** α-défensine ou β-défensine à utiliser selon l'une quelconque des revendications précédentes, la β-défensine étant administrée à un sujet qui en a besoin à raison d'une dose quotidienne comprise entre 0,1 mg de hBD2/kg et 10 mg de hBD2/kg ; ou
l'α-défensine étant administrée à un sujet qui en a besoin à raison d'une dose quotidienne comprise entre 0,1 mg de HD5/kg et 10 mg de HD5/kg.

**11.** α-défensine ou β-défensine à utiliser selon l'une quelconque des revendications précédentes, ladite défensine étant administrée en association avec de l'insuline/des analogues de l'insuline et/ou du peptide-1 de type glucagon (GLP-1)/des analogues du GLP-1 et/ou du peptide 2 de type glucagon (GLP-2)/des analogues du GLP-2 et/ou un inhibiteur de la dipeptidyl peptidase IV (DPP-IV) et/ou un inhibiteur de la metformine et/ou du transporteur 2 du sodium-glucose (SGLT-2) et/ou un antagoniste du récepteur du glucagon et/ou un antagoniste du membre 1 de la sous-famille V du canal cationique potentiel de récepteur transitoire (TRPV1) ou une combinaison de ceux-ci, éventuellement dans laquelle l'analogue du GLP-1 est choisi parmi l'exénatide, le liraglutide, le lixisénatide, l'albiglutide et le dulaglutide ; ou la lispro, l'asparte, la glulisine, l'insuline détémir, l'insuline dégludec et l'insuline glargine.

**12.** α-défensine ou β-défensine à utiliser selon l'une quelconque des revendications précédentes, l'α-défensine ou la β-défensine étant administrée à un sujet qui en a besoin au moins une fois par jour, comme une fois par jour, deux fois par jour ou trois fois par jour.

**13.** α-défensine ou β-défensine à utiliser selon l'une quelconque des revendications 3 à 5, dans laquelle le sujet a entrepris et/ou entreprend un traitement antibiotique ou une chimiothérapie ou une immunothérapie ou une thérapie immunosuppressive ou un autre traitement qui a des effets négatifs sur le microbiote intestinal.

**14.** Procédé destiné à favoriser la croissance musculaire du bétail, le procédé comprenant l'administration d'une quantité efficace d'une α-défensine et/ou β-défensine de mammifère choisie dans le groupe constitué de hBD1 (SEQ ID NO: 4), hBD2 (SEQ ID NO: 5), hBD3 (SEQ ID NO: 6), hBD4 (SEQ ID NO: 7), hBD2 tronquée (SEQ ID NO: 17), une β-défensine qui diffère de l'une des hBD1, hBD2, hBD3, hBD4 ou hBD2 tronquée (SEQ ID NO: 17) par moins de 5 substitutions conservatrices d'acides aminés, HD5 (SEQ ID NO: 8) et HD6 (SEQ ID NO: 9) à un sujet qui en a besoin, dans lequel le bétail est choisi parmi les vaches, les porcins, les moutons, les caprins, les chevaux, les canards, les oies, les pigeons, les dindes, les cailles et les poulets.

**15.** Procédé selon la revendication 14, dans lequel l'α-défensine et/ou la β-défensine de mammifère est administrée en la mélangeant à l'alimentation animale.

Fig. 1A

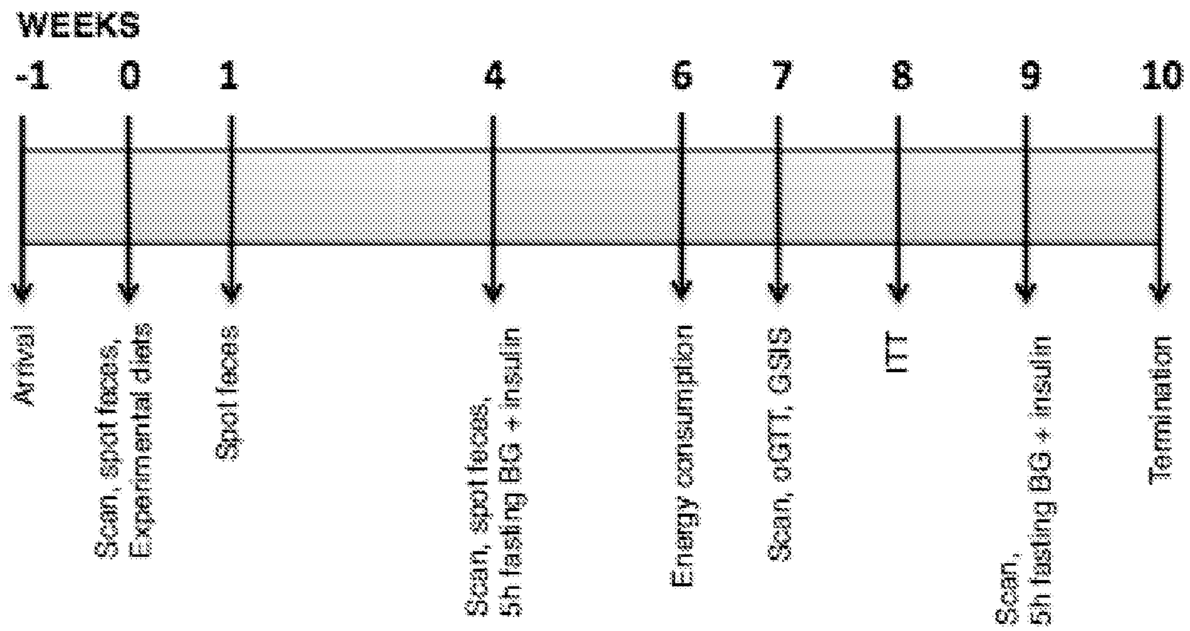
WEEKS
-1
0
1
4
6
7
8
9
10
Arrival
Scan, spot feces, Experimental diets
Spot feces
Scan, spot feces, 5h fasting BG + insulin
Energy consumption
Scan, oGTT, GSIS
ITT
Scan, 5h fasting BG + insulin
Termination

Fig. 1B

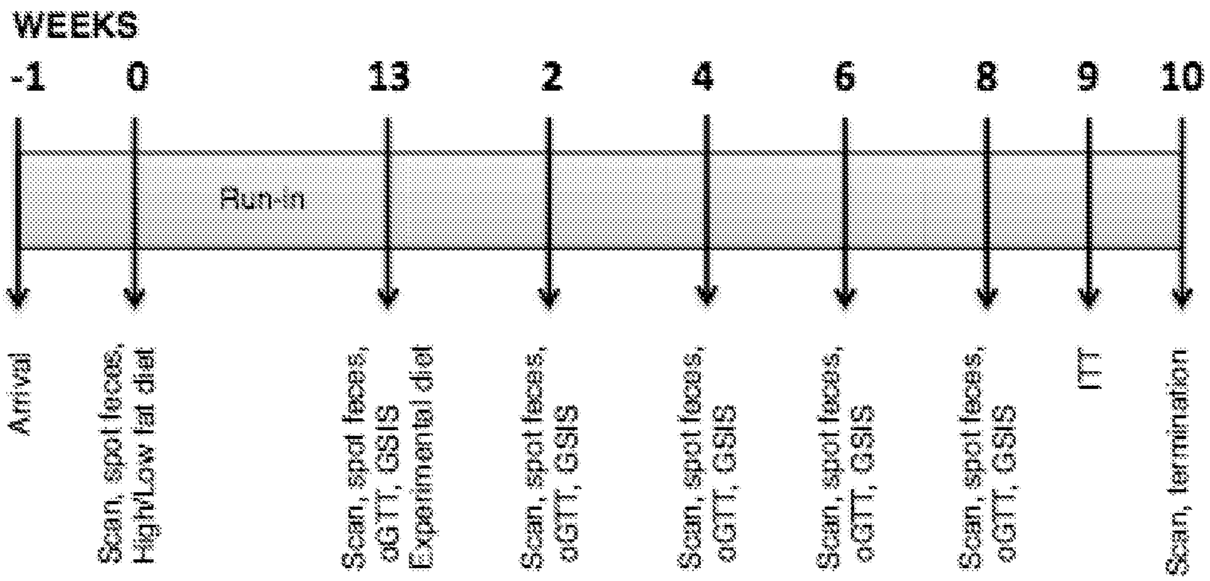
WEEKS
-1
0
13
2
4
6
8
9
10
Run-in
Arrival
Scan, spot feces, High/Low fat diet
Scan, spot feces, oGTT, GSIS Experimental diet
Scan, spot feces, oGTT, GSIS
Scan, spot feces, oGTT, GSIS
Scan, spot feces, oGTT, GSIS
Scan, spot feces, oGTT, GSIS
ITT
Scan, termination

Fig. 2

```
HBD1            ------DHYNCVSSGGQCLYSACPIFTKIQGTCYRGKAKCCK---
HBD2            ---GIGDPVTCLKSGAICHPVFCPRRYKQIGTCGLPGTKCCKKP-
HBD3            GIINTLQKYYCRVRGGRCAVLSCLPKEEQIGKCSTRGRKCCRRKK
HBD4            -----ELDRICGYGTARCR-KKCRSQEYRIGRCPN-TYACCLRK-
                          *    . *    *       * *      **
```

Fig. 3

```
HD5             -ATCYCRTGRCATRESLSGVCEISGRLYRLCCR
HD6             AFTCHCRR-SCYSTEYSYGTCTVMGINHRFCCL
                  **:**   * : *   *.* : *  :*:**
```

Fig. 4

```
HNP2            -CYCRIPACIAGERRYGTCIYQGRLWAFCC
HNP3            DCYCRIPACIAGERRYGTCIYQGRLWAFCC
HNP1            ACYCRIPACIAGERRYGTCIYQGRLWAFCC
                 *****************************
```

Fig. 5

```
hBD2            ---GIGDPVTCLKSGAICHPVFCPRRYKQIGTCGLPGTKCCKKP---
Chimpanzee      ---GISDPVTCLKSGAICHPVFCPRRYKQIGTCGLPGTKCCKKP---
Macaque         ---GIGDPVTCLKNGAICHPVFCPRRYKQIGTCGLPGTKCCKKP---
Orangutan       VFGDISNPVTCLRSGAICHPGFCPRRYKHIGTCGLSVIKCCKKP---
Goat            ---GIINHRSCYRNKGVCAPARCPRNMRQIGTCHGPPVKCCRK----
Bovine          ---GVGNPVSCVRNKGICVPIRCPGSMKQIGTCVGRAVKCCRK----
Horse           ---GIGNPISCARNRGVCIPIGCLPGMKQIGTCGLPGTKCCRK----
Porcine         ---NIGNSVSCLRNKGVCMPGKCAPKMKQIGTCGMPQVKCCKR----
Mouse           ---KINNPVSCLRKGGRCWN-RCIGNTRQIGSCGVPFLKCCKRK---
Chicken         ----------LFCKGGSCHFGGCPSHLIKVGSCFR---SCCKWPWNA
                             . . *    *     ::*:*     .**:
```

Fig. 6

A

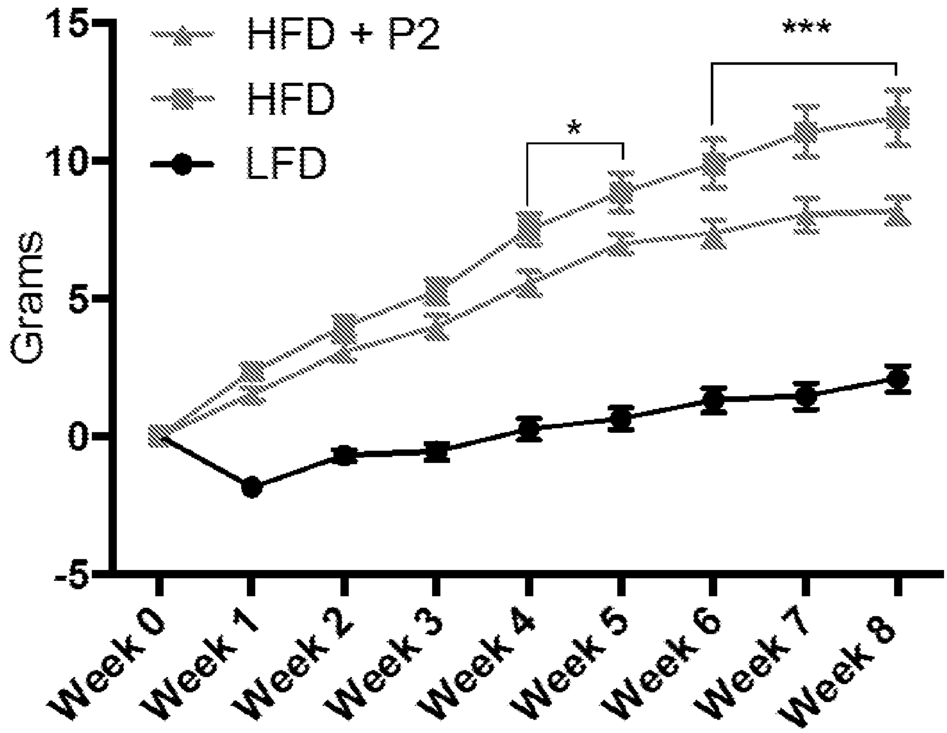
15
10
5
0
-5
Grams
HFD + P2
HFD
LFD
*
***
Week 0
Week 1
Week 2
Week 3
Week 4
Week 5
Week 6
Week 7
Week 8

B

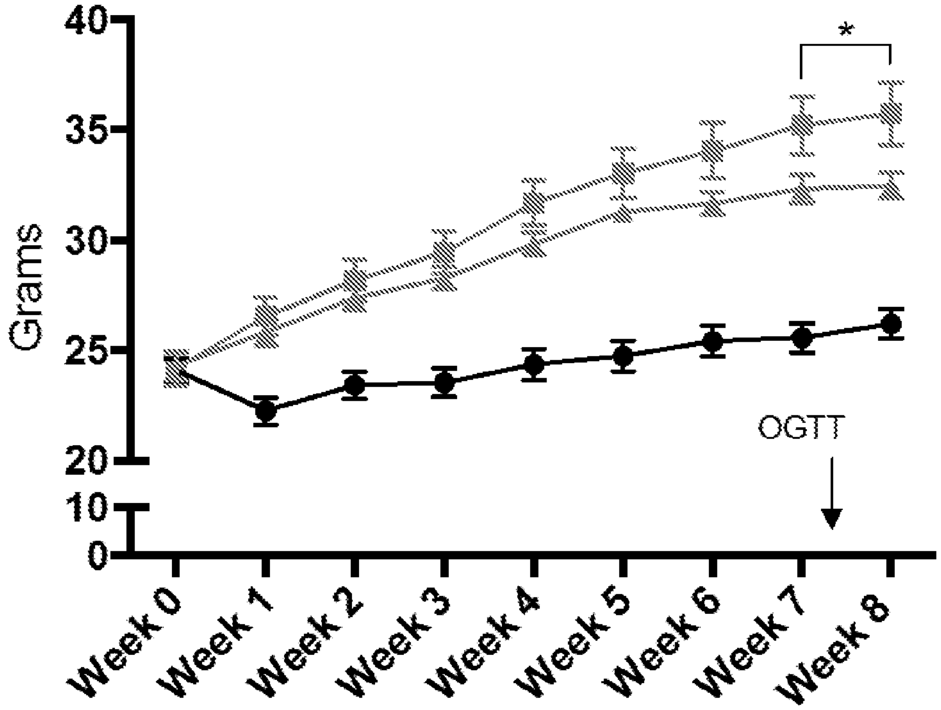
40
35
30
25
20
10
0
Grams
*
OGTT
Week 0
Week 1
Week 2
Week 3
Week 4
Week 5
Week 6
Week 7
Week 8

C

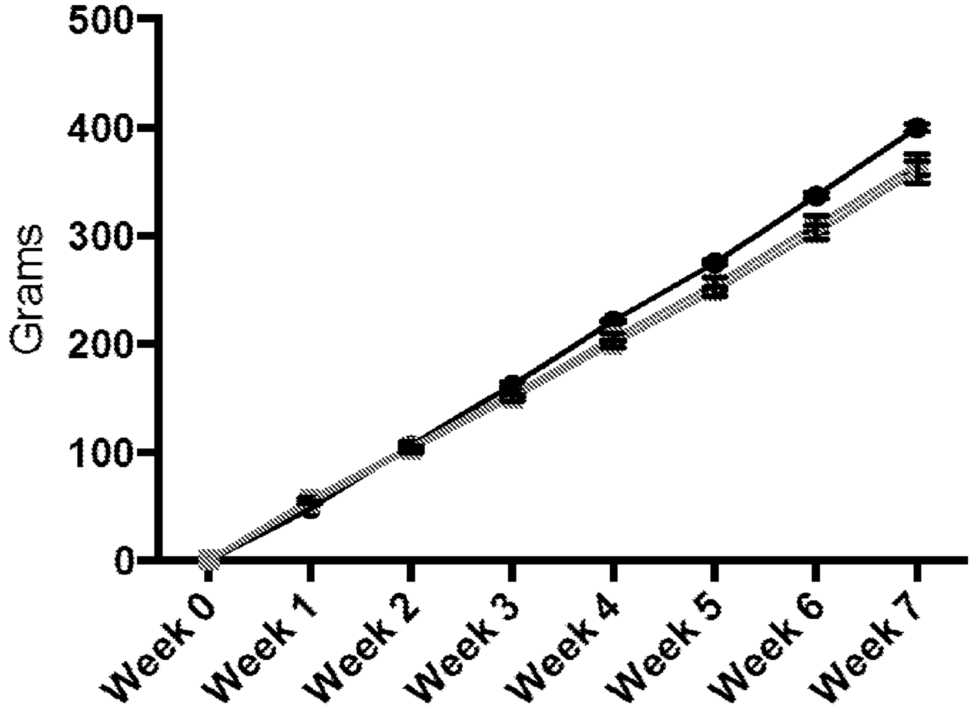
500
400
300
200
100
0
Grams
Week 0
Week 1
Week 2
Week 3
Week 4
Week 5
Week 6
Week 7

Fig. 7

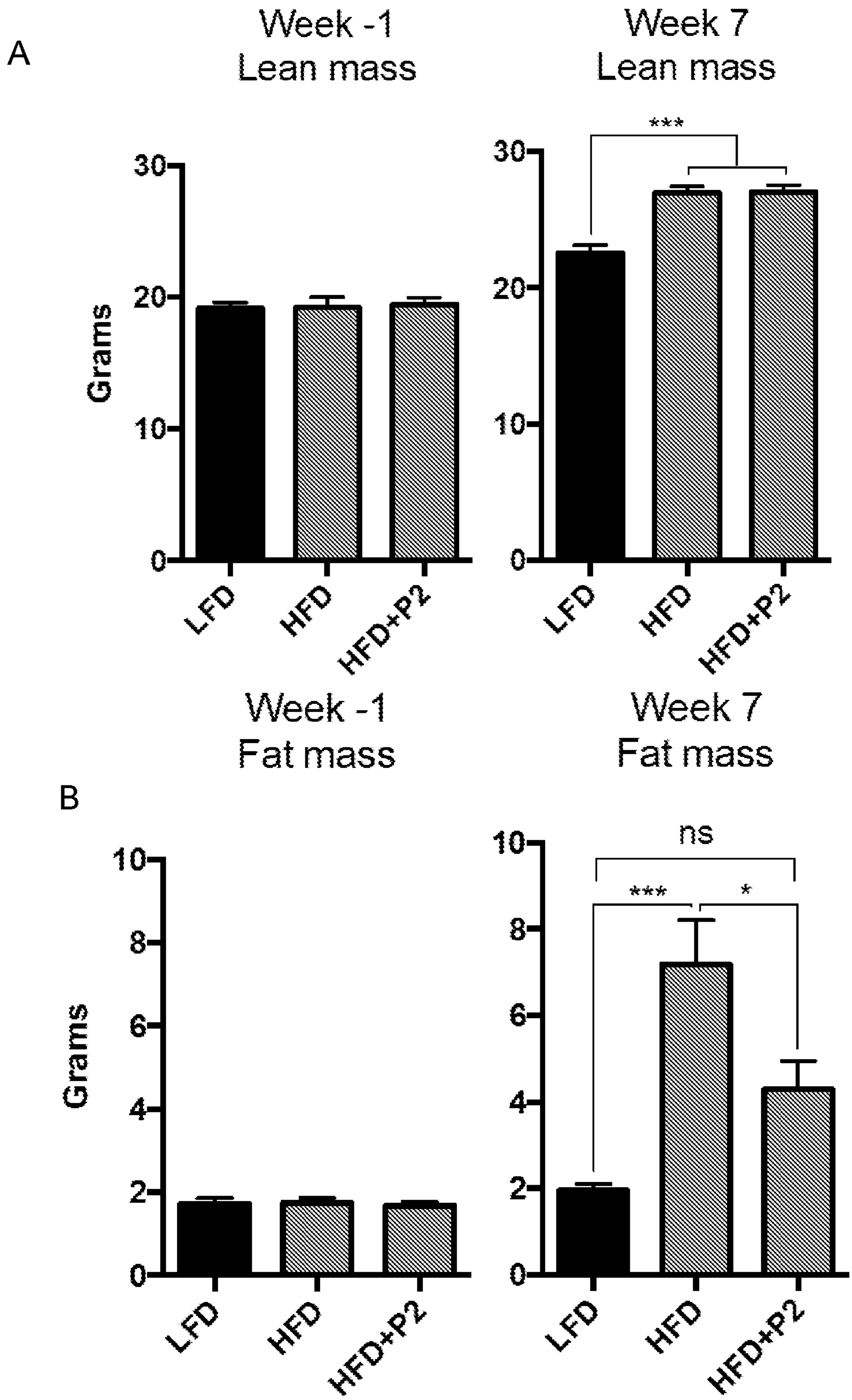
A
Week -1
Lean mass
Week 7
Lean mass
Grams
30
20
10
0
***
LFD
HFD
HFD+P2
B
Week -1
Fat mass
Week 7
Fat mass
Grams
10
8
6
4
2
0
ns
***
*

Fig. 8

A

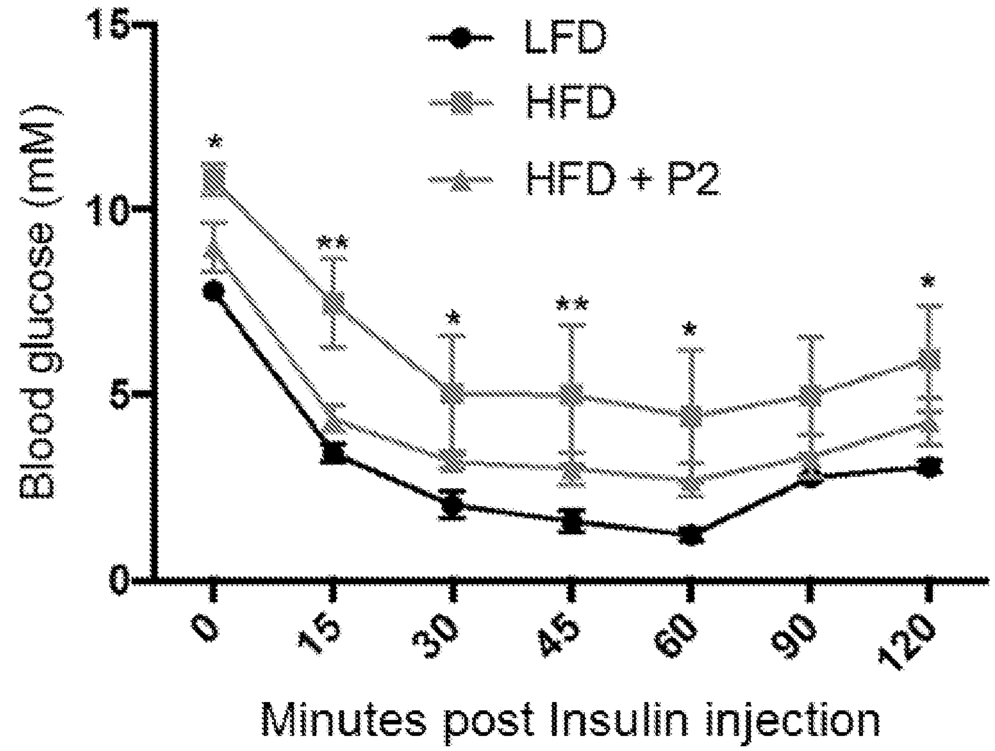
15
10
5
0
Blood glucose (mM)
LFD
HFD
HFD + P2
*
**
*
**
*
*
0
15
30
45
60
90
120
Minutes post Insulin injection

B

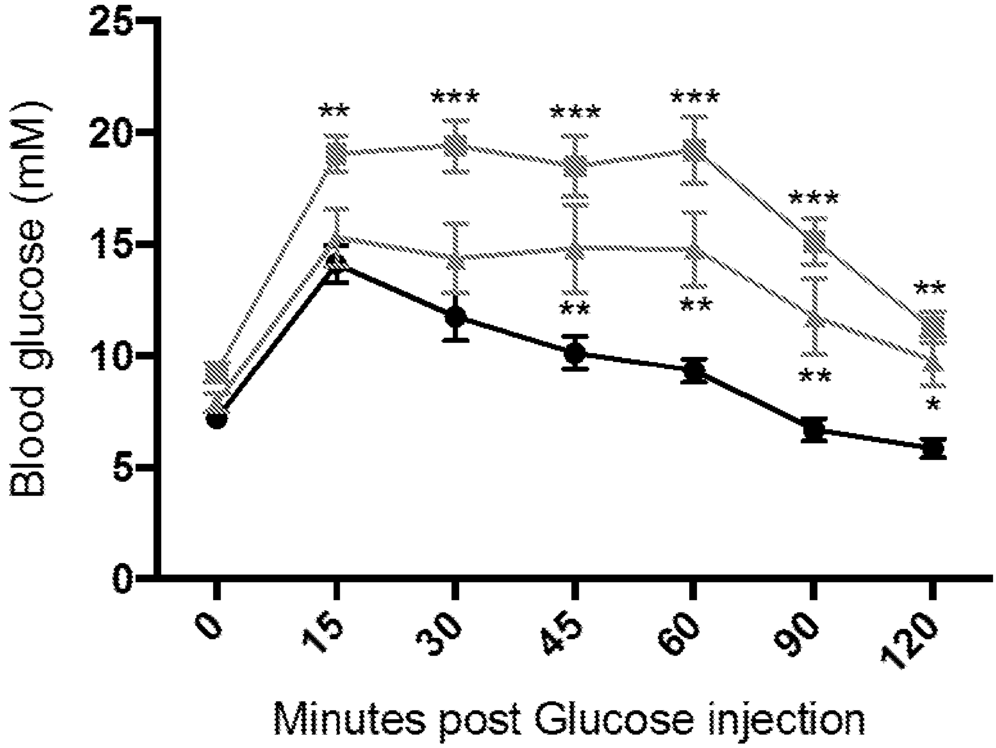
25
20
15
10
5
0
Blood glucose (mM)
**
***
***
***
***
**
**
**
**
*
0
15
30
45
60
90
120
Minutes post Glucose injection

C

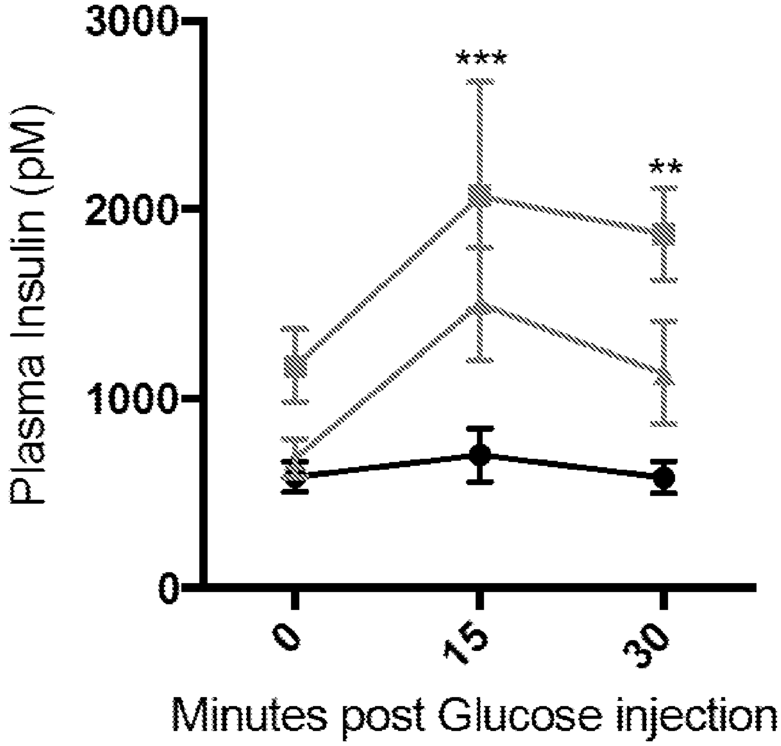
3000
2000
1000
0
Plasma Insulin (pM)
***
**
0
15
30
Minutes post Glucose injection

D

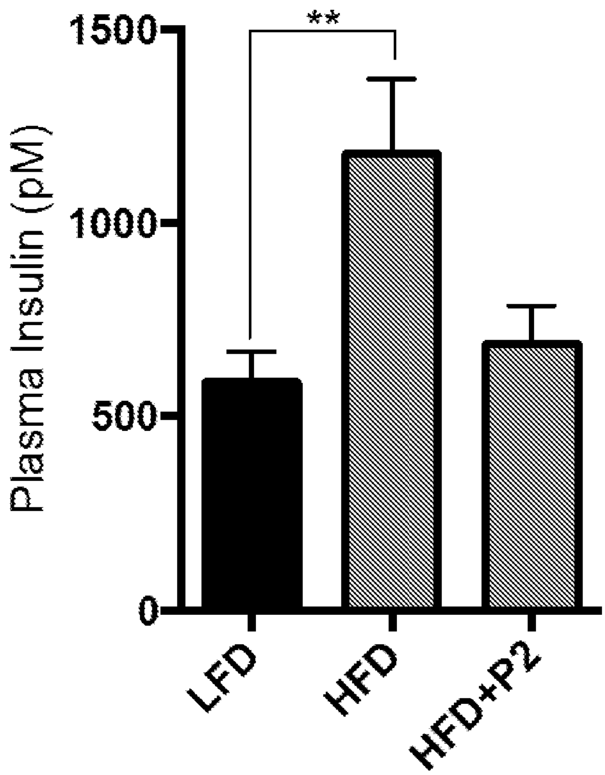
1500
1000
500
0
Plasma Insulin (pM)
**
LFD
HFD
HFD+P2

Fig. 9A

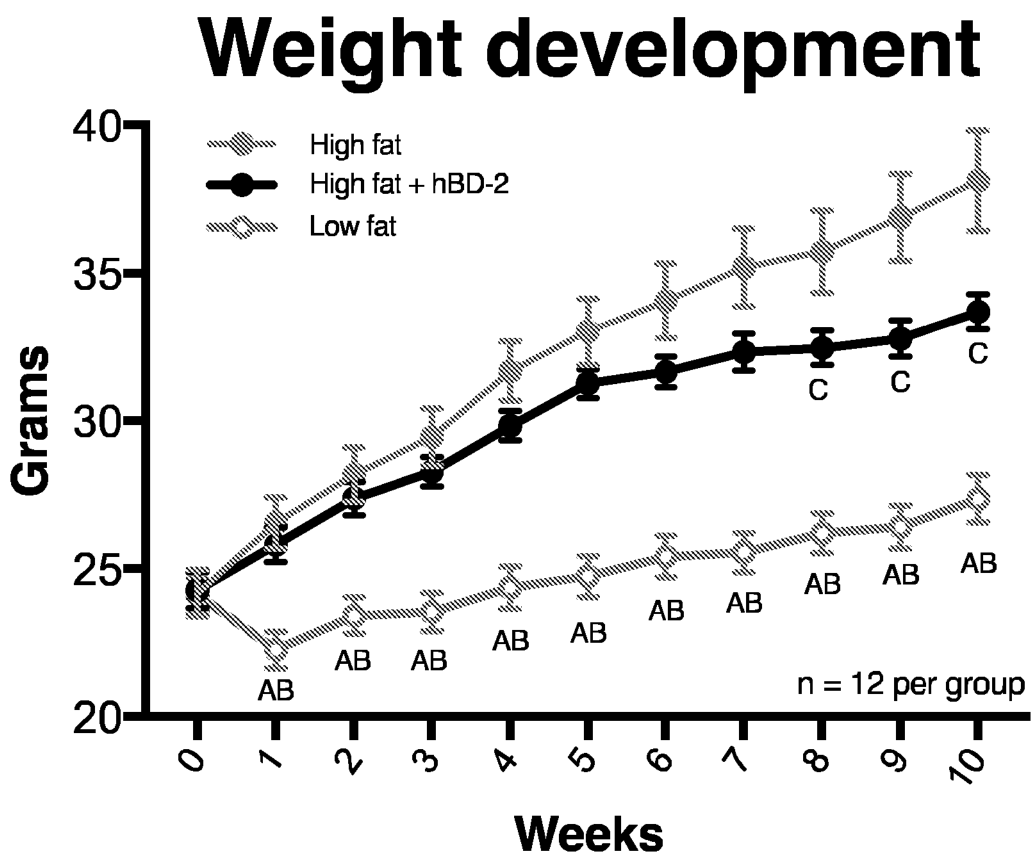
Weight development
High fat
High fat + hBD-2
Low fat
Grams
40
35
30
25
20
C
C
C
AB
AB
AB
AB
AB
AB
AB
AB
AB
AB
n = 12 per group
0
1
2
3
4
5
6
7
8
9
10
Weeks

Fig. 9B

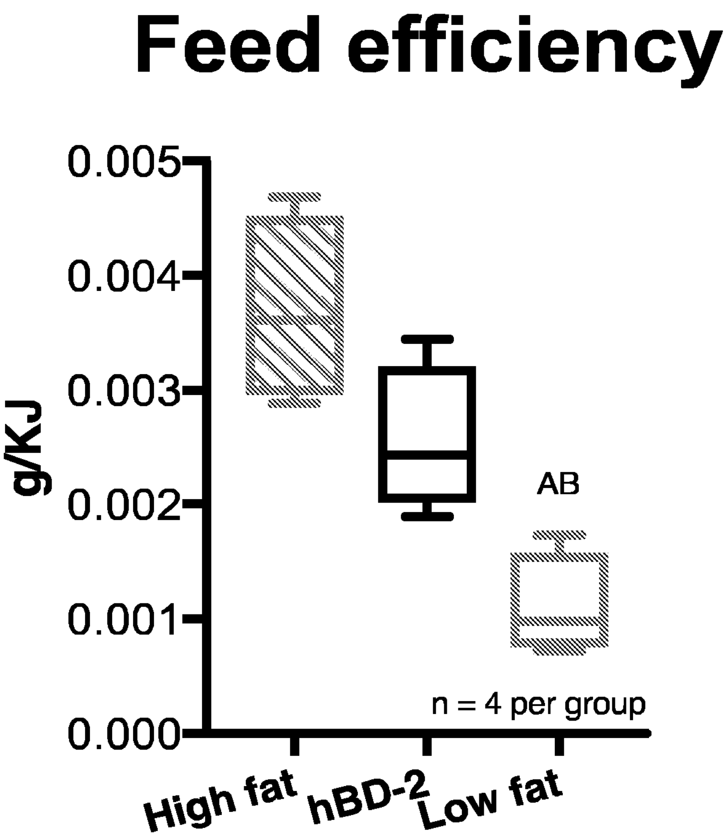
Feed efficiency
g/KJ
0.005
0.004
0.003
0.002
0.001
0.000
AB
n = 4 per group
High fat
hBD-2
Low fat

Fig. 9C

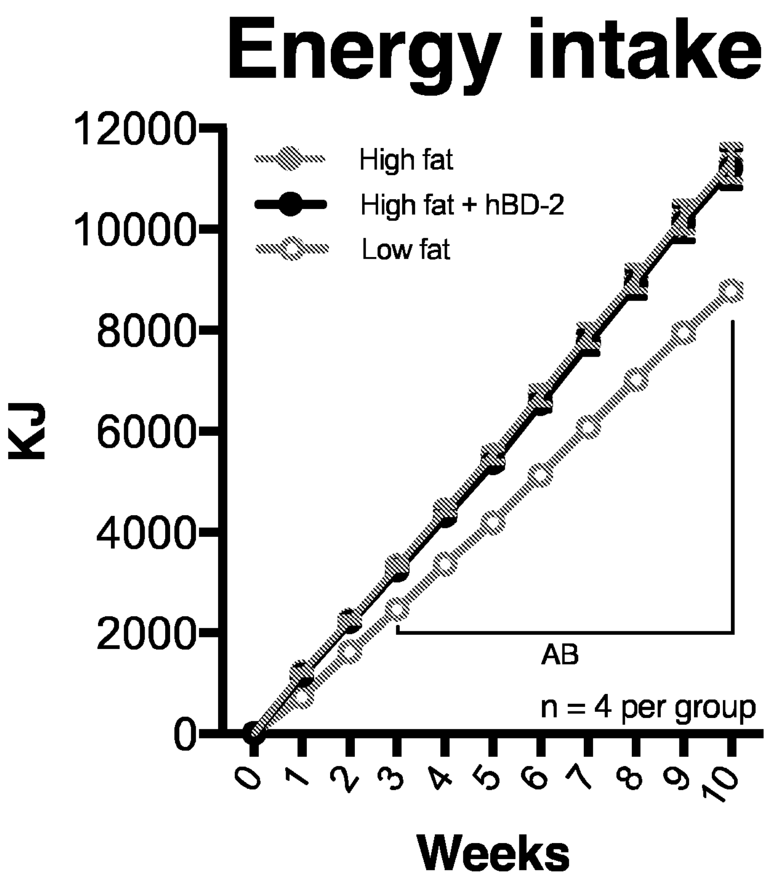
Energy intake
KJ
12000
10000
8000
6000
4000
2000
0
High fat
High fat + hBD-2
Low fat
AB
n = 4 per group
0
1
2
3
4
5
6
7
8
9
10
Weeks

Fig. 10A

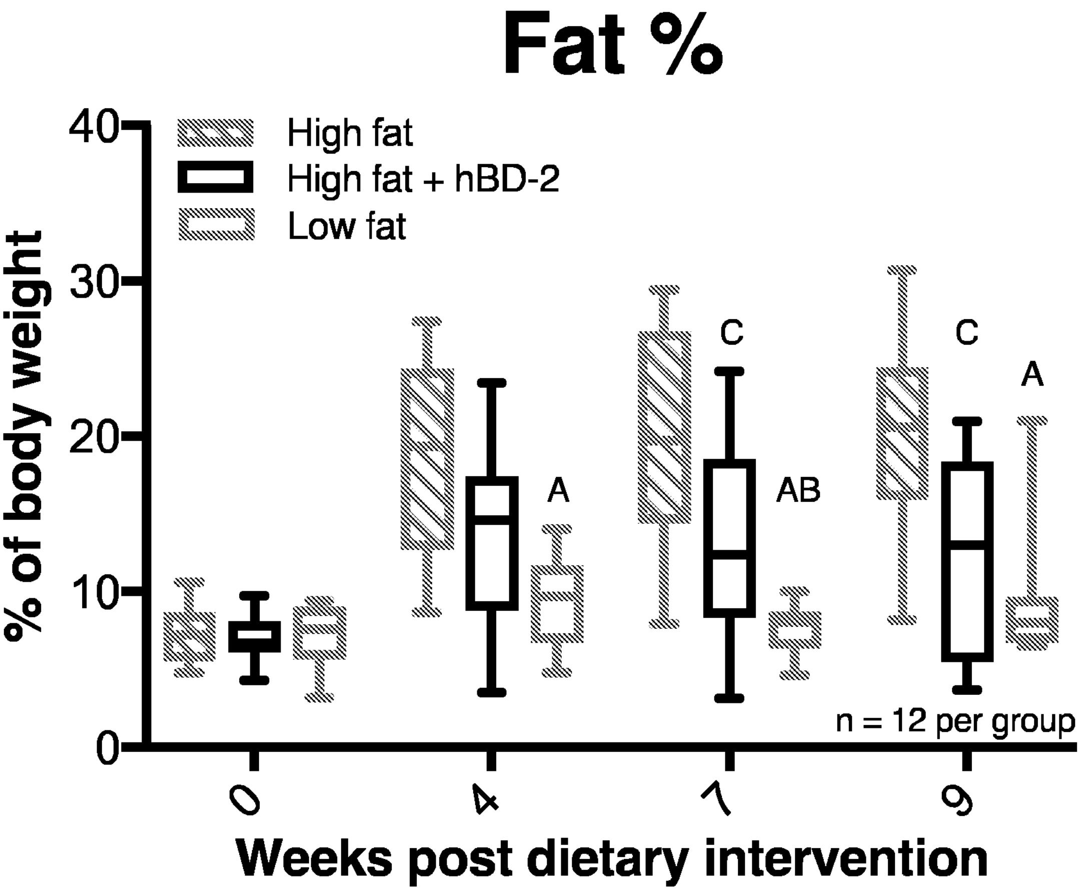
Fat %
High fat
High fat + hBD-2
Low fat
% of body weight
40
30
20
10
0
C
C
A
A
AB
n = 12 per group
0
4
7
9
Weeks post dietary intervention

Fig. 10B

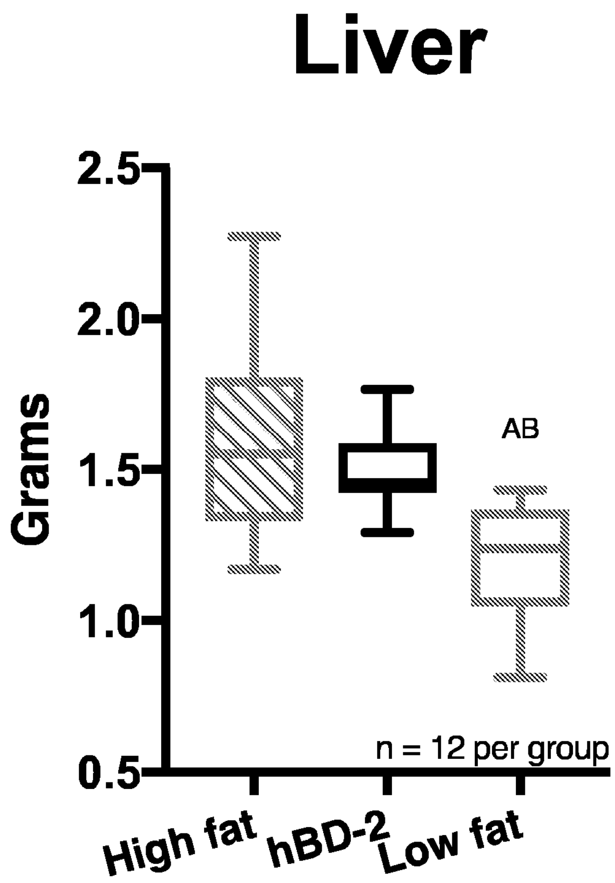


Fig. 10C

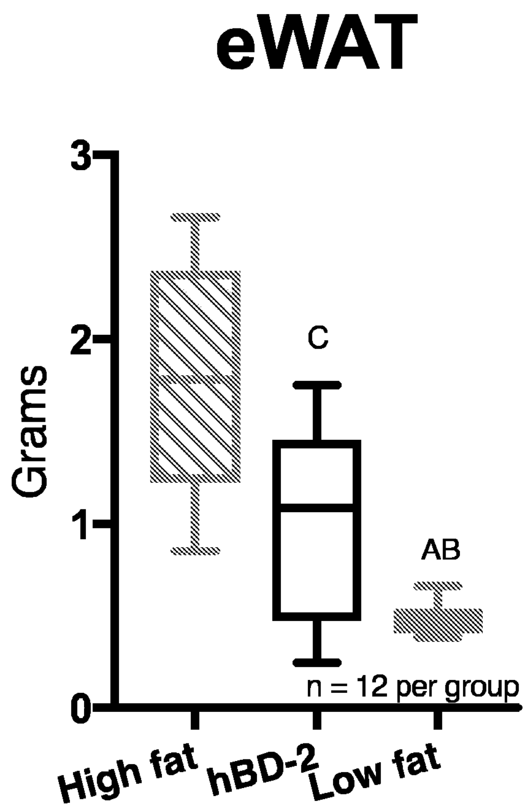

Fig. 11A

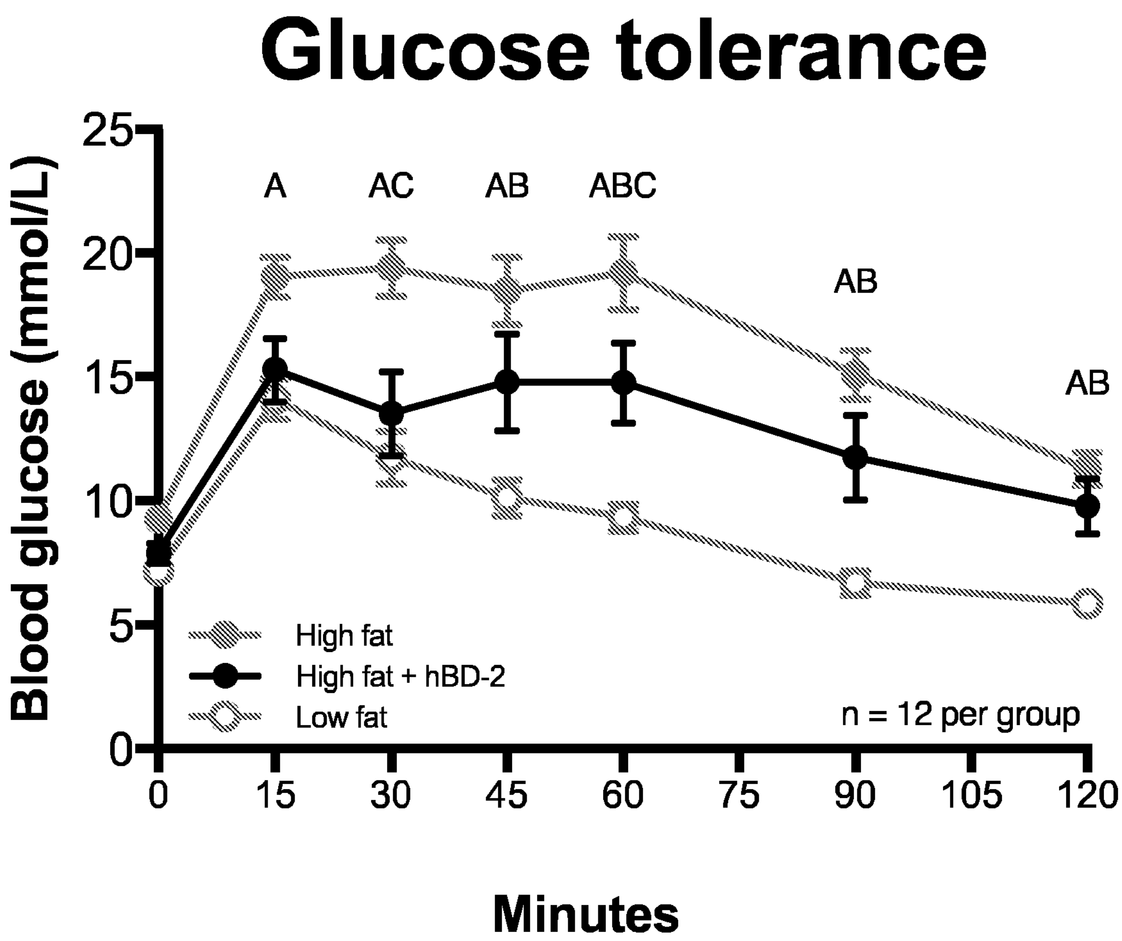
Glucose tolerance
Blood glucose (mmol/L)
25
20
15
10
5
0
A
AC
AB
ABC
AB
AB
High fat
High fat + hBD-2
Low fat
n = 12 per group
0
15
30
45
60
75
90
105
120
Minutes

Fig. 11B

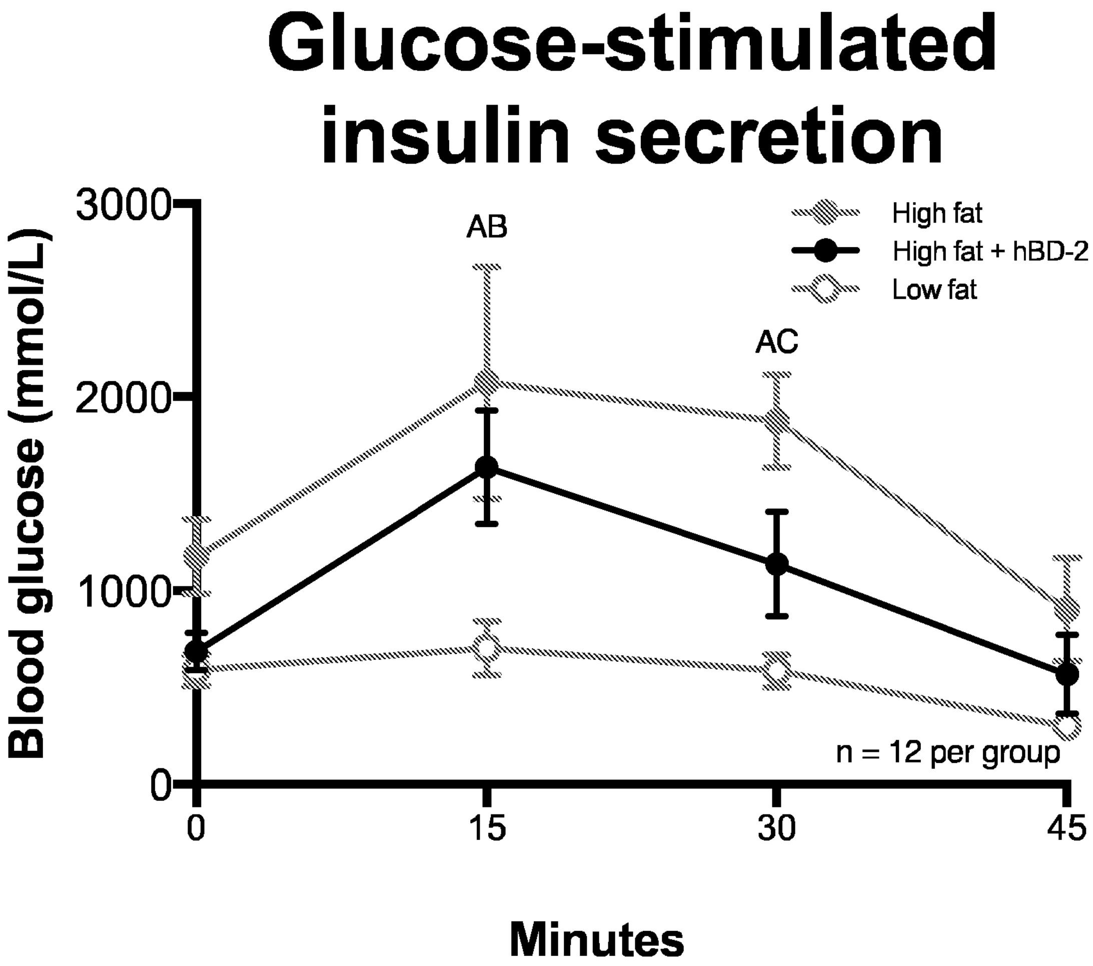
Glucose-stimulated insulin secretion
Blood glucose (mmol/L)
3000
2000
1000
0
0
15
30
45
Minutes
AB
AC
High fat
High fat + hBD-2
Low fat
n = 12 per group

Fig. 12A

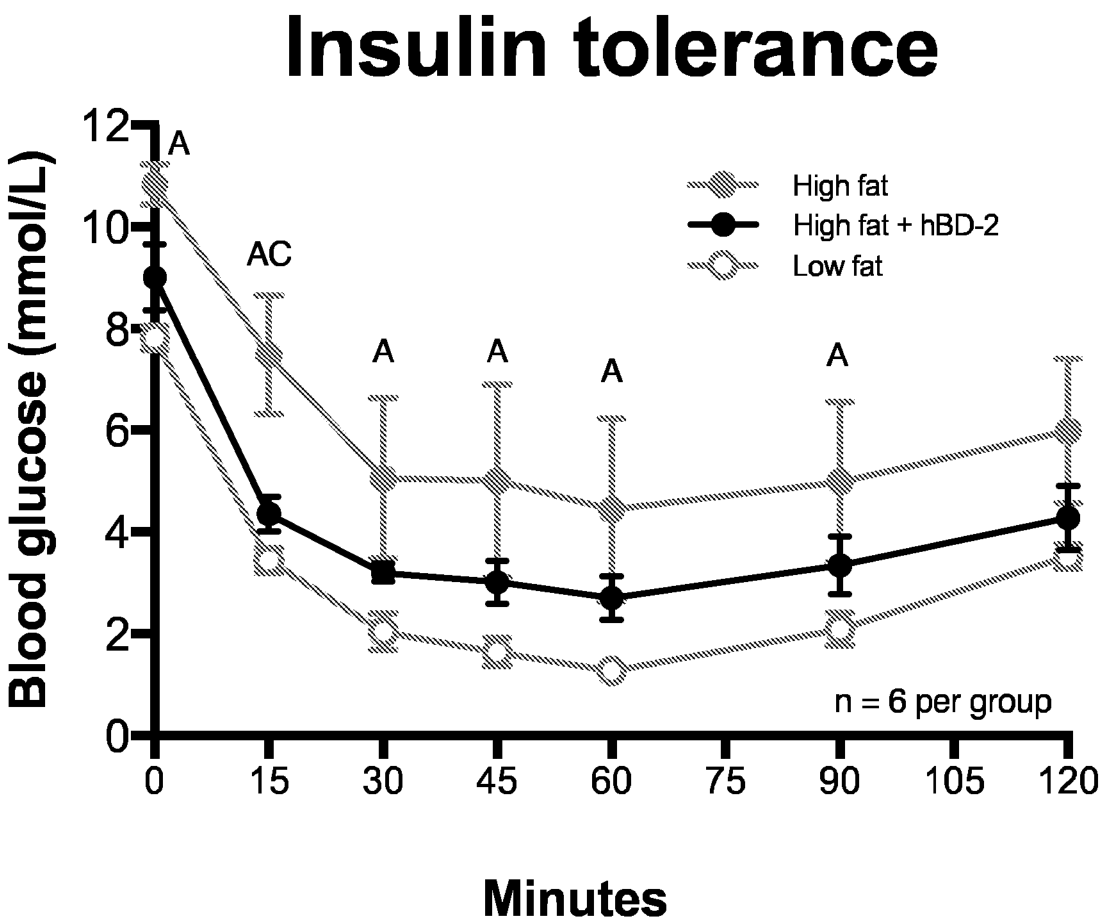
Insulin tolerance
Blood glucose (mmol/L)
0
2
4
6
8
10
12
Minutes
0
15
30
45
60
75
90
105
120
A
AC
A
A
A
A
High fat
High fat + hBD-2
Low fat
n = 6 per group

Fig. 12B

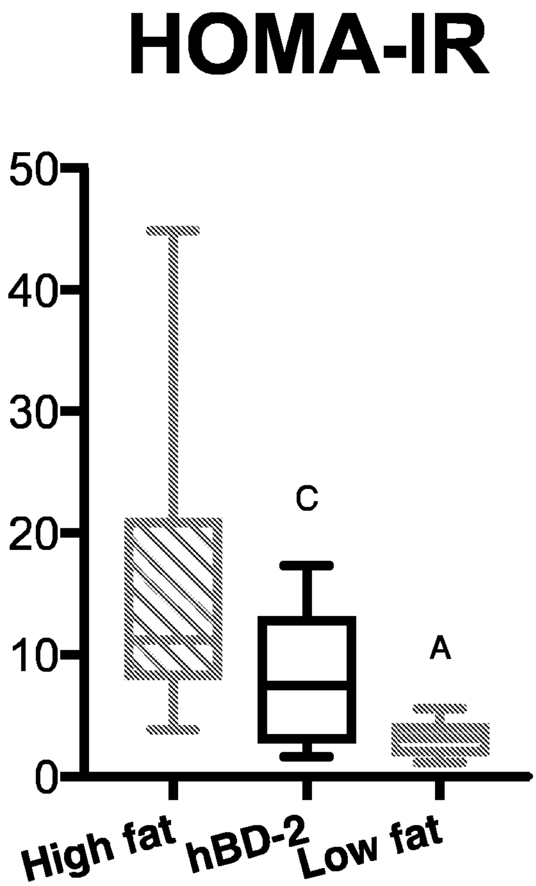

Fig. 13A

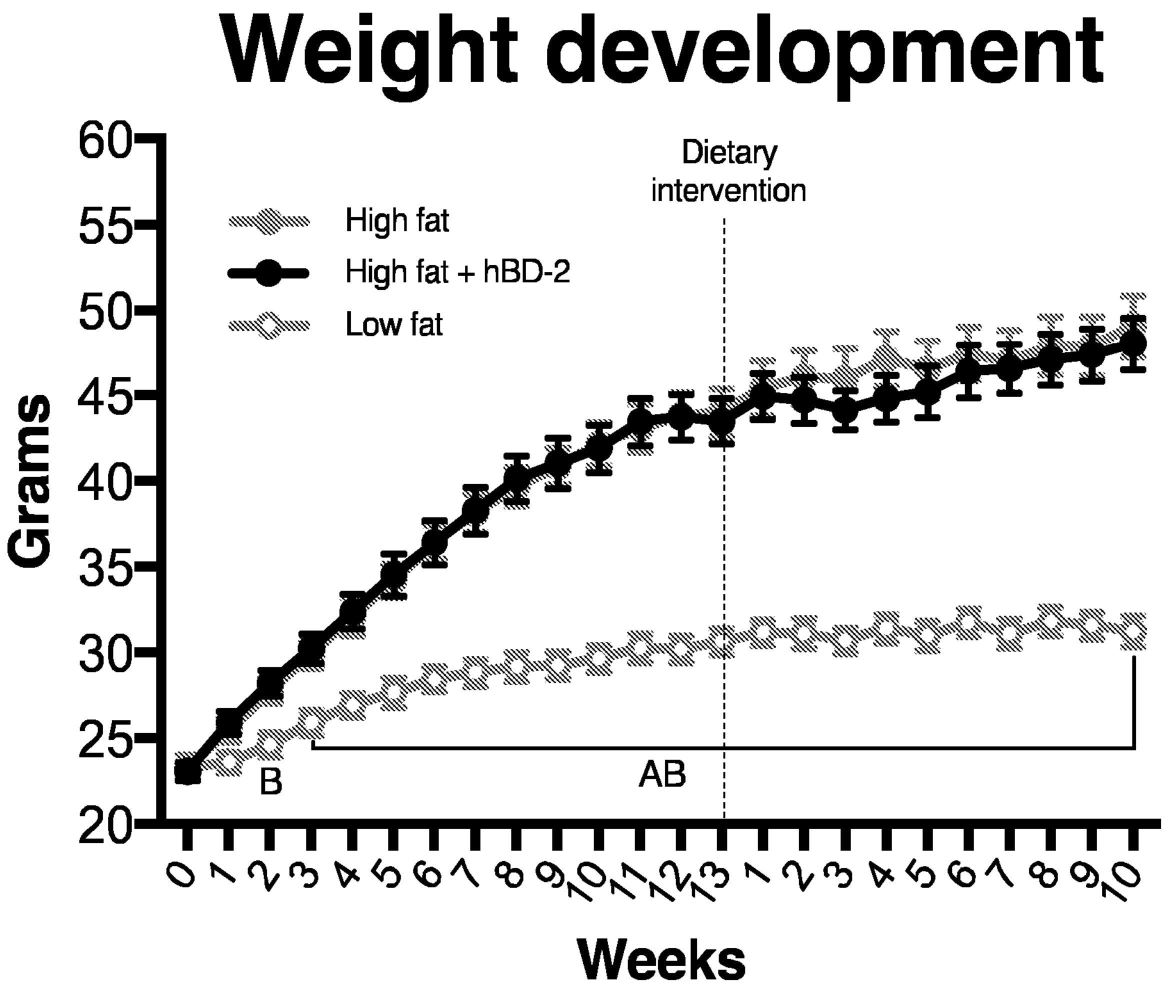
Weight development
Dietary intervention
High fat
High fat + hBD-2
Low fat
Grams
60
55
50
45
40
35
30
25
20
B
AB
0 1 2 3 4 5 6 7 8 9 10 11 12 13 1 2 3 4 5 6 7 8 9 10
Weeks

Fig. 13B

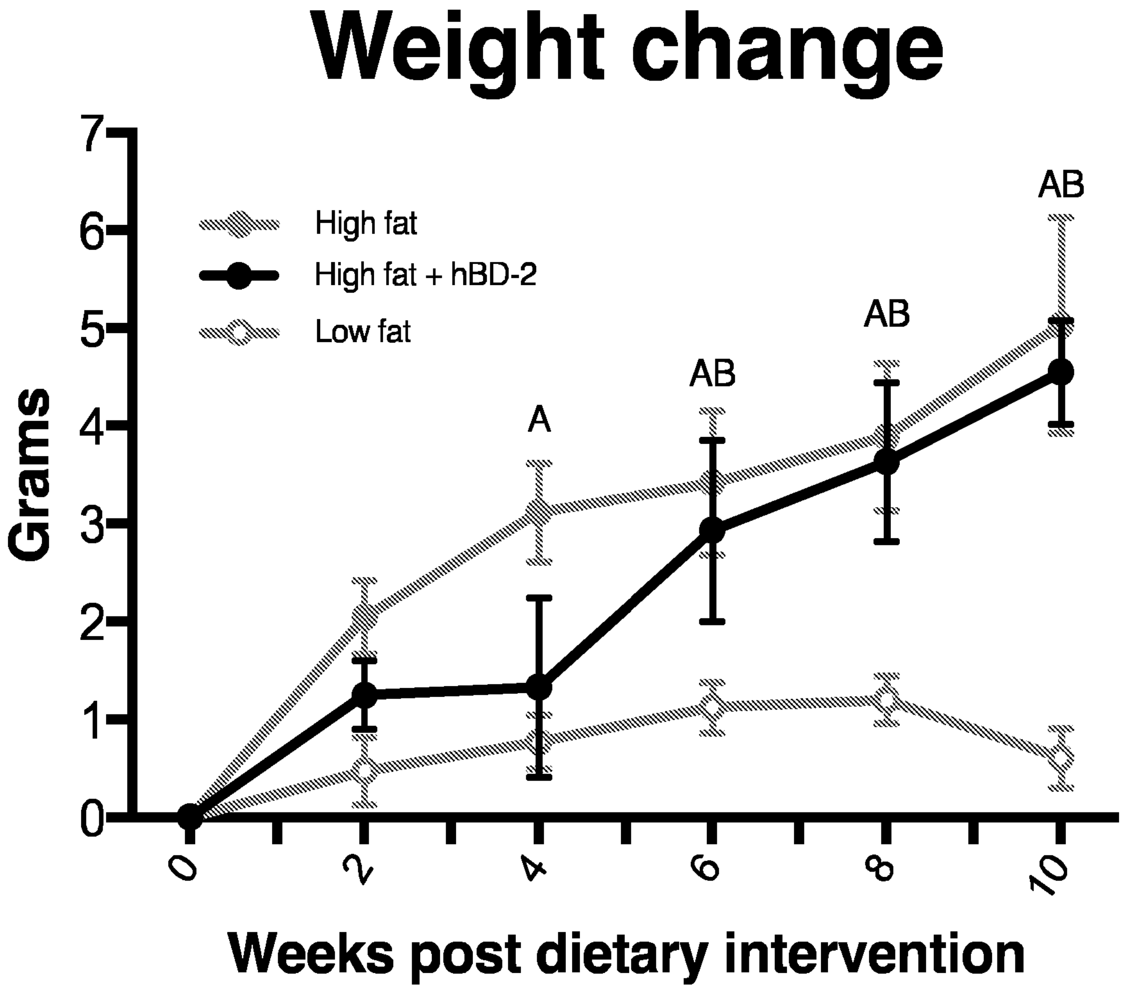
Weight change
High fat
High fat + hBD-2
Low fat
Grams
7
6
5
4
3
2
1
0
A
AB
AB
AB
AB
0
2
4
6
8
10
Weeks post dietary intervention

Fig. 14A

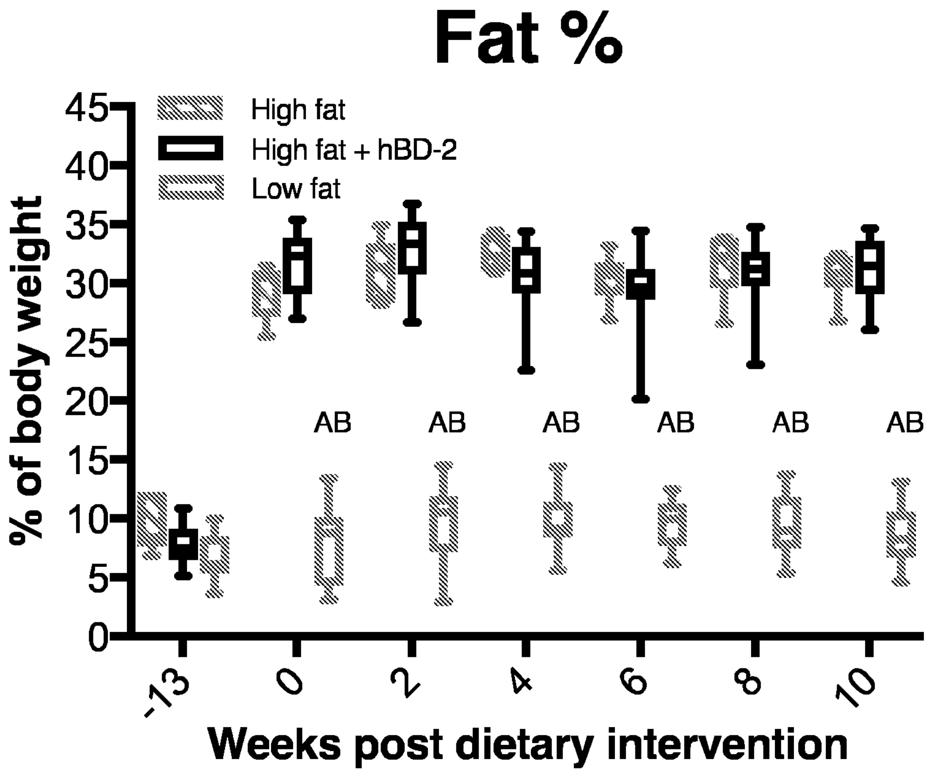


Fig. 14B

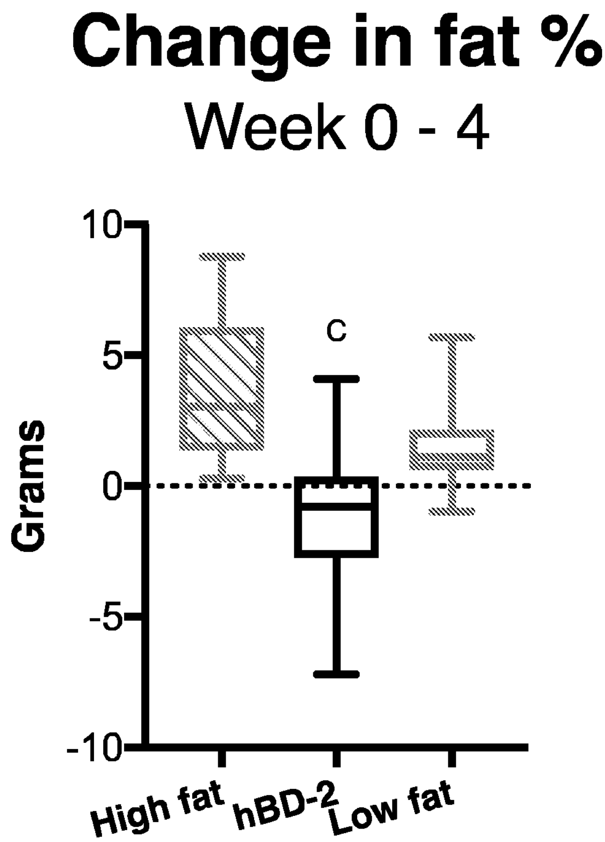

Fig. 15A

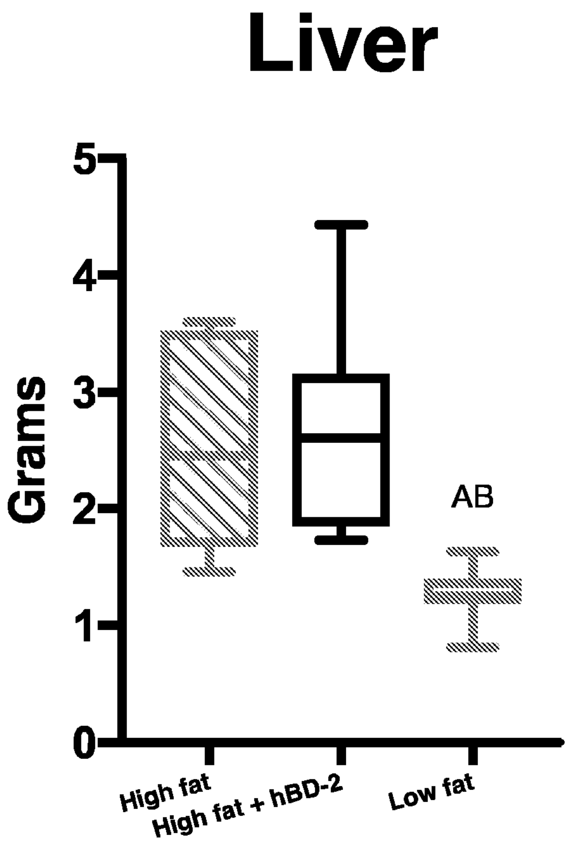


Fig. 15B

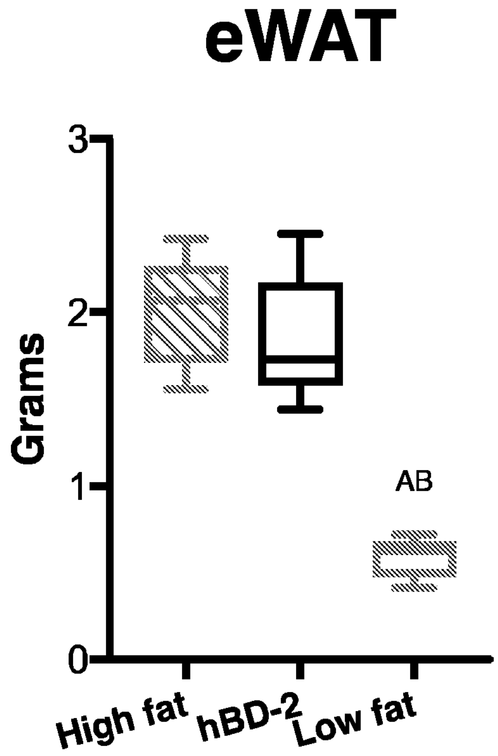

Fig. 16A

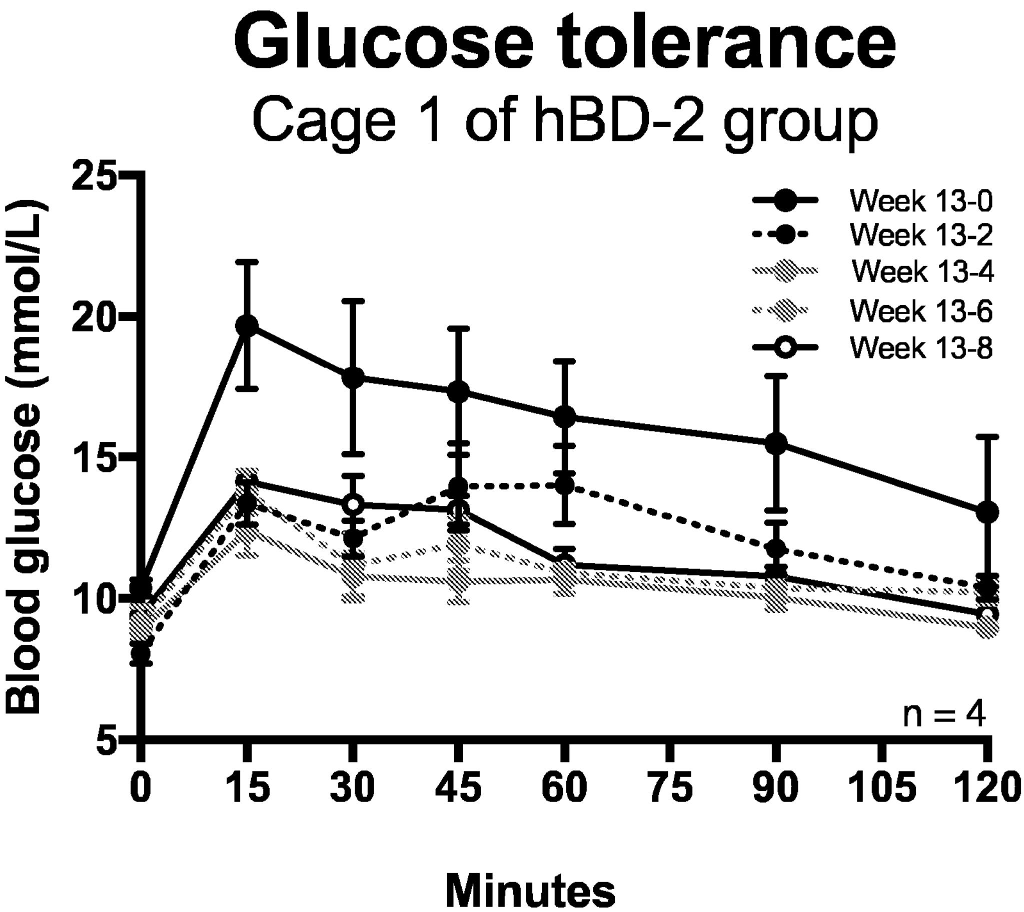
Glucose tolerance
Cage 1 of hBD-2 group
Blood glucose (mmol/L)
25
20
15
10
5
0
15
30
45
60
75
90
105
120
Minutes
Week 13-0
Week 13-2
Week 13-4
Week 13-6
Week 13-8
n = 4

Fig. 16B

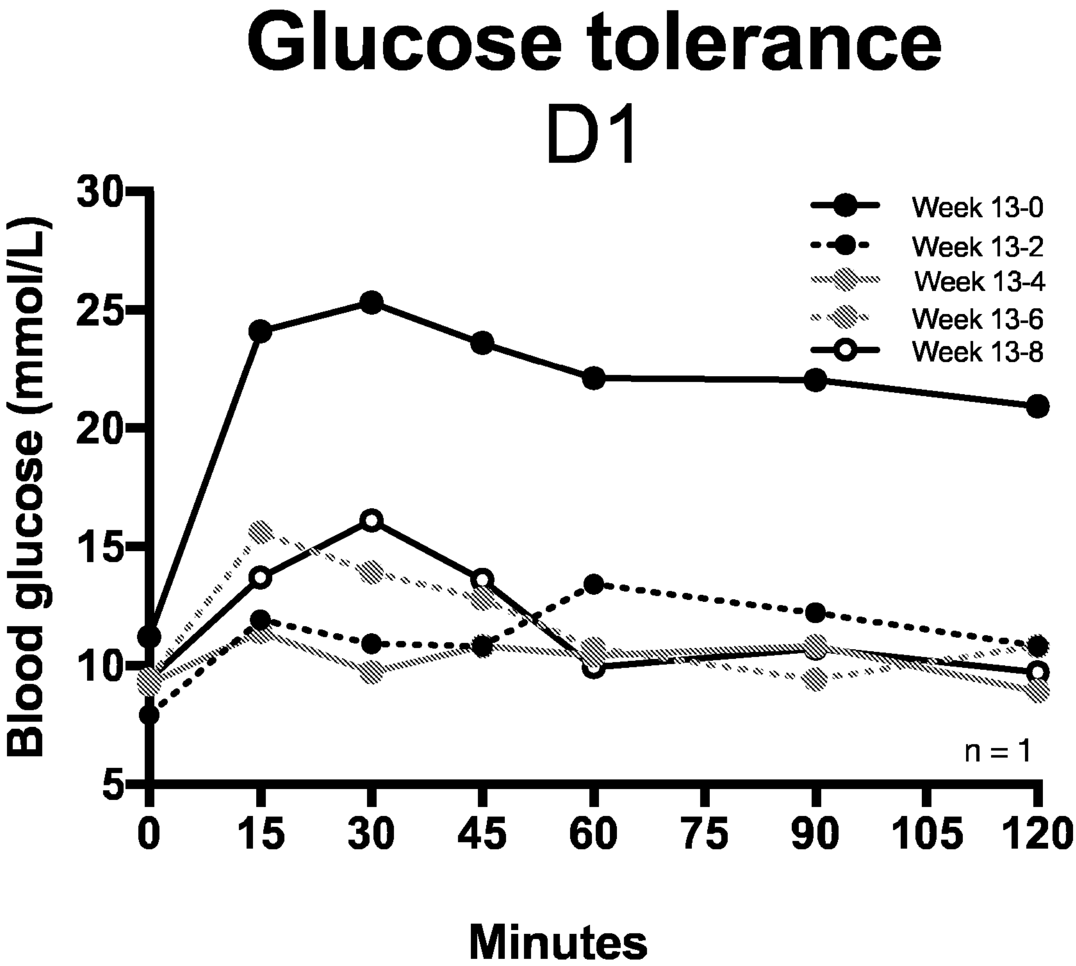
Glucose tolerance
D1
Blood glucose (mmol/L)
30
25
20
15
10
5
Week 13-0
Week 13-2
Week 13-4
Week 13-6
Week 13-8
n = 1
0
15
30
45
60
75
90
105
120
Minutes

Fig 16C

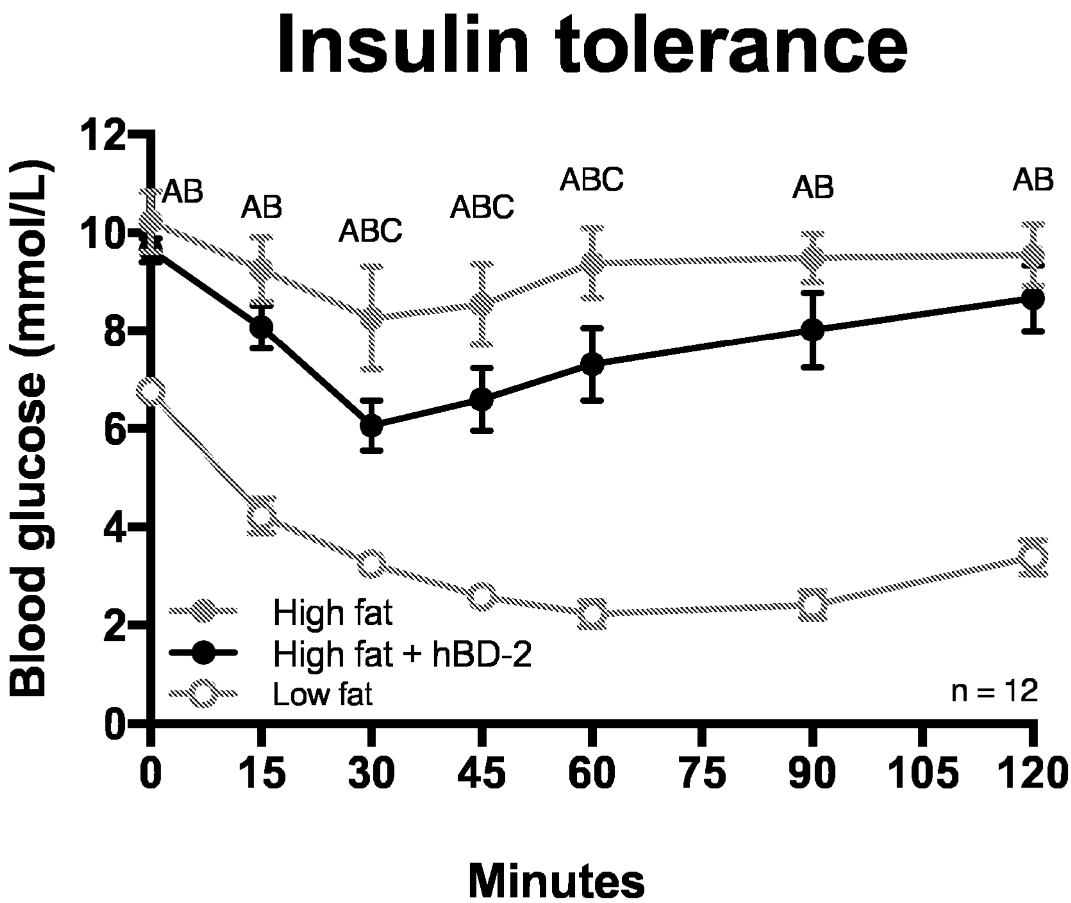
Insulin tolerance
Blood glucose (mmol/L)
12
10
8
6
4
2
0
AB
AB
ABC
ABC
ABC
AB
AB
High fat
High fat + hBD-2
Low fat
n = 12
0
15
30
45
60
75
90
105
120
Minutes

Fig. 17A

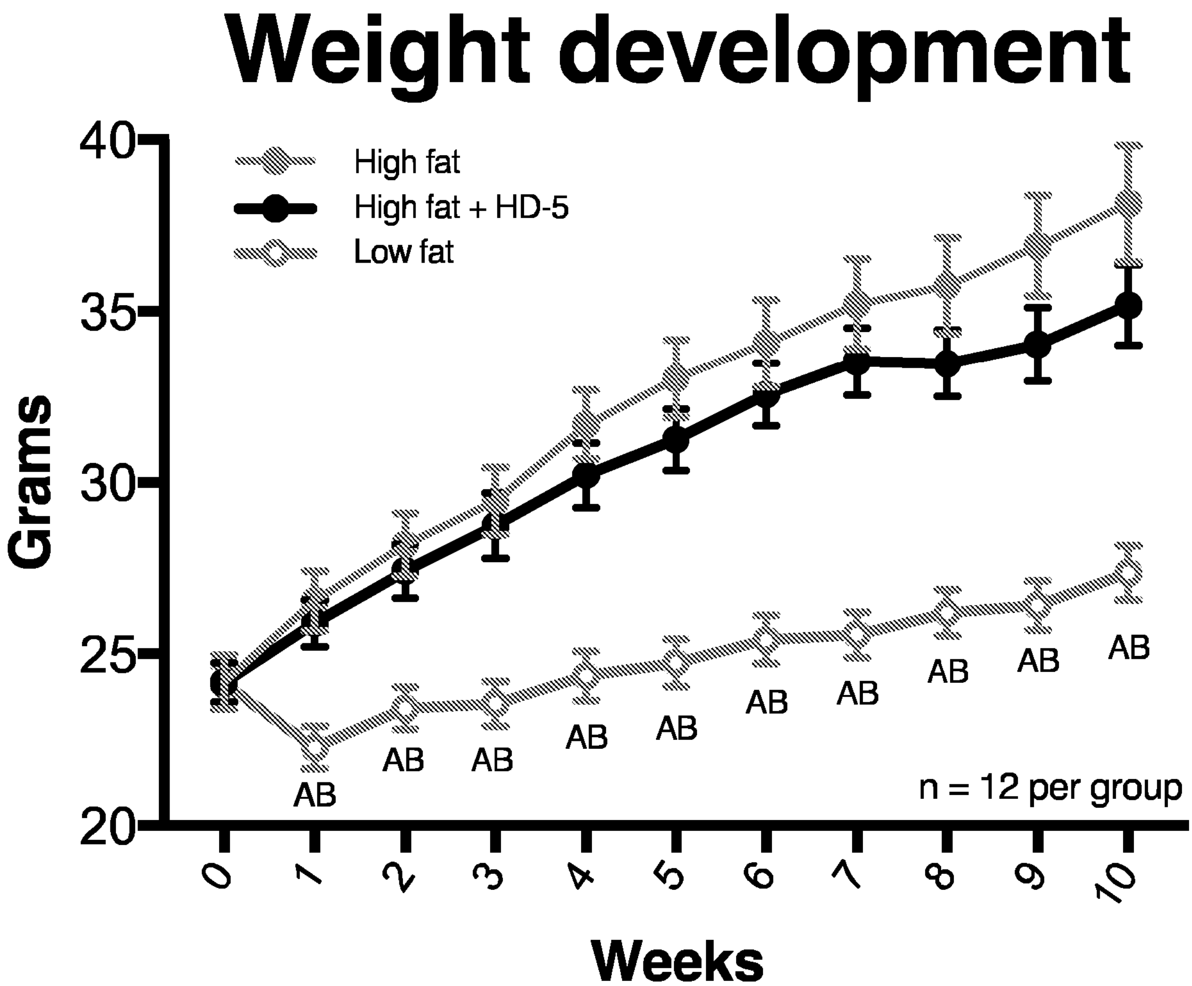
Weight development
High fat
High fat + HD-5
Low fat
40
35
30
25
20
Grams
AB
AB
AB
AB
AB
AB
AB
AB
AB
AB
n = 12 per group
0
1
2
3
4
5
6
7
8
9
10
Weeks

Fig. 17B

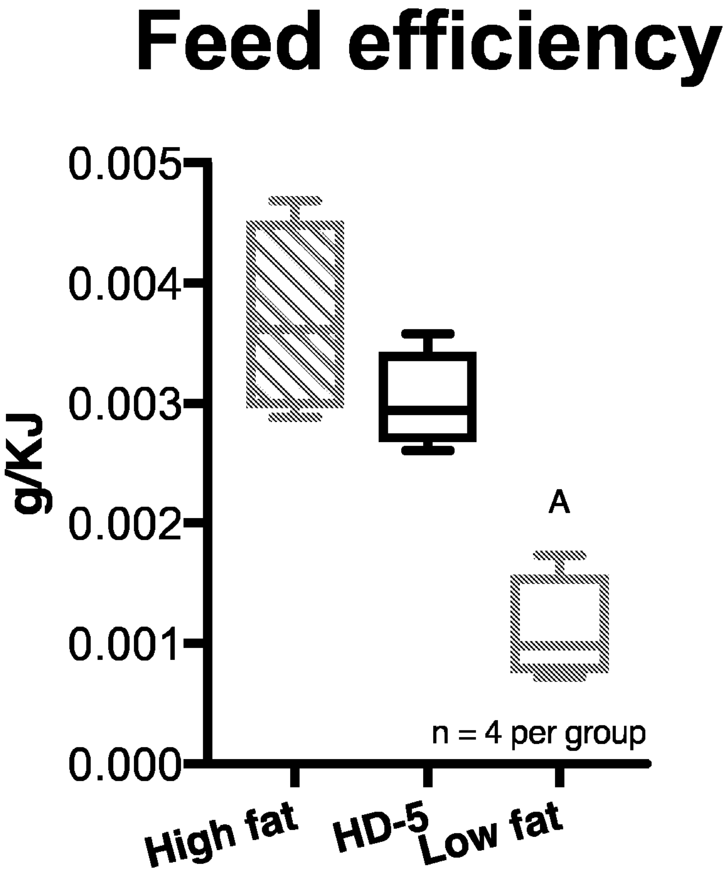


Fig. 17C

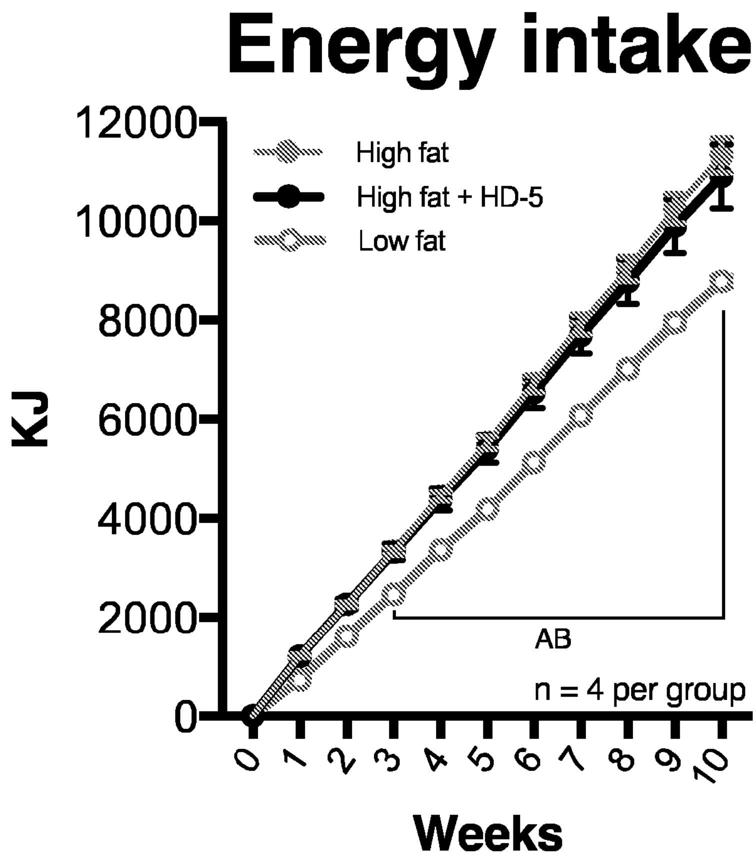

Fig. 18A

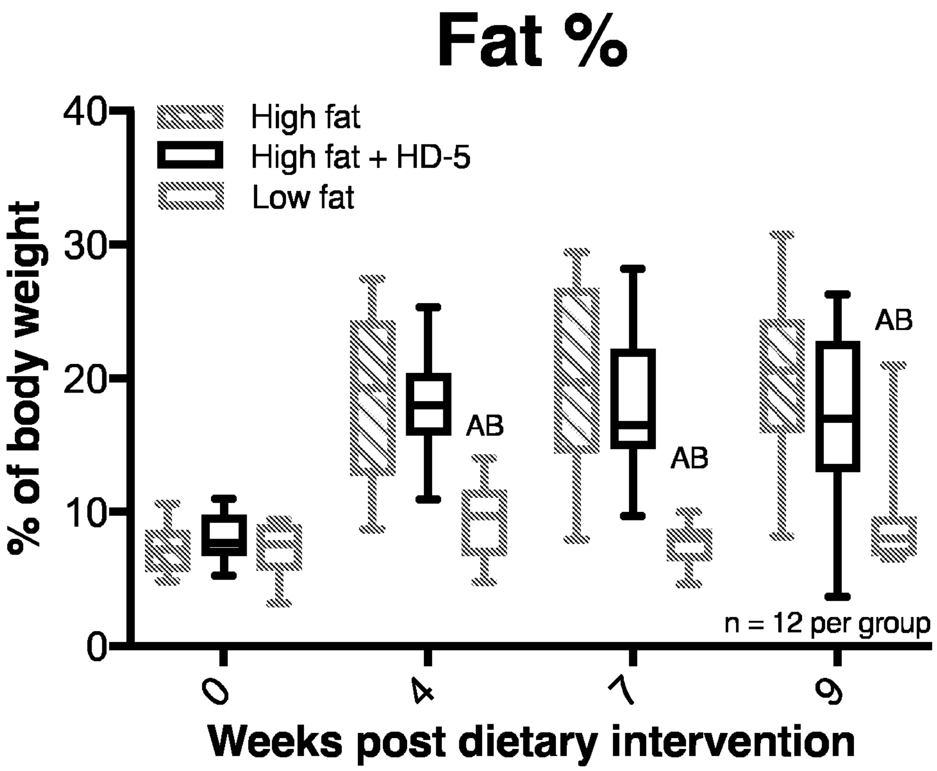


Fig. 18B

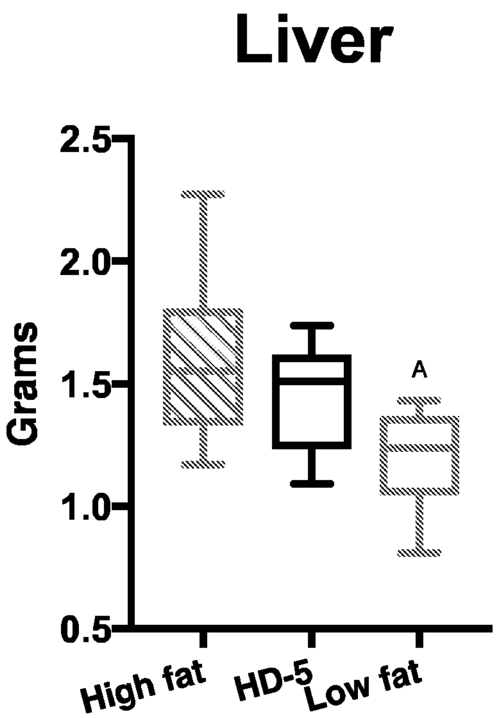


Fig. 18C

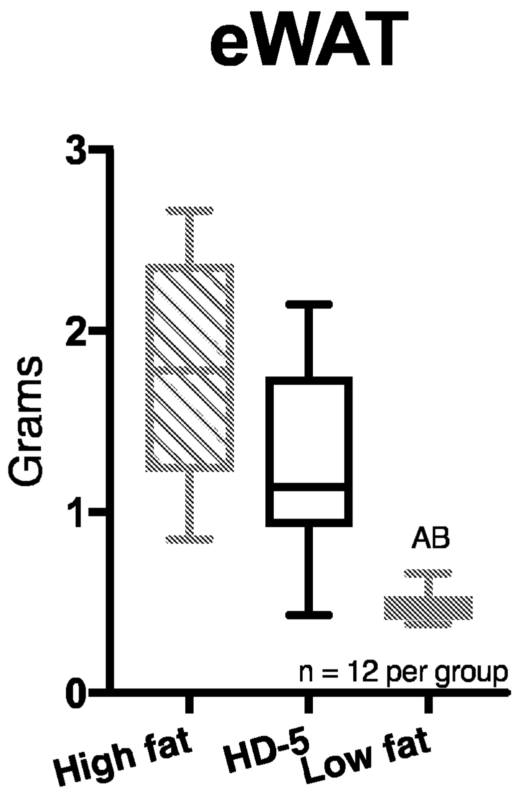

Fig. 19A

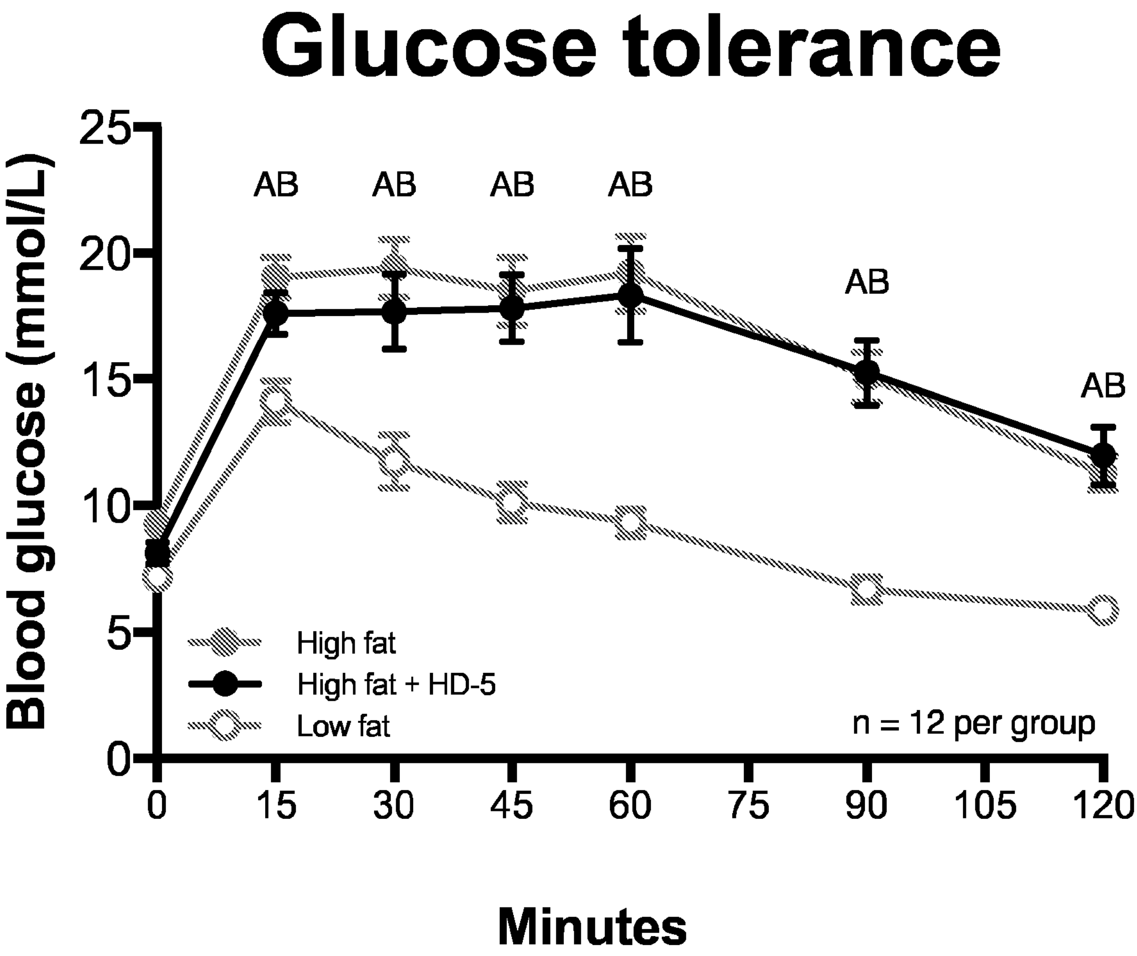
Glucose tolerance
Blood glucose (mmol/L)
25
20
15
10
5
0
AB
AB
AB
AB
AB
AB
High fat
High fat + HD-5
Low fat
n = 12 per group
0
15
30
45
60
75
90
105
120
Minutes

Fig 19B

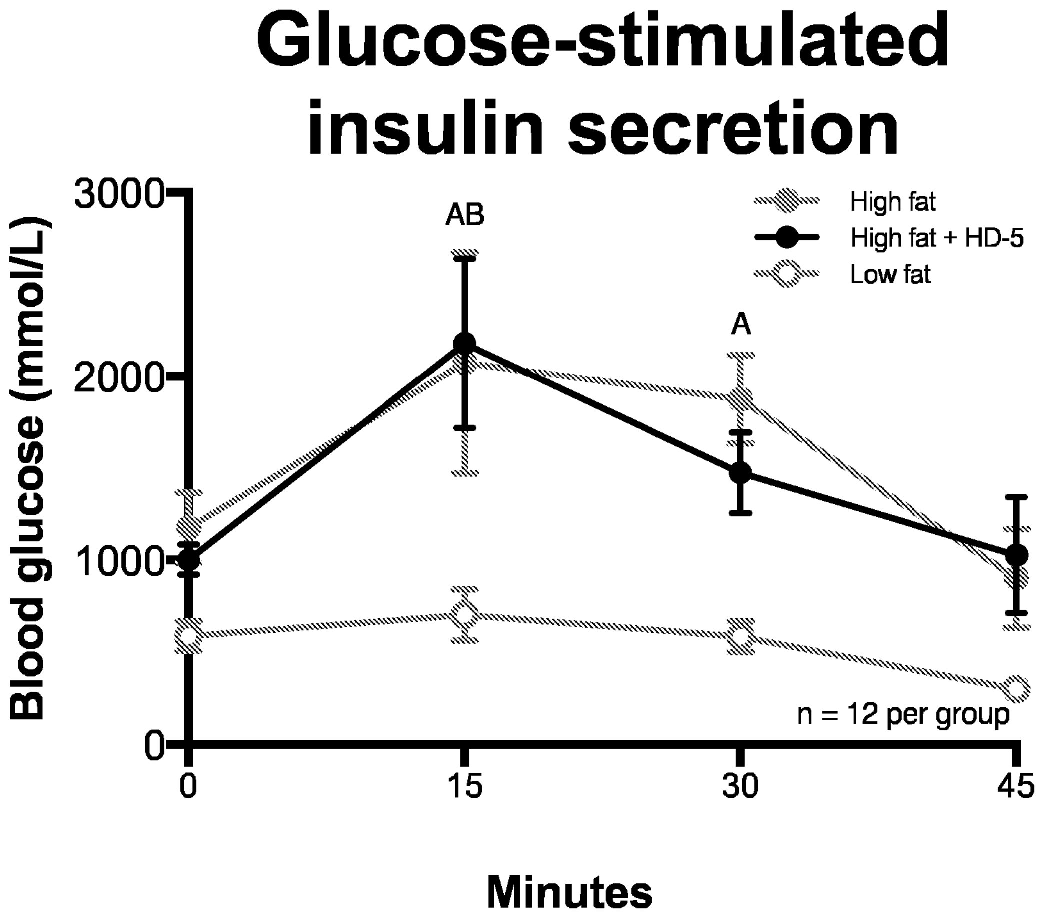
Glucose-stimulated insulin secretion
Blood glucose (mmol/L)
3000
2000
1000
0
AB
A
High fat
High fat + HD-5
Low fat
n = 12 per group
0
15
30
45
Minutes

Fig. 20A

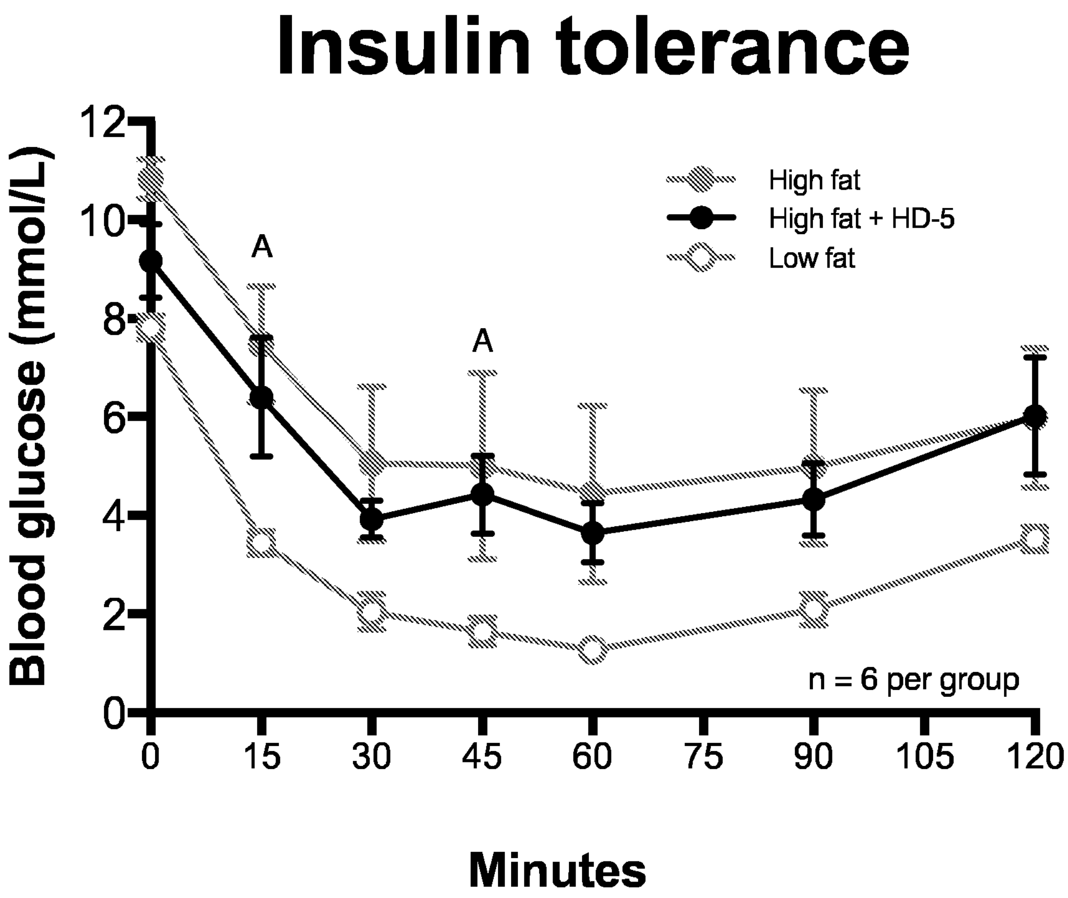


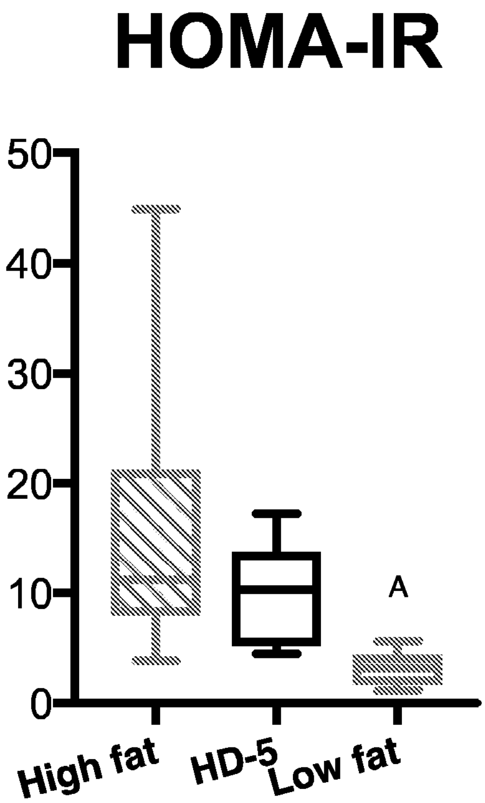


Fig. 20B

Fig. 21A

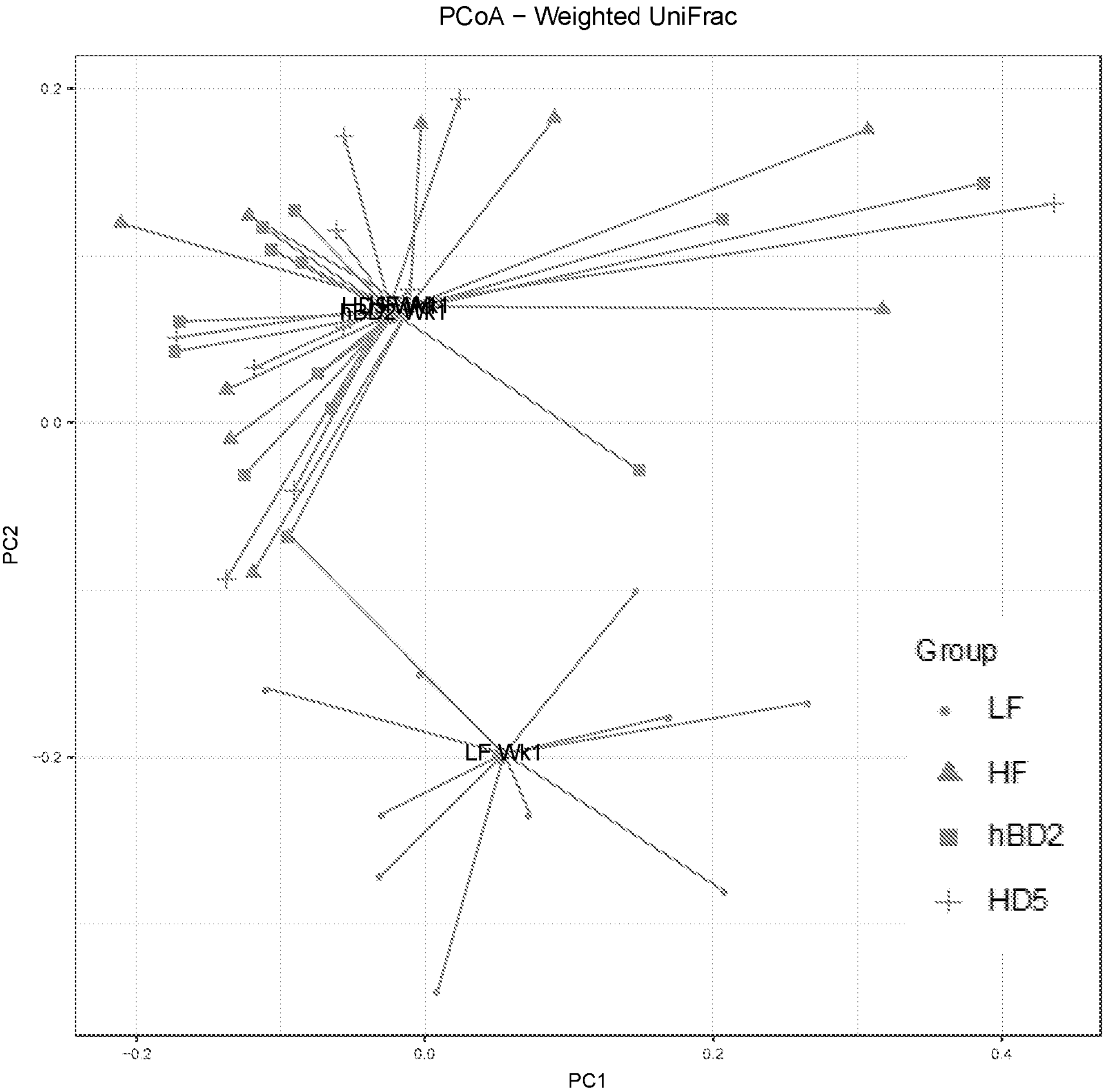
PCoA − Weighted UniFrac
0.2
0.0
-0.2
PC2
-0.2
0.0
0.2
0.4
PC1
hBD2 Wk1
LF Wk1
Group
LF
HF
hBD2
HD5

Fig. 21B:

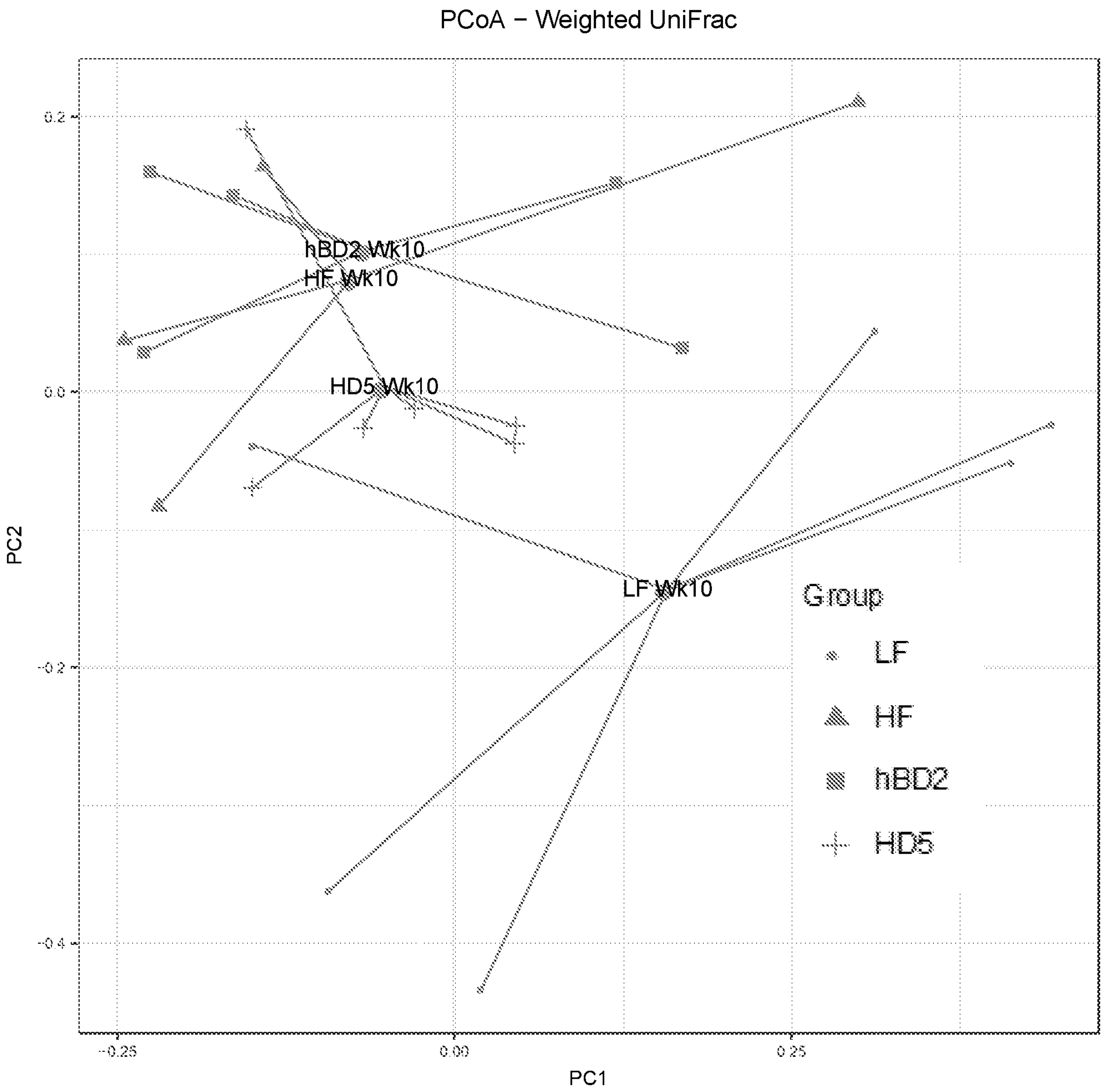
PCoA − Weighted UniFrac
0.2
0.0
-0.2
-0.4
PC2
-0.25
0.00
0.25
PC1
hBD2 Wk10
HF Wk10
HD5 Wk10
LF Wk10
Group
LF
HF
hBD2
HD5

Fig. 21C:

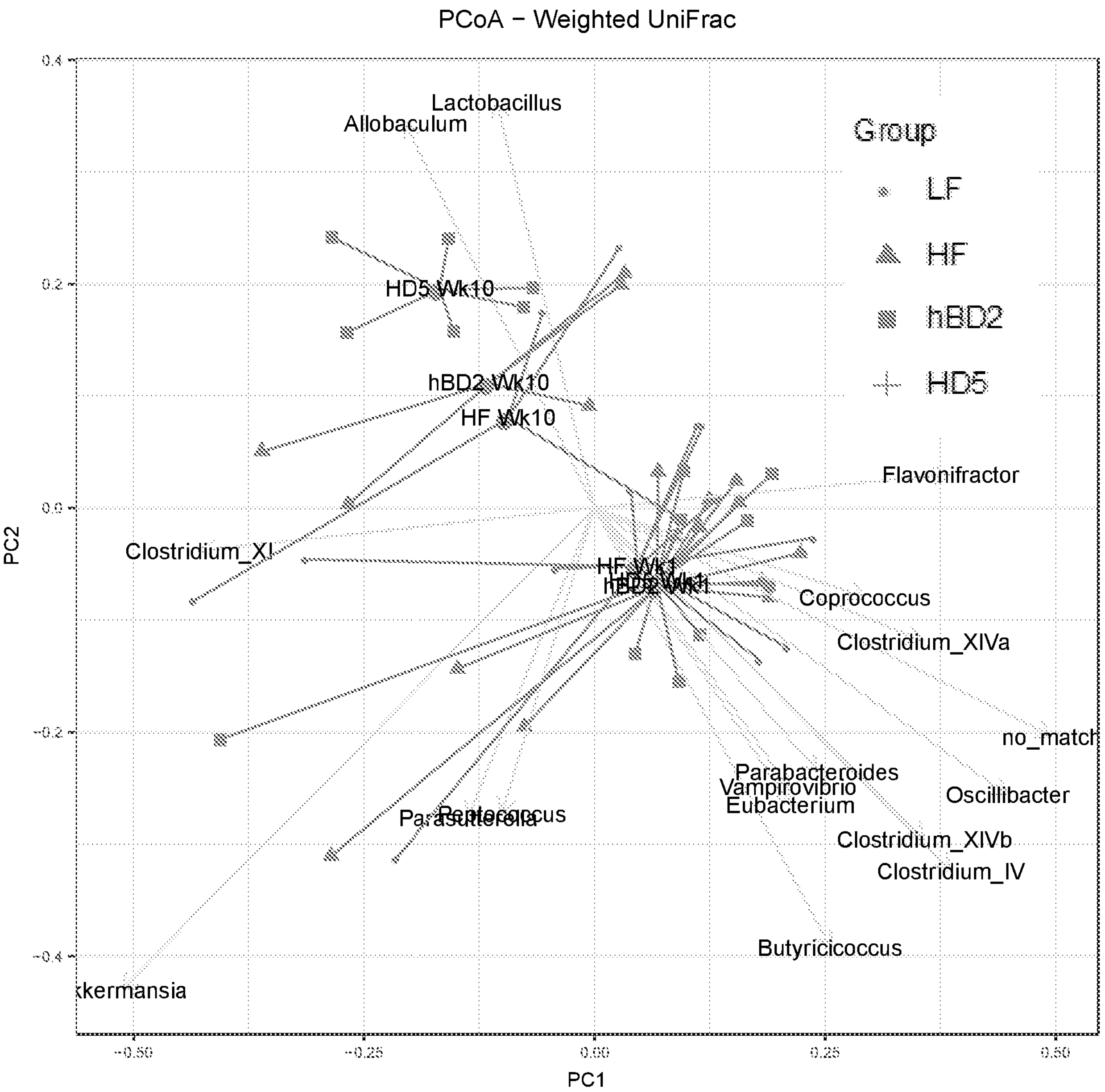
PCoA − Weighted UniFrac
Group
LF
HF
hBD2
HD5
Lactobacillus
Allobaculum
HD5 Wk10
hBD2 Wk10
HF Wk10
Flavonifractor
Clostridium_XI
HF Wk1
Coprococcus
Clostridium_XIVa
no_match
Parabacteroides
Vampirovibrio
Eubacterium
Oscillibacter
Clostridium_XIVb
Clostridium_IV
Butyricicoccus
kkermansia
PC2
PC1
0.4
0.2
0.0
−0.2
−0.4
−0.50
−0.25
0.00
0.25
0.50

Fig. 22A

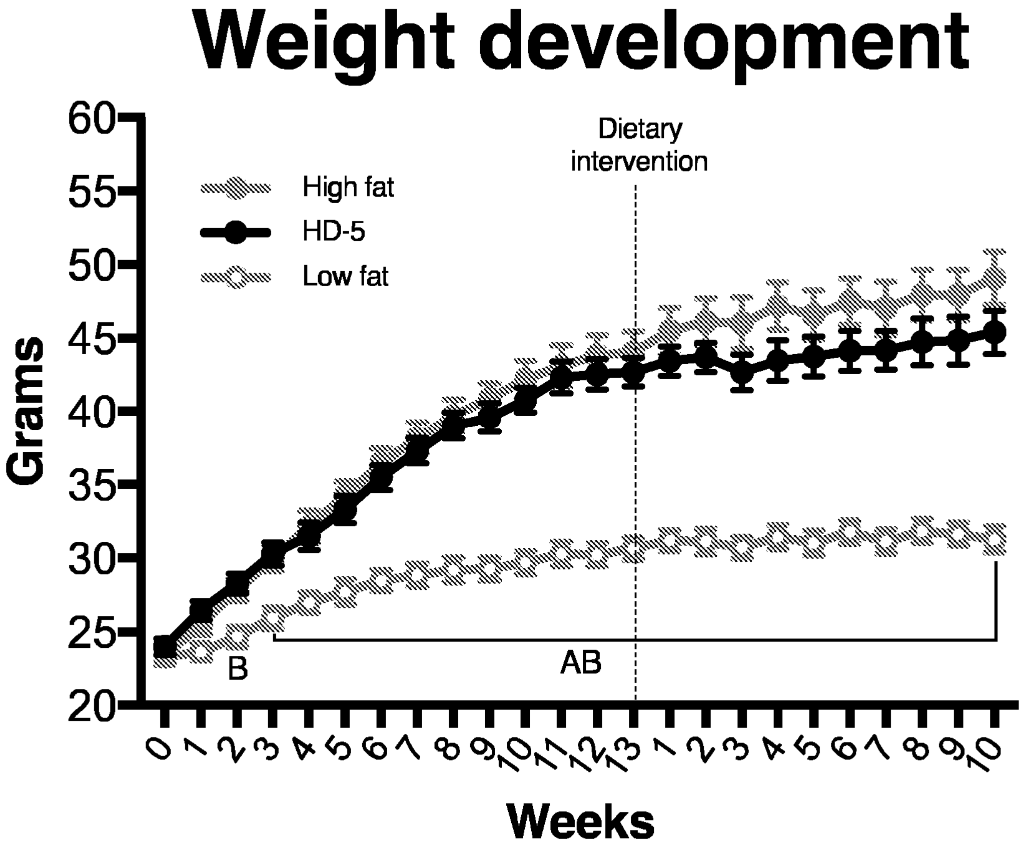


Fig. 22B

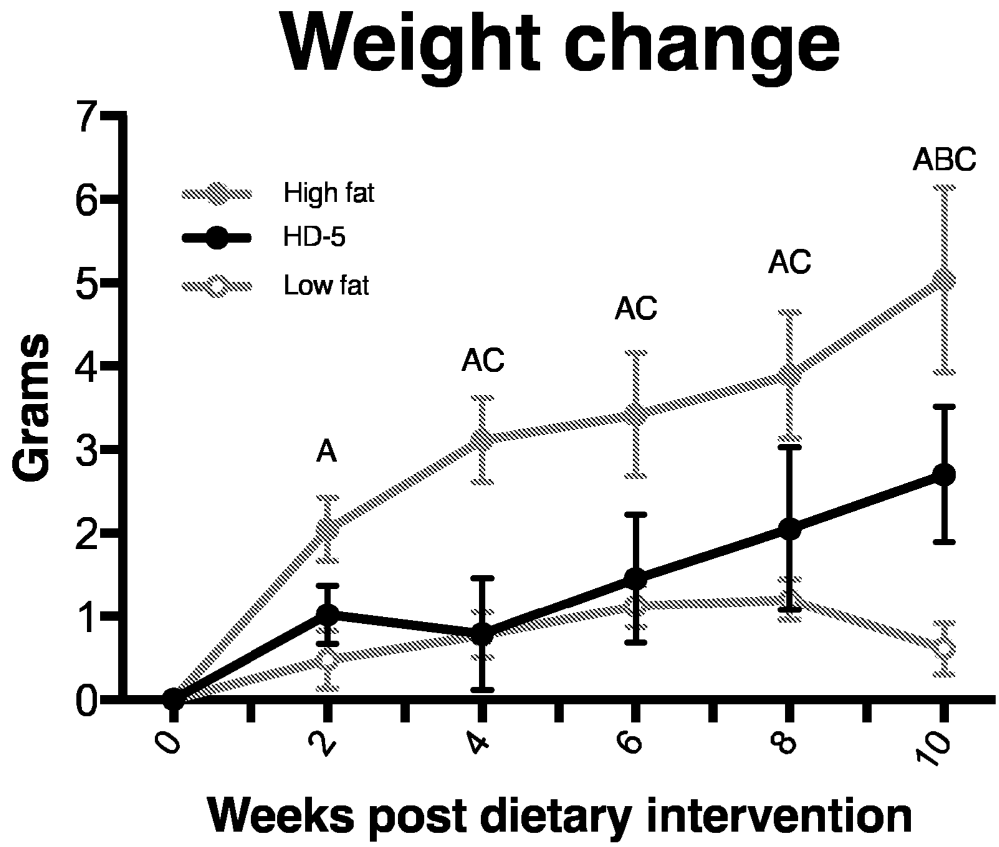

Fig. 23A

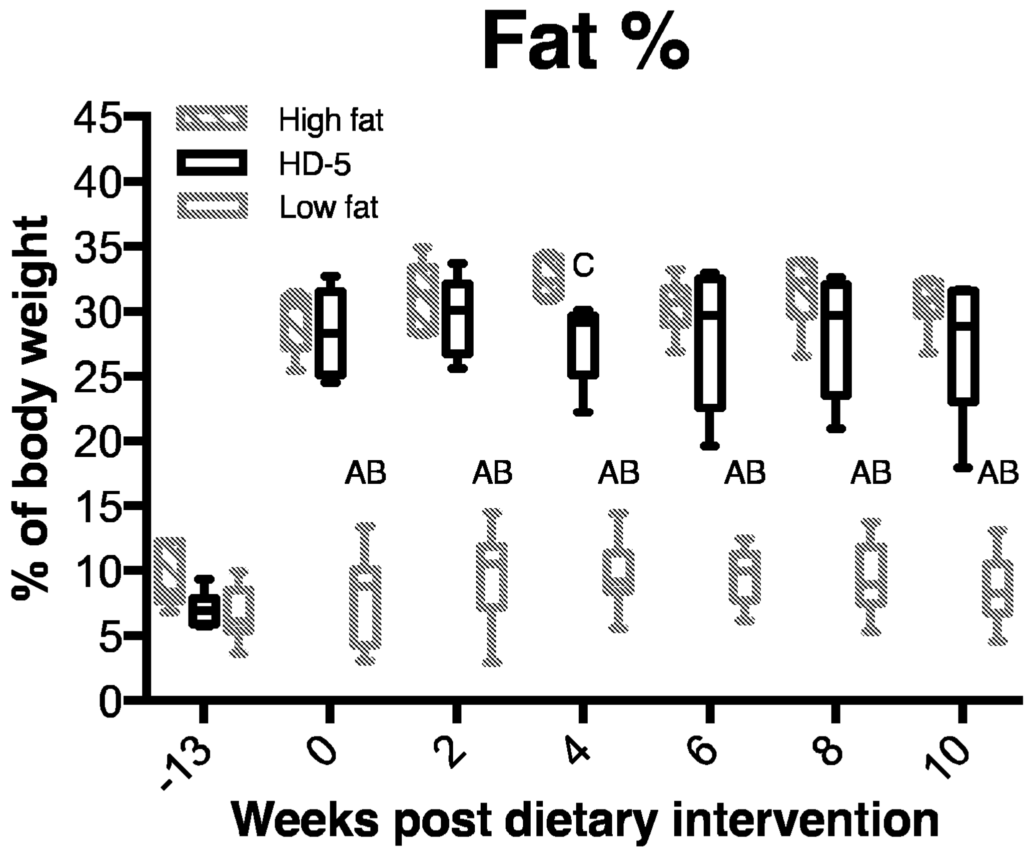


Fig. 23B

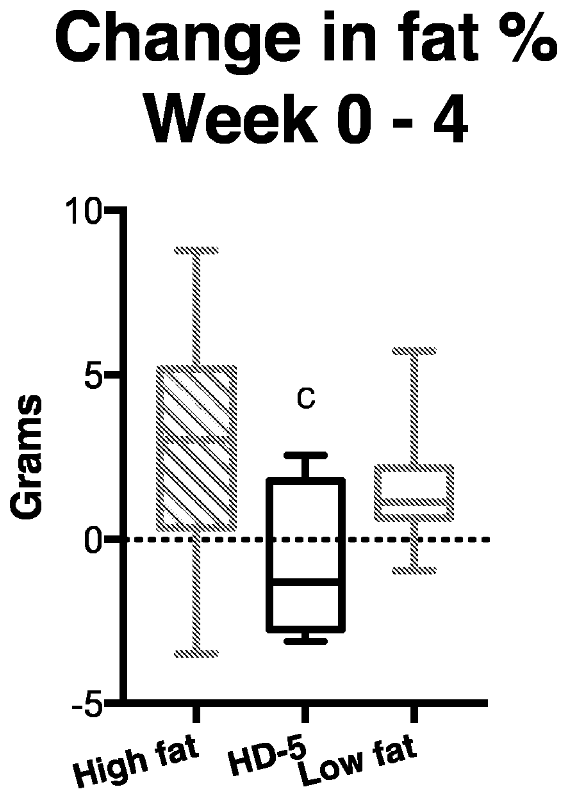

Fig. 24A

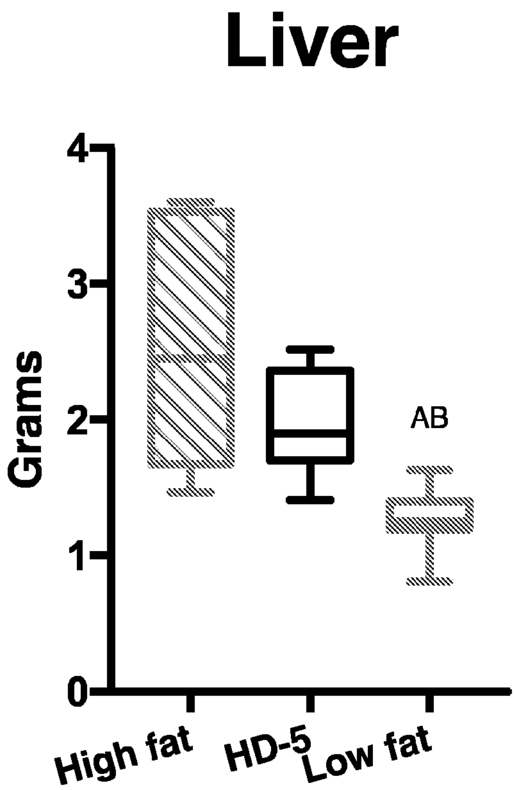


Fig. 24B

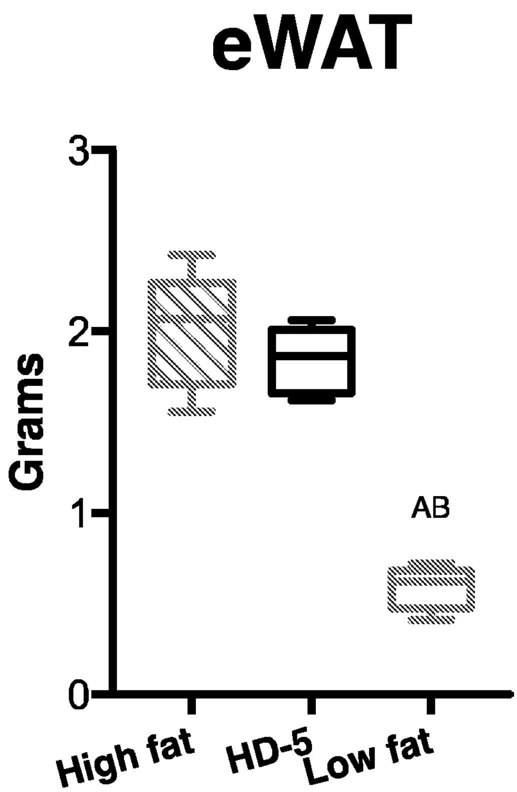

Fig. 25A

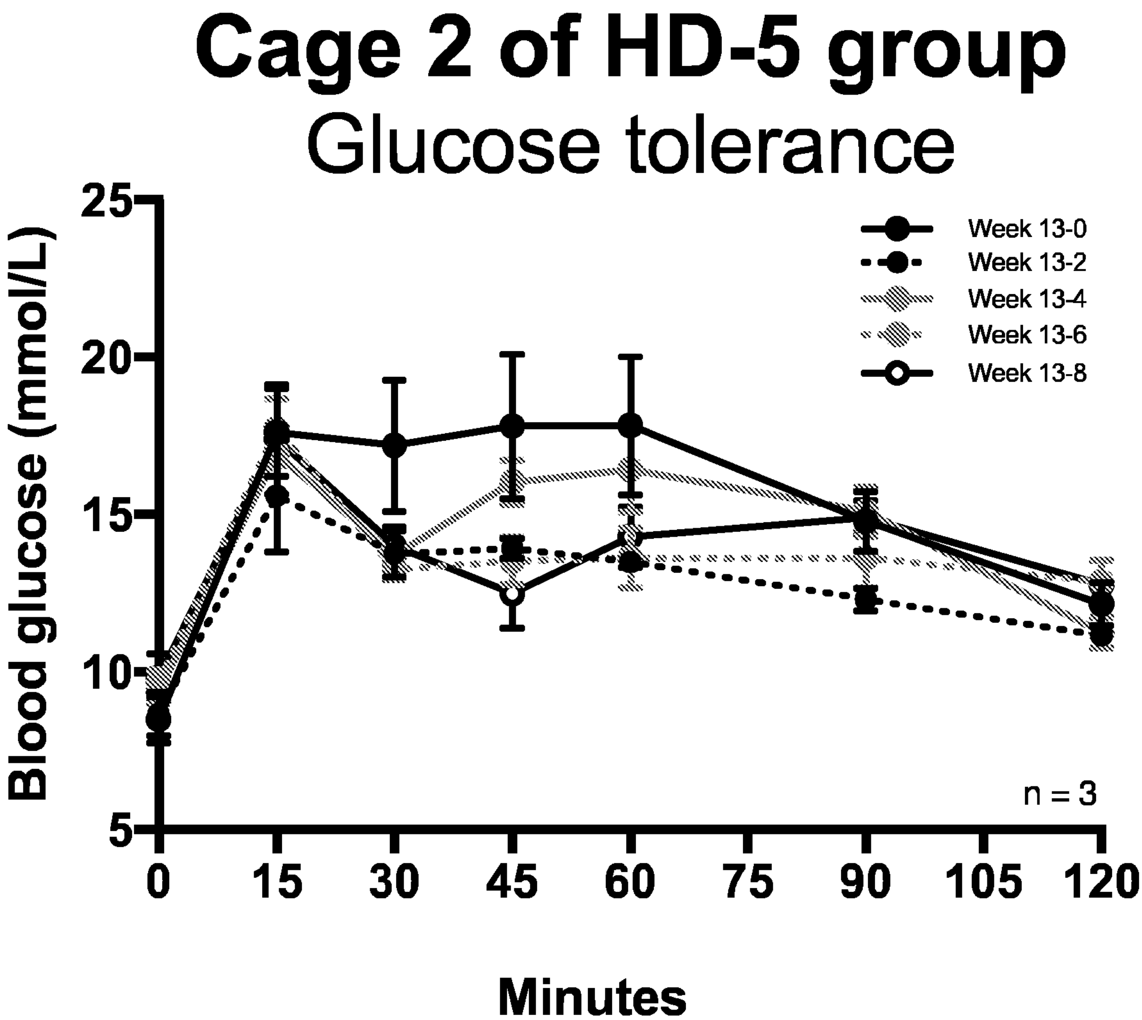
Cage 2 of HD-5 group
Glucose tolerance
Blood glucose (mmol/L)
25
20
15
10
5
Week 13-0
Week 13-2
Week 13-4
Week 13-6
Week 13-8
n = 3
0
15
30
45
60
75
90
105
120
Minutes

Fig. 25B

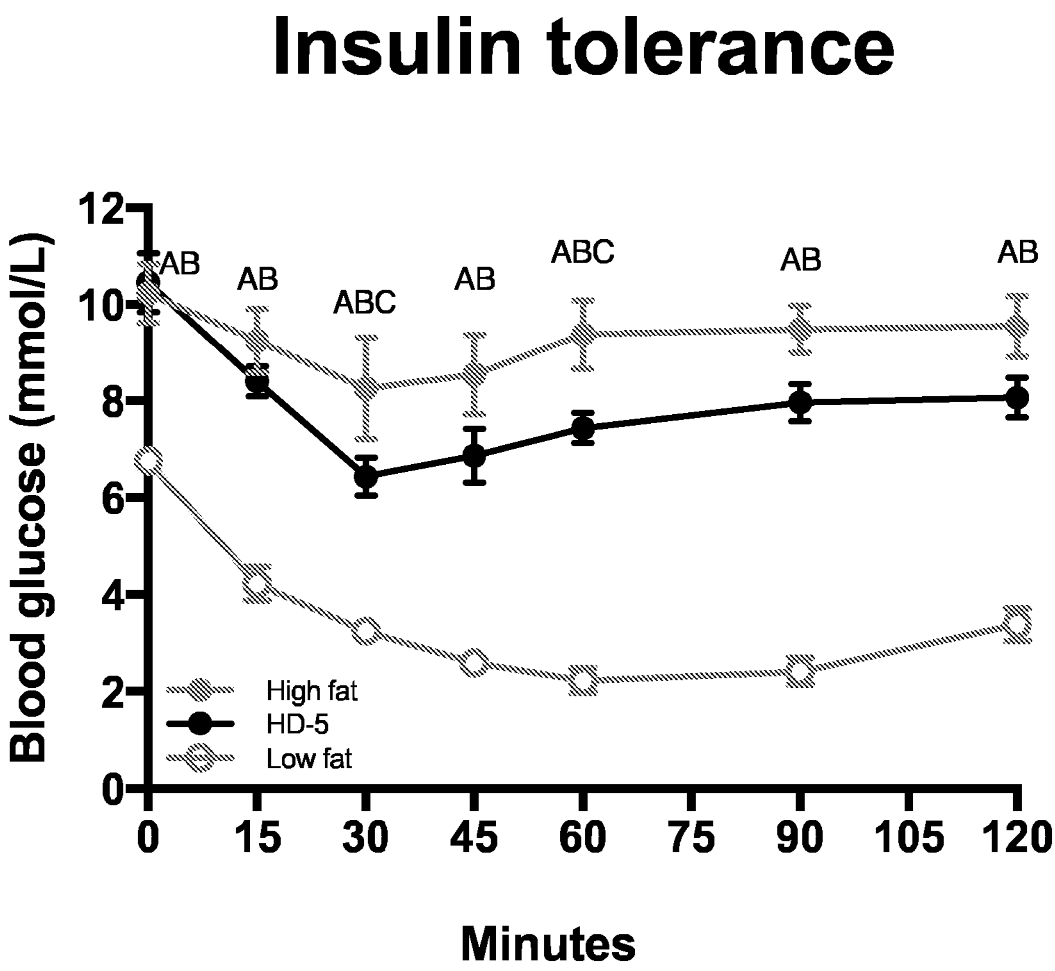
Insulin tolerance
Blood glucose (mmol/L)
12
10
8
6
4
2
0
AB
AB
ABC
AB
ABC
AB
AB
High fat
HD-5
Low fat
0
15
30
45
60
75
90
105
120
Minutes

Fig. 26.

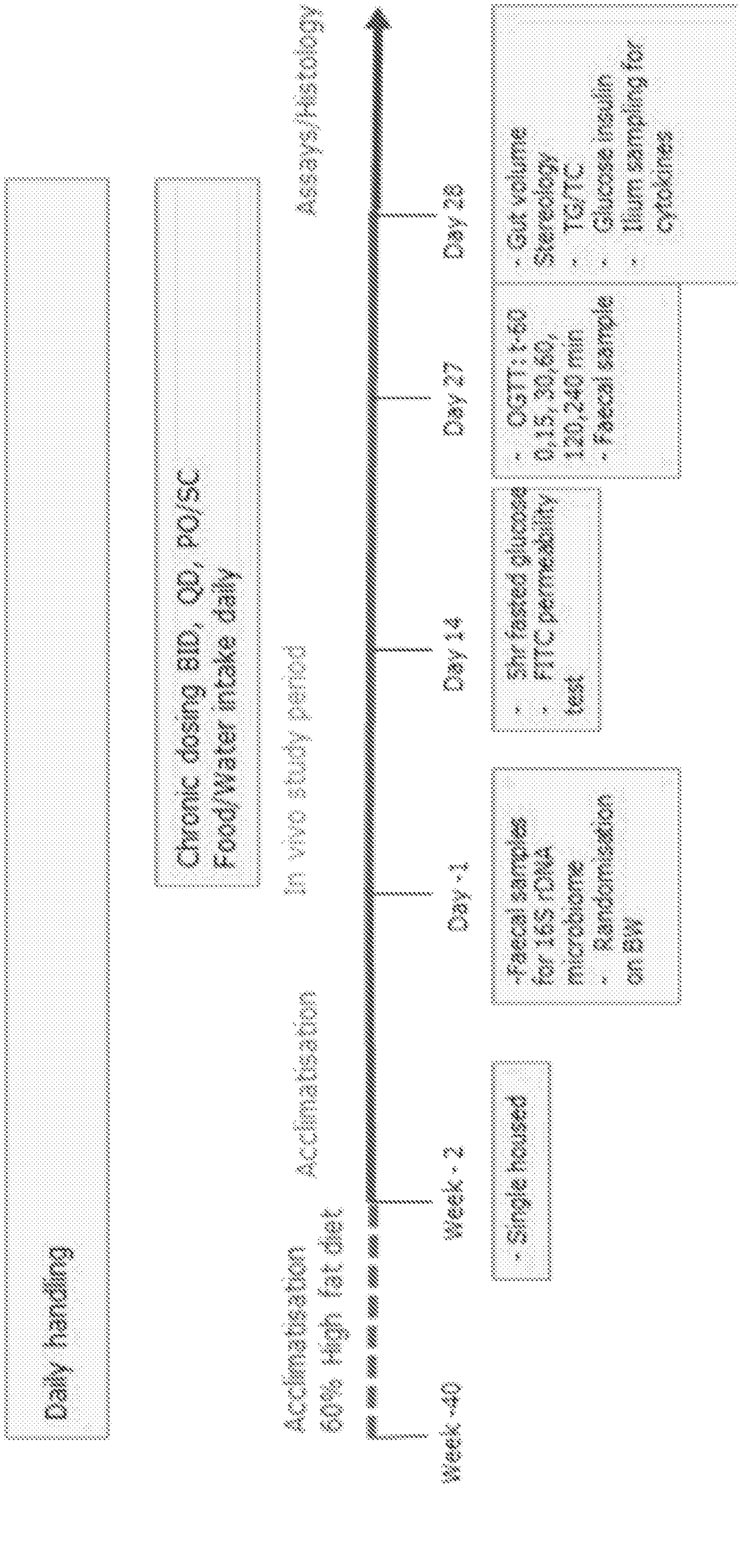
Daily handling
Chronic dosing BID, QD, PO/SC
Food/Water intake daily
Acclimatisation
60% High fat diet
Acclimatisation
In vivo study period
Assays/Histology
Week -40
Week - 2
Day -1
Day 14
Day 27
Day 28
- Single housed
-Faecal samples for 16S rDNA microbiome
- Randomisation on BW
- 5hr fasted glucose
- FITC permeability test
- OGTT: t-60 0,15, 30,60, 120,240 min
- Faecal sample
- Gut volume
- Stereology
- TG/TC
- Glucose insulin
- Ilium sampling for cytokines

Fig. 27A

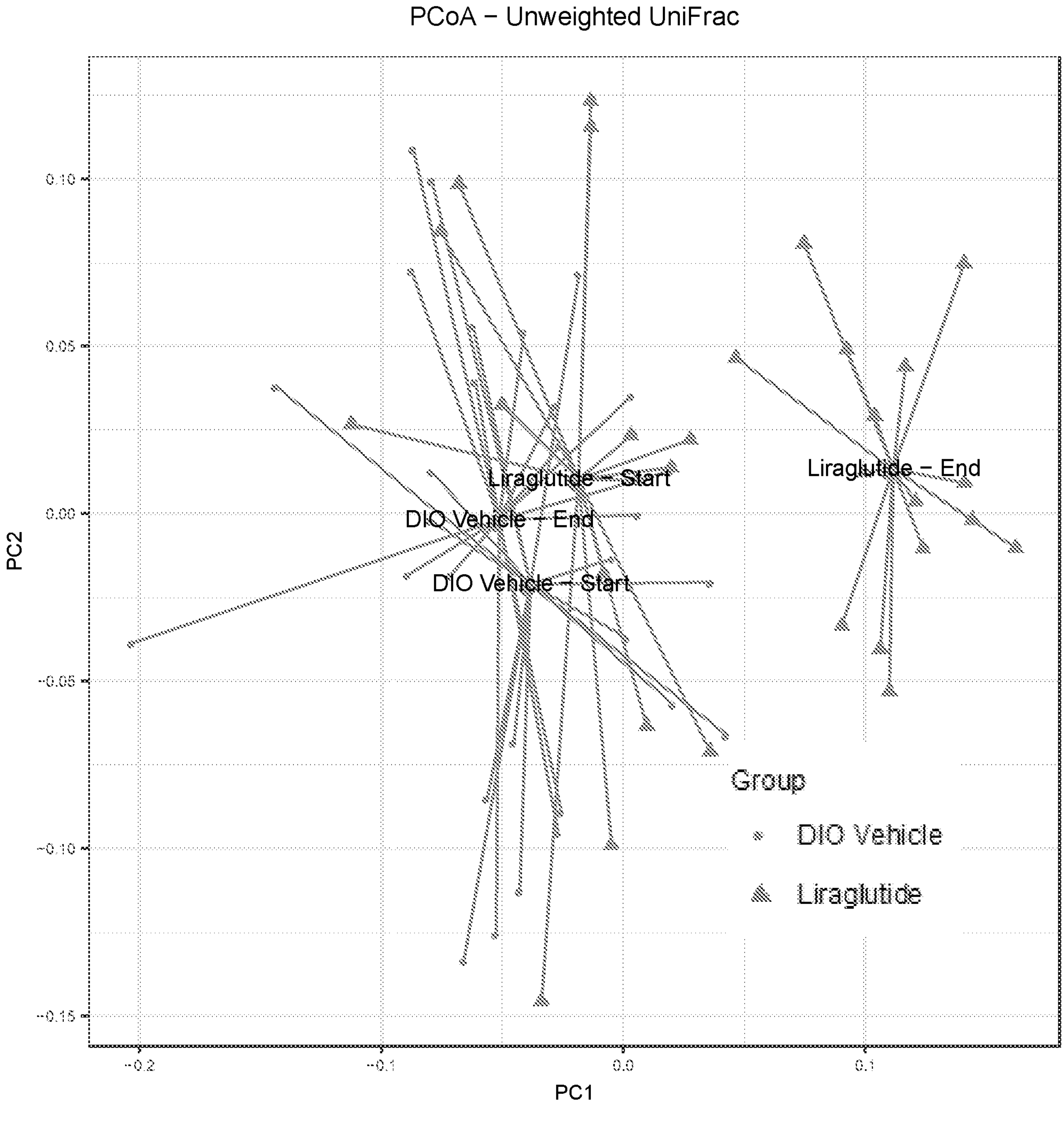
PCoA − Unweighted UniFrac
PC2
PC1
0.10
0.05
0.00
−0.05
−0.10
−0.15
−0.2
−0.1
0.0
0.1
Liraglutide − Start
Liraglutide − End
DIO Vehicle − End
DIO Vehicle − Start
Group
DIO Vehicle
Liraglutide

Fig. 27B

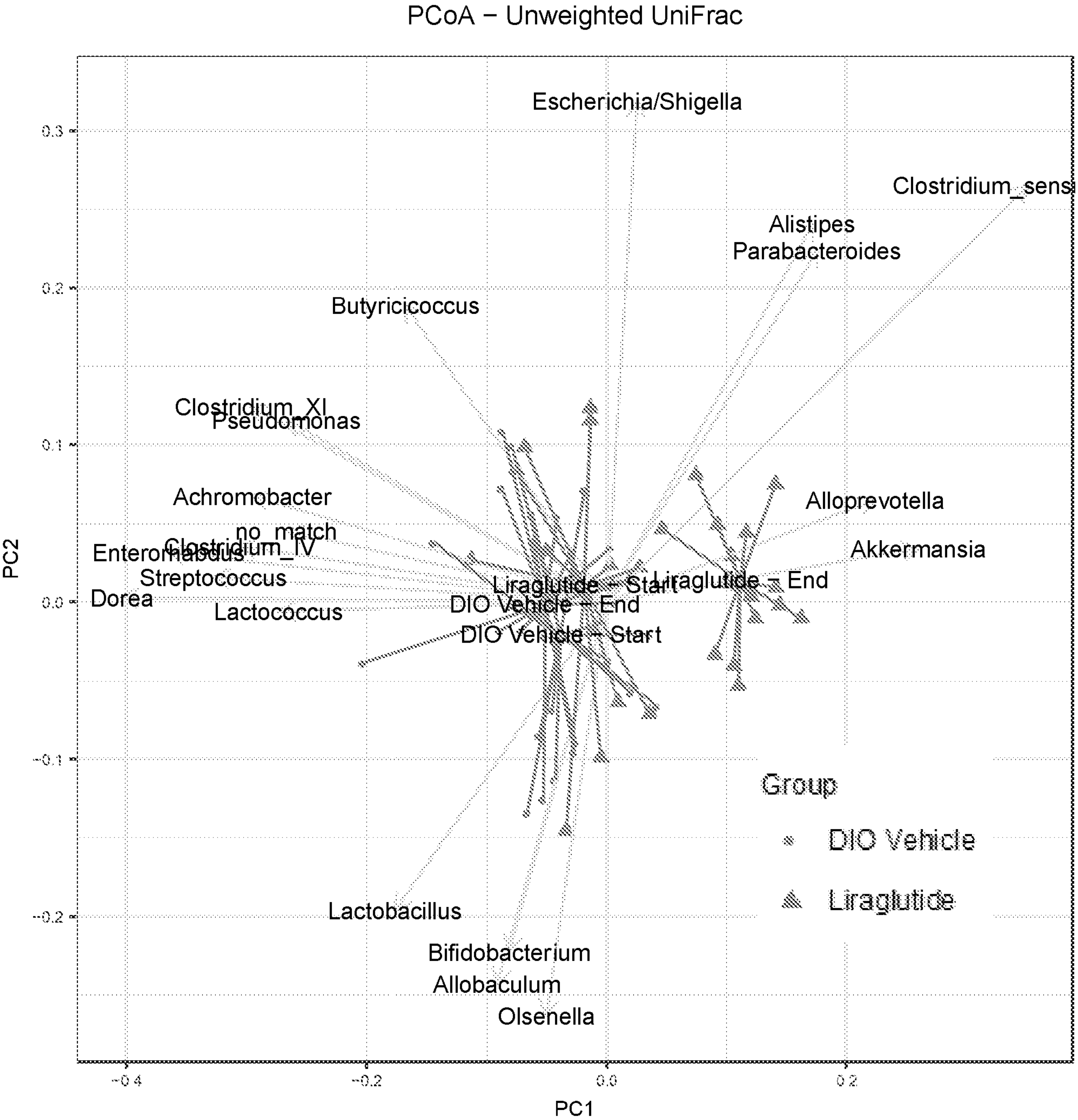
PCoA − Unweighted UniFrac
Escherichia/Shigella
Clostridium_sensu
Alistipes
Parabacteroides
Butyricicoccus
Clostridium_XI
Pseudomonas
Achromobacter
no_match
Enterorhabdus
Clostridium_IV
Streptococcus
Dorea
Lactococcus
Alloprevotella
Akkermansia
Liraglutide − Start
Liraglutide − End
DIO Vehicle − End
DIO Vehicle − Start
Lactobacillus
Bifidobacterium
Allobaculum
Olsenella
Group
DIO Vehicle
Liraglutide
PC1
PC2
0.3
0.2
0.1
0.0
−0.1
−0.2
−0.4
−0.2
0.0
0.2

Fig. 28

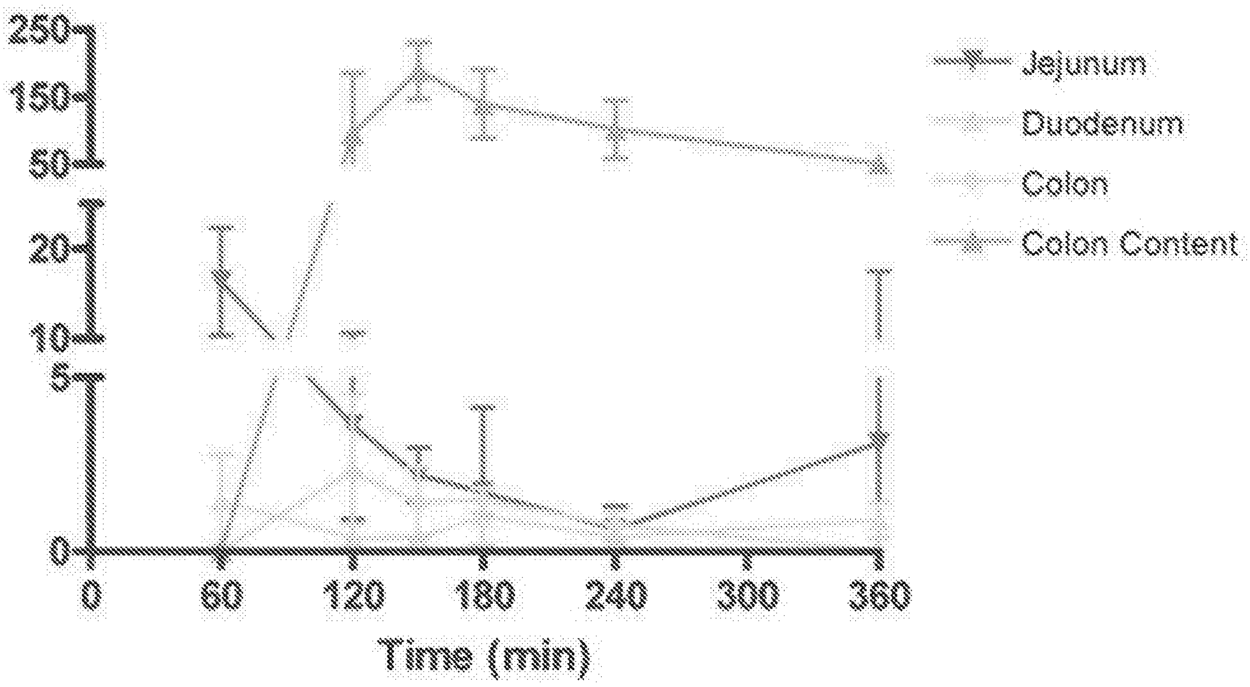
250
150
50
20
10
5
0
0
60
120
180
240
300
360
Time (min)
Jejunum
Duodenum
Colon
Colon Content

Fig. 29

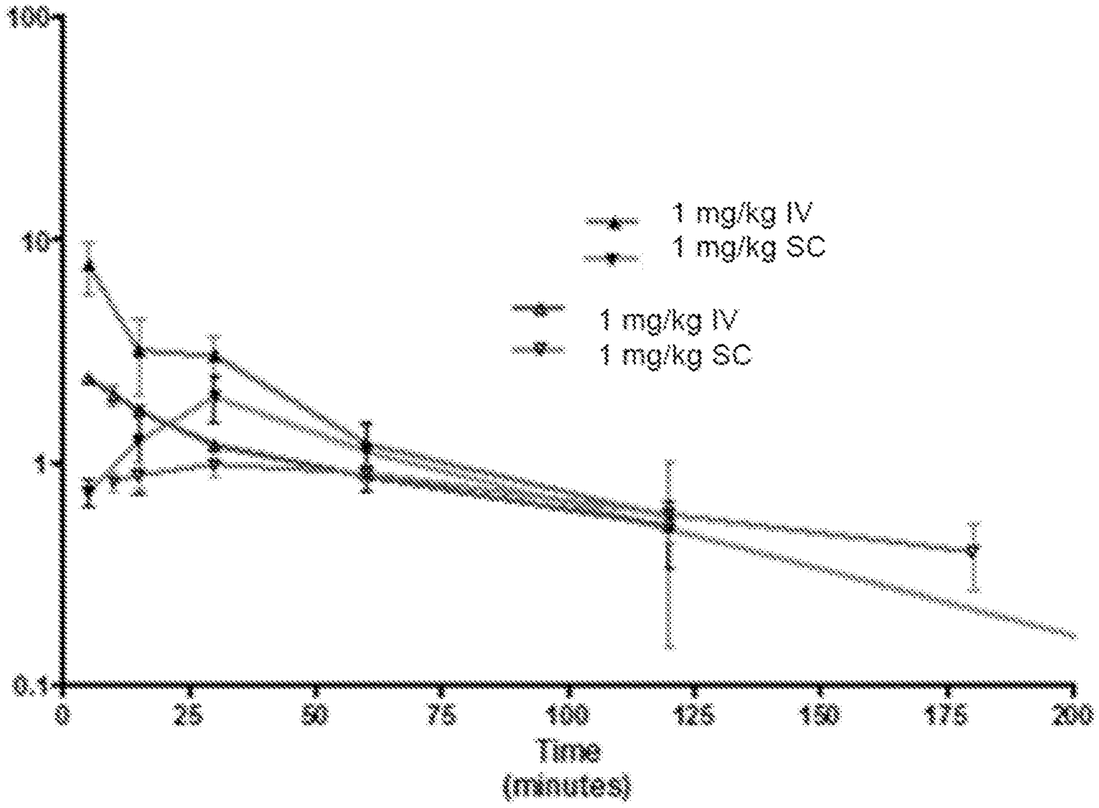
100
10
1
0.1
0
25
50
75
100
125
150
175
200
Time
(minutes)
1 mg/kg IV
1 mg/kg SC
1 mg/kg IV
1 mg/kg SC

Fig. 30

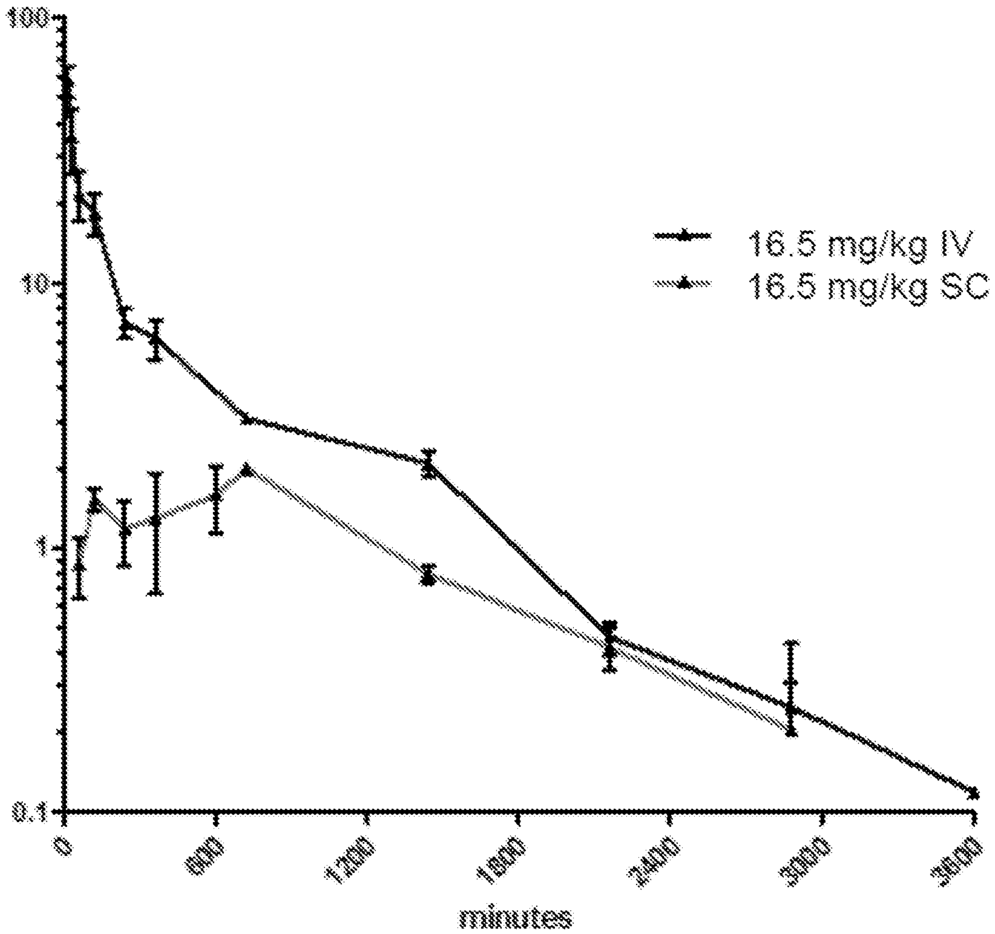
100
10
1
0.1
0
600
1200
1800
2400
3000
3600
minutes
16.5 mg/kg IV
16.5 mg/kg SC

Fig. 31

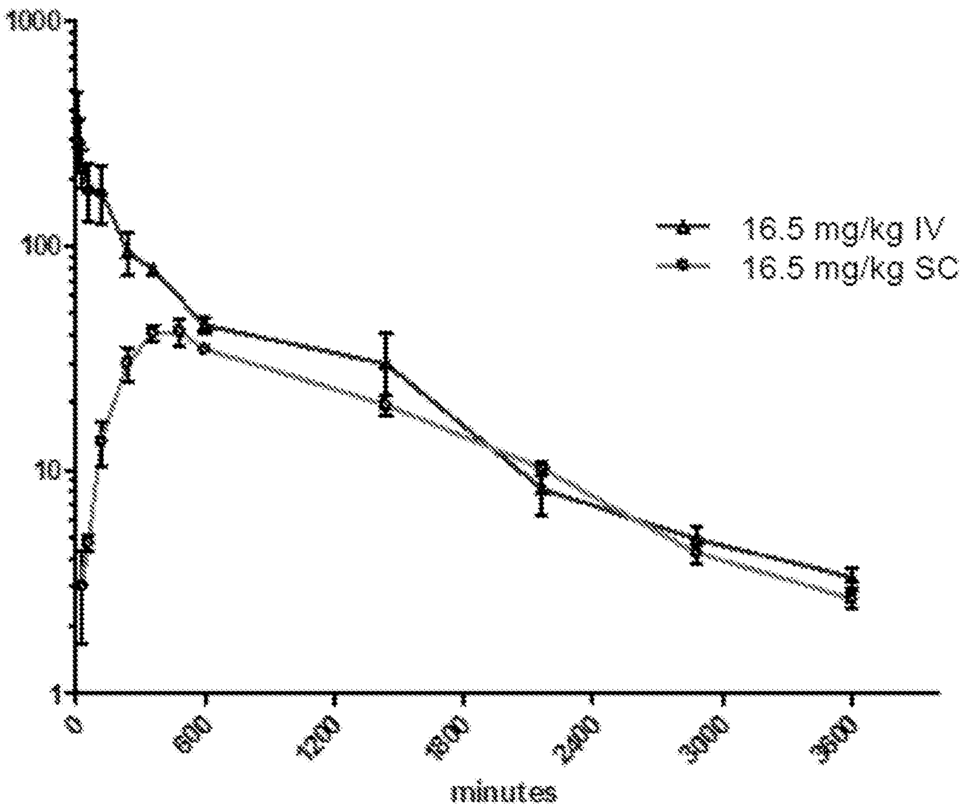
1000
100
10
1
0
600
1200
1800
2400
3000
3600
minutes
16.5 mg/kg IV
16.5 mg/kg SC

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008115390 A
- WO 2007133373 A
- CN 104971343
- WO 2010007165 A
- WO 2013026794 A
- WO 9517413 A
- WO 9522625 A
- US 5223409 A
- WO 9206204 A
- WO 2009095479 A
- WO 2010092135 A
- WO 2010007166 A
- DK PA201670041
- DK PA201670483


### Non-patent literature cited in the description


- **TANG et al.** *Animal Science Journal,* 2015, vol. 87 (10), 1258-1266
- **NEEDLEMAN ; WUNSCH.** *J. Mol. Biol.,* 1970, vol. 48, 443-453
- **CUNNINGHAM ; WELLS.** *Science,* 1989, vol. 244, 1081-1085
- **HILTON et al.** *J. Biol. Chem.,* 1996, vol. 271, 4699-4708
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochem.,* 1991, vol. 30, 10832-10837
- **DERBYSHIRE et al.** *Gene,* 1986, vol. 46, 145
- **NER et al.** *DNA,* 1988, vol. 7, 127
- **PAIGE.** *Pharmaceutical Research,* 1995, vol. 12 (12
- **SHECHTER et al.** *Bioconjugate Chem.,* 2005, vol. 16, 913-920
- *International Journal of Peptide Research and Therapeutics,* June 2007, vol. 13 (1-2
- **GENNARO.** Remington's Pharmaceutical Sciences. 1990
- **AJSLEV TA et al.** Trends in parent-child correlations of childhood body mass index during the development of the obesity epidemic. *PLoS One,* 2014, vol. 9, 10
- **ANGELAKIS E ; RAOULT D.** The increase of Lactobacillus species in the gut flora of newborn broiler chicks and ducks is associated with weight gain. *PLoS One,* 2010, vol. 5, 5
- **ANGELAKIS E. et al.** An evaluation of the effects of Lactobacillus ingluviei on body weight, the intestinal microbiome and metabolism in mice. *Microb Pathog,* 2012, vol. 52 (1), 61-8
- **ARMOGIDA SA et al.** Identification and quantification of innate immune system mediators in human breast milk. *Allergy Asthma Proc.,* 2004, vol. 25 (5), 297-304
- **BÄCKHED F et al.** Mechanisms underlying the resistance to diet-induced obesity in germ-free mice. *Proc Natl Acad Sci USA,* 2007, vol. 104 (3), 979-84
- **BELKAID W ; HAND TW.** Role of the microbiota in immunity and inflammation. *Cell,* 2014, vol. 157 (1), 121-41
- **BOWIE JU ; SAUER RT.** Identifying determinants of folding and activity for a protein of unknown structure. *Proc. Natl. Acad. Sci. USA,* 1989, vol. 86, 2152-2156
- **CHASSAING B et al.** Dietary emulsifiers impact the mouse gut microbiota promoting colitis and metabolic syndrome. *Nature,* 2015, vol. 519 (7541), 92-6
- **CUNNINGHAM BC ; WELLS JA.** High-resolution epitope mapping of hGH-receptor interactions by alanine-scanning mutagenesis. *Science,* 1989, vol. 244, 1081-1085
- **DERBYSHIRE KM ; SALVO JJ ; GRINDLEY ND.** A simple and efficient procedure for saturation mutagenesis using mixed oligodeoxynucleotides. *Gene,* 1986, vol. 46, 145-152
- **EVERARD A ; CANI PD.** Diabetes, obesity and gut microbiota. *Best Pract Res Clin Gastroenterol,* 2013, vol. 27 (1), 73-83
- **FAULDS MH ; DAHLMAN-WRIGHT K.** Metabolic diseases and cancer risk. *Curr Opin Oncol.,* 2012, vol. 24 (1), 58-61
- **FAVRE-GODAL Q et al.** Comprehensive approach for the detection of antifungal compounds using a susceptible strain of Candida albicans and confirmation of in vivo activity with the Galleria mellonella model. *Phytochemistry,* 2014, vol. 105, 68-78

- **FENG Q et al.** Gut microbiome development along the colorectal adenoma-carcinoma sequence. *Nat Commun,* 2015, vol. 6, 6528
- **GIANNOULI M et al.** Use of larvae of the wax moth Galleria mellonella as an in vivo model to study the virulence of Helicobacter pylori. *BMC Microbiol,* 2014, vol. 14, 228
- **HILTON DJ et al.** Saturation mutagenesis of the WSXWS motif of the erythropoietin receptor. *J. Biol. Chem.,* 1996, vol. 271, 4699-4708
- **KHAN M et al.** Growth-promoting effects of single-dose intragastrically administered probiotics in chickens. *Br Poult Sci,* 2007, vol. 48 (6), 732-5
- **KOREN O et al.** Host remodeling of the gut microbiome and metabolic changes during pregnancy. *Cell,* 2012, vol. 150 (3), 470-80
- **LE CHATELIER E et al.** Richness of human gut microbiome correlates with metabolic markers. *Nature,* 2013, vol. 500 (7464), 541-6
- **LEVITEN M.** The Finnish connection. *Biocentury Innovations,* 16 June 2016
- **LIU HY et al.** Suppression of hepatic glucose production by human neutrophil α-defensin through a signaling pathway distinct from insulin. *The Journal of Biological Chemistry,* 2008, vol. 283 (18), 12056-12063
- **LOWMAN HB ; BASS SH ; SIMPSON N ; WELLS JA.** Selecting high-affinity binding proteins by monovalent phage display. *Biochem,* 1991, vol. 30, 10832-10837
- **MOWAT AM ; AGACE WW.** Regional specialization within the intestinal immune system. *Nat Rev Immunol.,* 2014, vol. 14 (10), 667-85
- **NEEDLEMAN SB ; WUNSCH CD.** A general method applicable to the search for similarities in the amino acid sequence of two proteins. *J. Mol. Biol.,* 1970, vol. 48, 443-453
- **NER SS ; GOODIN DB ; SMITH M.** A simple and efficient procedure for generating random point mutations and for codon replacements using mixed oligodeoxynucleotides. *DNA,* 1988, vol. 7, 127-134
- **NEURATH H ; HILL RL.** The Proteins. Academic Press, 1979
- **QIN J et al.** A metagenome-wide association study of gut microbiota in type 2 diabetes. *Nature,* 2012, vol. 490 (7418), 55-60
- **REIDHAAR-OLSON JF ; SAUER RT.** Combinatorial cassette mutagenesis as a probe of the informational content of protein sequences. *Science,* 1988, vol. 241, 53-57
- **GENNARO AR.** Remington 's Pharmaceutical Sciences. 1990
- **RICE P ; LONGDEN I ; BLEASBY A.** EMBOSS: the European Molecular Biology Open Software Suite. *Trends in Genetics,* 2000, vol. 16, 276-277
- **RIDAURA VK et al.** Gut microbiota from twins discordant for obesity modulate metabolism in mice. *Science,* 2013, vol. 341 (6150), 1241214
- **SALZMAN NH ; UNDERWOOD MA ; BEVINS CL.** Paneth cells, defensins, and the commensal microbiota: a hypothesis on intimate interplay at the intestinal mucosa. *Semin Immunol,* 2007, vol. 19 (2), 70-83
- **SUEZ J et al.** Artificial sweeteners induce glucose intolerance by altering the gut microbiota. *Nature,* 2014, vol. 514 (7521), 181-6
- **TRASANDE L ; BLUSTEIN J ; LIU M ; CORWIN E ; COX LM ; BLASER MJ.** Infant antibiotic exposures and early-life body mass. *Int J Obes (Lond),* 2012, vol. 37 (1), 16-23
- **TURNBAUGH PJ ; LEY RE ; MAHOWALD MA ; MAGRINI V ; MARDIS ER ; GORDON JI.** An obesity-associated gut microbiome with increased capacity for energy harvest. *Nature,* 21 December 2006, vol. 444 (7122), 1027-31
- **TURNBAUGH PJ ; BÄCKHED F ; FULTON L ; GORDON JI.** Diet-induced obesity is linked to marked but reversible alterations in the mouse distal gut microbiome. *Cell Host Microbe,* 2008, vol. 3 (4), 213-23
- **VRIEZE A et al.** Transfer of intestinal microbiota from lean donors increases insulin sensitivity in individuals with metabolic syndrome. *Gastroent,* 2012, vol. 143 (4), 913-6
- **WALTER.** Murine gut microbiota-diet trumps genes. *Cell Host Microbe,* 2015, vol. 17 (1), 3-5
- **WEHKAMP J et al.** Innate immunity and colonic inflammation: enhanced expression of epithelial alpha-defensins. *Dig Dis Sci.,* 2002, vol. 47 (6), 1349-55
- **ZHANG X et al.** The oral and gut microbiomes are perturbed in rheumatoid arthritis and partly normalized after treatment. *Nat Med,* 2015, vol. 21 (8), 895-905